# EUROPÄISCHE PATENTSCHRIFT

(11) **EP 1 341 746 B1**
(45) Veröffentlichungstag und Bekanntmachung des Hinweises auf die Patenterteilung: **15.09.2004**
(21) Anmeldenummer: 01980458.2
(22) Anmeldetag: 01.10.2001
(51) Int. Cl.: C07C 43/225, C09K 19/30

(54) **FLÜSSIGKRISTALLINE VERBINDUNGEN**
LIQUID CRYSTALLINE COMPOUNDS
COMPOSES CRISTAUX LIQUIDES

(30) Priorität: 12.12.2000 DE 10061790
(43) Veröffentlichungstag der Anmeldung: 10.09.2003
(73) Patentinhaber: MERCK PATENT GmbH, 64293 Darmstadt (DE)
(72) Erfinder: HECKMEIER, Michael, 69502 Hemsbach (DE); LÜSSEM, Georg, 64372 Ober-Ramstadt (DE); KLEMENT, Dagmar, 64846 Gross-Zimmern (DE); KIRSCH, Peer, 64342 Seeheim-Jugenheim (DE); PAULUTH, Detlef, 64372 Ober-Ramstadt (DE); KRAUSE, Joachim, 64807 Dieburg (DE)
(86) Internationale Anmeldenummer: PCT/EP2001/011322
(87) Internationale Veröffentlichungsnummer: WO 2002/048081

(56) Entgegenhaltungen:
- EP-A- 0 786 445
- DE-A- 19 814 550

## Beschreibung

Die vorliegende Erfindung betrifft flüssigkristalline Verbindungen sowie ein flüssigkristallines Medium, dessen Verwendung für elektrooptische Zwecke und dieses Medium enthaltende Anzeigen.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldem und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nichtlinearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitem wie z.B. CdSe oder TFT's auf der Basis von polykristallinem oder amorphem Silizium. An letzterer Technologie wird weltweit mit großer Intensität gearbeitet

Die TFT-Matrix ist auf der Innenseite der einen Glasplatte der Anzeige aufgebracht, während die andere Glasplatte auf der Innenseite. die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfemseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei lowvolt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d ·Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- kleine optische Doppelbrechung (Δn) für reflektive Anwendungen
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)
- kleine Rotationsviskosität für kleine Schaltzeiten

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Der Erfindung liegt die Aufgabe zugrunde, Medien insbesondere für derartige MFK-, TN- oder STN-Anzeigen bereitzustellen, die die oben angegebenen Nachteile nicht oder nur in geringerem Maße, und vorzugsweise gleichzeitig sehr hohe spezifische Widerstände und niedrige Schwellenspannungen aufweisen. Für diese Aufgabe werden flüssigkristalline Verbindungen benötigt, die einen hohen Klärpunkt und eine niedrige Rotationsvikosität besitzen.

Mit den aus dem Stand der Technik zur Verfügung stehenden Medien ist es nicht möglich, diese Vorteile unter gleichzeitigem Erhalt der übrigen Parameter zu realisieren.

Es wurde nun gefunden, dass diese Aufgabe gelöst werden kann, wenn man die erfindungsgemäßen flüssigkristallinen Verbindungen verwendet.

Gegenstand der Erfindung sind somit flüssigkristalline Verbindungen der Formel I, 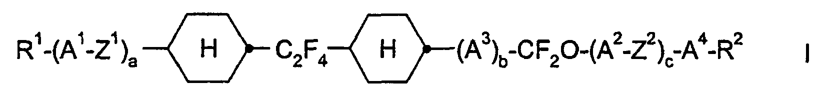 worin
- R¹ und R²: jeweils unabhängig voneinander einen unsubstituierten, einen einfach durch CN oder CF₃ oder mindestens einfach durch Halogen substituierten Alkylrest mit 1 bis zu 15 C-Atomen, wobei in diesen Resten auch eine oder mehrere CH₂-Gruppen durch -O-, -S-, -CH=CH-, -C≡C-, - OC-O- oder -O-CO- so ersetzt sein können, dass O-Atome nicht direkt miteinander verknüpft sind, und R² auch CN, SF₅, F, CI, NCS oder SCN,
- A¹, A², A³ und A⁴: a) einen 1,4-Cyclohexenylen- oder 1,4-Cyclohexylenrest, worin eine oder zwei nicht benachbarte CH₂-Gruppen durch -O- oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl-, 1,4-Bicyclo[2,2,2]-octylen-, einen Naphthalin-2,6-diyl, Decahydronaphthalin-2,6-diyl, und 1,2,3,4-Tetrahydronaphthalin-2,6-diyl
wobei die Reste a), b) und c) ein oder mehrfach durch Halogenatome substituiert sein können,
- Z¹ und Z²: jeweils unabhängig voneinander -CO-O-, -O-CO-, -CF₂O-, -OCF₂-, -CH₂O-, -OCH₂-, -CH₂CH₂-,-(CH₂)₄-, -C₂F₄-, -CH₂CF₂-, -CF₂CH₂-, -CF=CF-, -CH=CH-, -C≡C- oder eine Einfachbindung,
- a: 0, 1 oder 2,
- b: 0, 1 oder 2, und
- c: 0, 1 oder 2, wobei a + b + c ≤ 2 ist,
bedeutet.

Gegenstand der Erfindung ist weiterhin die Verwendung der Verbindungen der Formel I in flüssigkristallinen Medien.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Insbesondere zeichnen sich die erfindungsgemäßen Verbindungen durch ihren hohen Klärpunkt und ihre niedrigen Werte für die Rotationsviskosität aus. Chemisch, thermisch und gegen Licht sind sie stabil.

Gegenstand der Erfindung sind insbesondere die Verbindungen der Formel I, worin R¹ Alkyl mit 1 bis 10 C-Atomen oder einen Alkenylrest mit 2 bis 10 C-Atomen bedeutet.

Insbesondere bevorzugt sind Verbindungen der Formel I, worin c = 0 ist. Z¹ und Z² sind vorzugsweise eine Einfachbindung, ferner -CF₂O- -OCF₂-, -C₂F₄-, -CH₂O-, -OCH₂- oder -COO-. a ist vorzugsweise 0.

Falls R¹ und/oder R² einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ und/oder R² einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und/oder R² einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ und/oder R² einen einfach durch CN oder CF₃ substituierten Alkyloder Alkenylrest bedeutet, so ist dieser Rest vorzugsweise geradkettig. Die Substitution durch CN oder CF₃ ist in beliebiger Position.

Falls R¹ und/oder R² einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R¹ und R² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

R² bedeutet vorzugsweise F, Cl, CN, CF₃, SF₅, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCFHCFHCF₃, OCH₂CF₂CF₃, OCF₂CF₂CF₃, OCF₂CFHCFH₂, OCF₂CH₂CF₂H, OCFHCF₂CFH₂, OCFHCFHCF₂H, OCFHCH₂CF₃, OCH₂CFHCF₃, OCH₂CF₂CF₂H, OCF₂CFHCH₃, OCF₂CH₂CFH₂, OCFHCF₂CH₃, OCFHCFHCFH₂, OCFHCH₂CF₃, OCH₂CF₂CFH₂, OCH₂CFHCF₂H, OCF₂CH₂CH₃, OCFHCFHCH₃, OCFHCH₂CFH₂, OCH₂CF₂CH₃, OCH₂CFHCFH₂, OCH₂CH₂CF₂H, OCHCH₂CH₃, OCH₂CFHCH₃, OCH₂CH₂CF₂H, OCCIFCF₃, OCCIFCCIF₂, OCCIFCFH₂, OCFHCCl₂F, OCClFCF₂H, OCClFCClF₂, OCF₂CClH₂, OCF₂CCl₂H, OCF₂CCl₂F, OCF₂CClFH, OCF₂CClF₂, OCF₂CF₂CClF₂, OCF₂CF₂CCl₂F, OCClFCF₂CF₃, OCClFCF₂CF₂H, OCClFCF₂CClF₂, OCClFCFHCF₃, OCClFCClFCF₃, OCCl₂CF₂CF₃, OCClHCF₂CF₃, OCClFCF₂CF₃, OCClFCClFCF₃, OCF₂CClFCFH₂, OCF₂CF₂CCl₂F, OCF₂CCl₂CF₂H, OCF2CH₂CClF₂, OCClFCF₂CFH₂, OCFHCF₂CCl₂F, OCClFCFHCF₂H, OCClFCClFCF₂H, OCFHCFHCClF₂, OCClFCH₂CF₃, OCFHCCl₂CF₃, OCCl₂CFHCF₃, OCH₂CClFCF₃, OCCl₂CF₂CF₂H, OCH₂CF₂CClF₂, OCF₂CClFCH₃, OCF₂CFHCCl₂H, OCF₂CCl₂CFH₂, OCF₂CH₂CCl₂F, OCClFCF₂CH₃, OCFHCF₂CCl₂H, OCClFCClFCFH₂, OCFHCFHCCl₂F, OCClFCH₂CF₃, OCFHCCl₂CF₃, OCCl₂CF₂CFH₂, OCH₂CF₂CCl₂F, OCCl₂CFHCF₂H, OCClHCClFCF₂H, OCF₂CClHCClH₂, OCF₂CH₂CCl₂H, OCClFCFHCH₃, OCF₂CClFCCl₂H, OCClFCH₂CFH₂, OCFHCCl₂CFH₂, OCCl₂CF₂CH₃, OCH₂CF₂CClH₂, OCCl₂CFHCFH₂, OCH₂CClFCFCl₂, OCH₂CH₂CF₂H, OCClHCClHCF₂H, OCH₂CCl₂CF₂H, OCClFCH₂CH₃, OCFHCH₂CCl₂H, OCClHCFHCClH₂, OCH₂CFHCCl₂H, OCCl₂CH₂CF₂H, OCH₂CCl₂CF₂H, CH=CF₂, CF=CF₂, OCH=CF₂, OCF=CF₂, CH=CHF, OCH=CHF, CF=CHF, OCF=CHF, insbesondere F, Cl, CN, CF₃, SF₅, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCClFCF₂CF₃.

Der Einfachheit halber bedeuten im folgenden Cyc einen 1,4-Cyclohexylenrest, Che einen 1,4-Cyclohexenylrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr eine Pyrimidin-2,5-diylrest, Bi einen Bicyclo[2.2.2]octylenrest, PheF einen 2- oder 3-Fluor-1,4-phenylenrest, PheFF einen 2,3-Difluor- oder 2,6-Difluor-1,4-phenylenrest, Nap einen substituierten oder unsubstituierten Naphthalinrest, Dec einen Decahydronaphthalinrest.

Die Verbindungen der Formel I umfassen dementsprechend die bevorzugten Verbindungen mit drei Ringen der Teilformeln Ia bis Ig:

R¹-Cyc-C₂F₄-Cyc-CF₂O-Phe-R² Ia

R¹-Cyc-C₂F₄-Cyc-CF₂O-PheF-R² Ib

R¹-Cyc-C₂F₄-Cyc-CF₂O-PheFF-R² Ic

R¹-Cyc-C₂F₄-Cyc-CF₂O-Bi-R² Id

R¹-Cyc-C₂F₄-Cyc-CF₂O-Nap-R² Ie

R¹-Cyc-C₂F₄-Cyc-CF₂O-Dec-R² If

R¹-Cyc-C₂F₄-Cyc-CF₂O-Bi-R² Ig

Verbindungen mit vier Ringen der Teilformeln Ih und Iw:

R¹-Cyc-C₂F₄-Cyc-Cyc-CF₂O-Phe-R² Ih

R¹-Cyc-C₂F₄-Cyc-Cyc-CF₂4-PheF-R² Ii

R¹-Cyc-C₂F₄-Cyc-Cyc-CF₂O-PheFF-R² Ij

R¹-Cyc-C₂F₄-Cyc-Cyc-CF₂O-Phe-R² Ik

R¹-Cyc-C₂F₄-Cyc-Cyc-CF₂O-PheF-R² Il

R¹-Cyc-C₂F₄-Cyc-Cyc-CF₂O-PheFF-R² Im

R¹-Cyc-C₂F₄-Cyc-Cyc-CF₂O-Nap-R² In

R¹-Cyc-C₂F₄-Cyc-Cyc-CF₂O-Dec-R² Io

R¹-Cyc-C₂F₄-Cyc-Cyc-CF₂O-Bi-R² Ip

R¹-Cyc-C₂F₄-Cyc-Phe-CF₂O-Phe-R² Iq

R¹-Cyc-C₂F₄-Cyc-Phe-CF₂O-PheF-R² Ir

R¹-Cyc-C₂F₄-Cyc-Phe-CF₂O-PheFF-R² Is

R¹-Cyc-C₂F₄-Cyc-PheF-CF₂O-Phe-R² It

R¹-Cyc-C₂F₄-Cyc-PheFF-CF₂O-Phe-R² Iu

R¹-Cyc-C₂F₄-Cyc-PheFF-CF₂O-PheFF-R² Iv

R¹-Cyc-C₂F₄-Cyc-CF₂O-Phe-Cyc-R² Iw

Darunter sind besonders diejenigen Verbindungen der Teilformeln la, Ib und Ic bevorzugt.

In den Verbindungen der vor- und nachstehenden Formeln bedeutet R² vorzugsweise F, CN, OCF₃, OCHF₂, CF₃, OCHFCF₃, OC₂F₅ oder OCF₂CHFCF₃, geradkettiges Alkyl oder Alkoxy.

R¹ bedeutet vorzugsweise geradkettiges unsubstituiertes Alkyl, Alkoxy, Alkenyloxy oder Alkenyl mit bis zu 10 C-Atomen.

A² bedeutet vorzugsweise Phe, PheF, PheFF, Cyc oder Che, ferner Pyr oder Dio, Dec oder Nap. Bevorzugt enthalten die Verbindungen der Formel I nicht mehr als einen der Reste Bi, Pyd, Pyr, Dio, Dit, Nap oder Dec.

Bevorzugt sind auch alle Verbindungen der Formel I sowie aller Teilformeln, in denen A¹ ein ein- oder zweifach substituiertes 1,4-Phenylen bedeutet. Insbesondere sind dies 2-Fluor-1,4-phenylen, 3-Fluor-1,4-phenylen, 2,3-Difluor-1,4-phenylen sowie 2,6-Difluor-1,4-phenylen.

Bevorzugte kleinere Gruppen von Verbindungen der Formel I sind diejenigen der Teilformeln I1 bis 124: 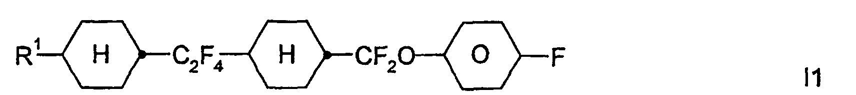 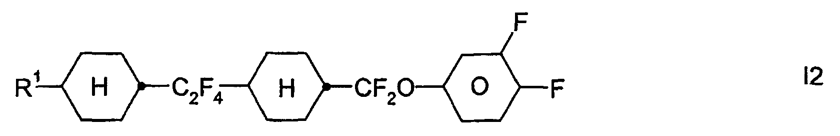 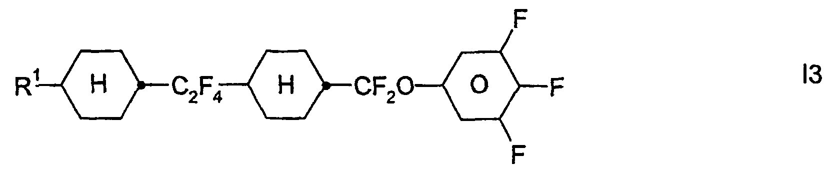 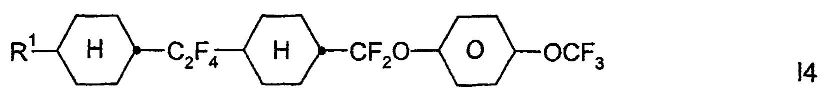 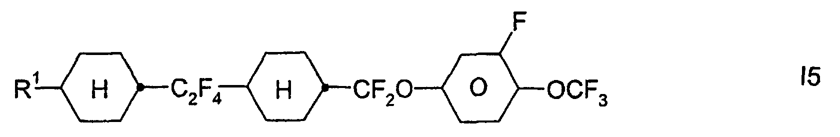 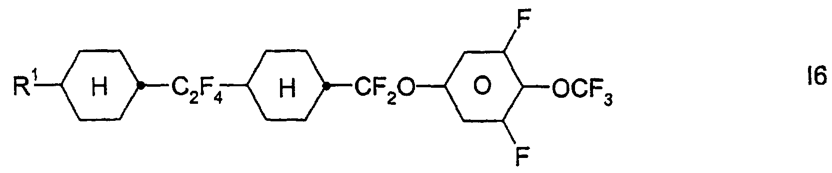 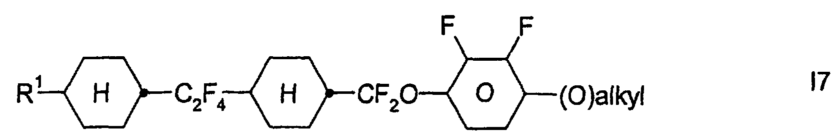 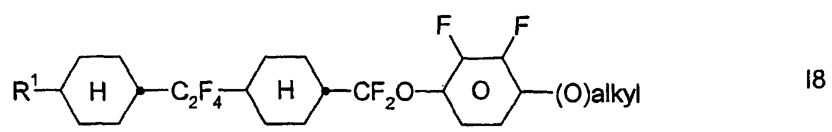 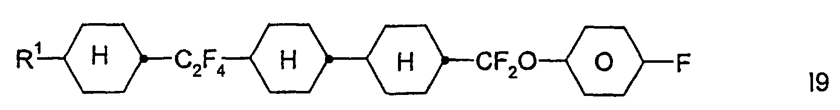 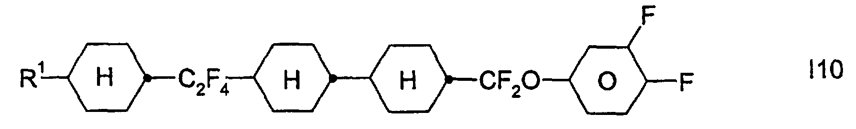 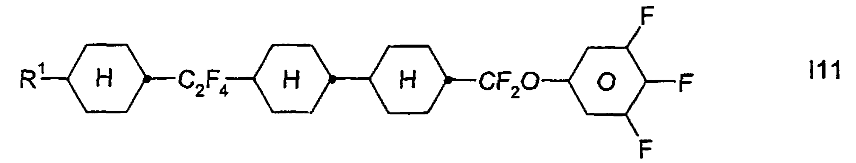 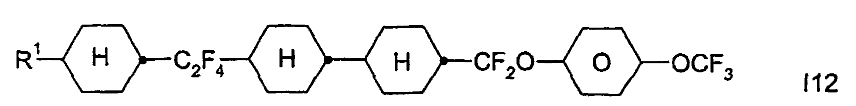 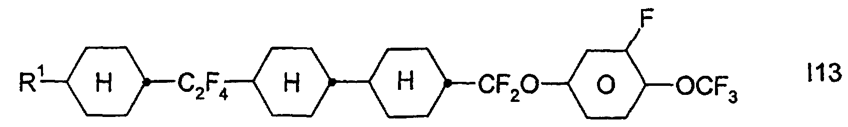 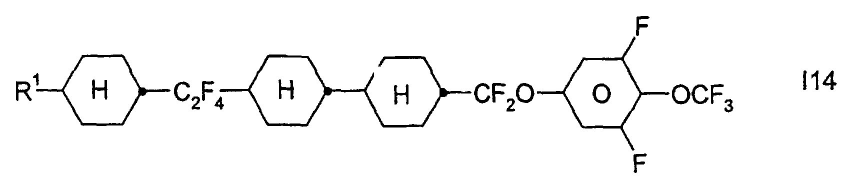 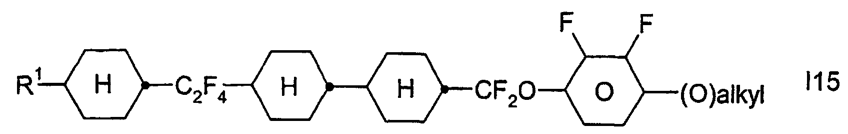 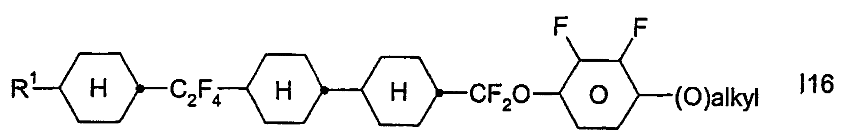 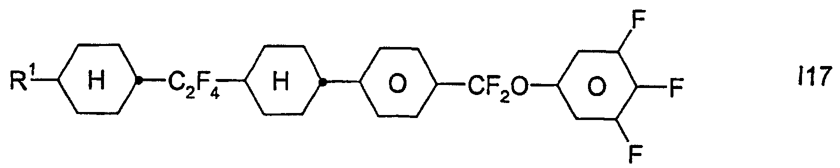 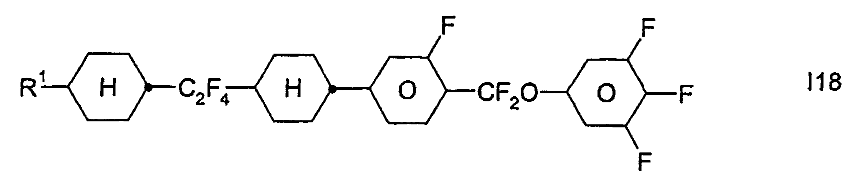 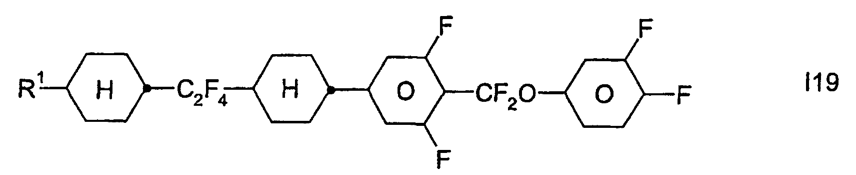 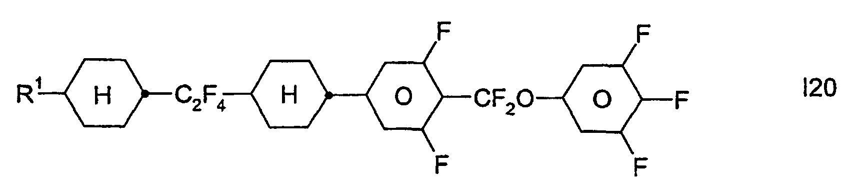 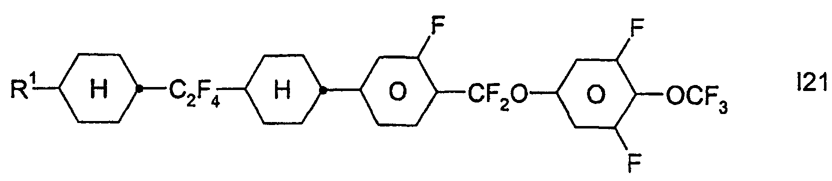 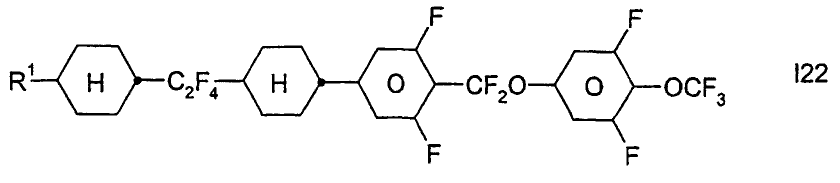 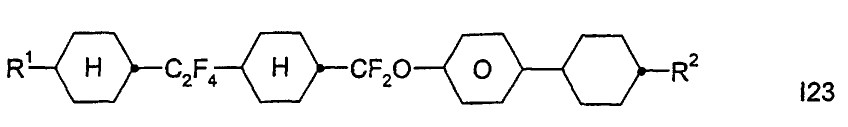 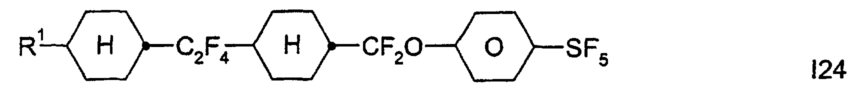 worin
R¹ die in Anspruch 1 angegebene Bedeutung hat und "alkyl" ein geradkettiger oder verzweigter Alkylrest mit 1-9 C-Atomen ist.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Verbindungen können z.B. wie folgt hergestellt werden:

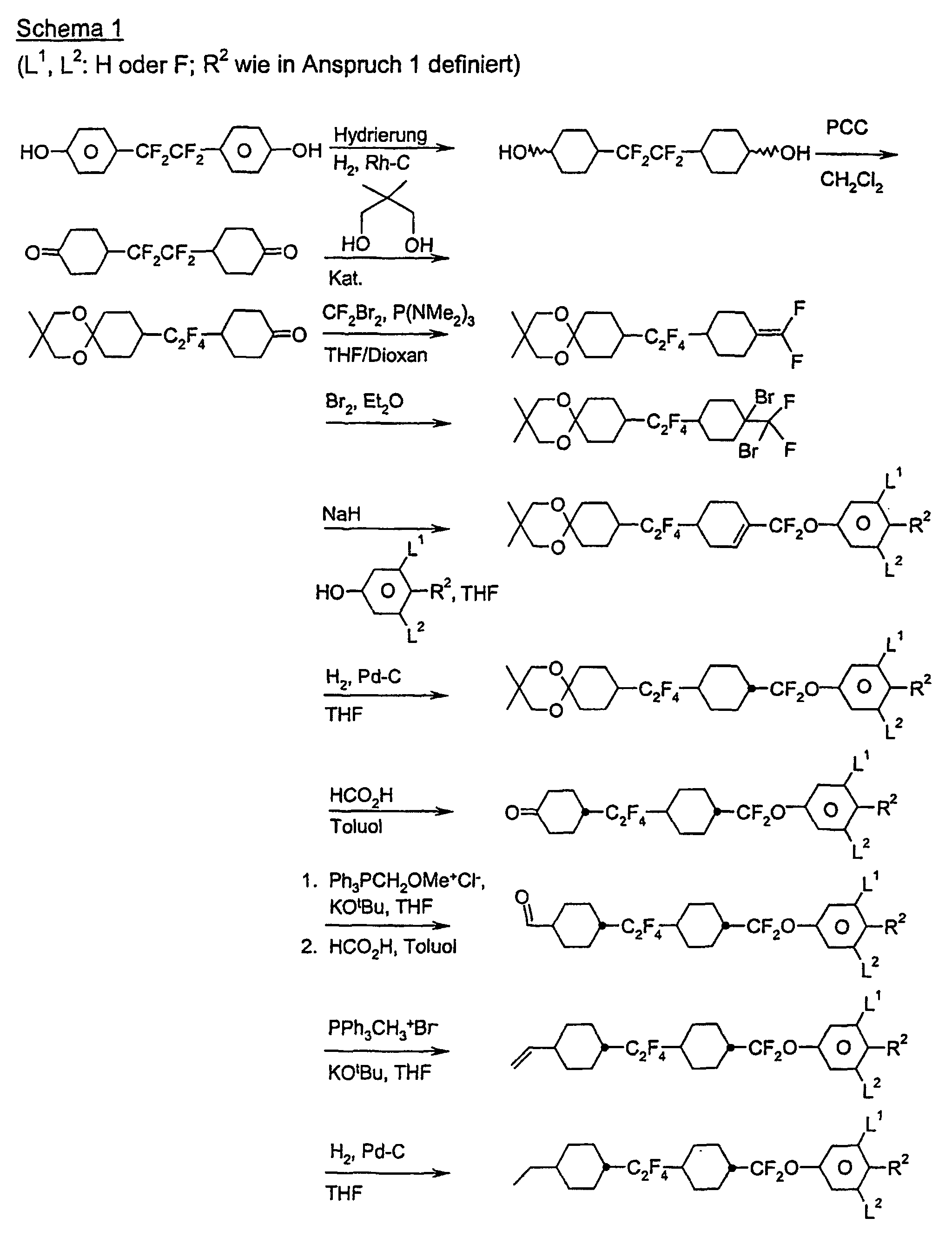

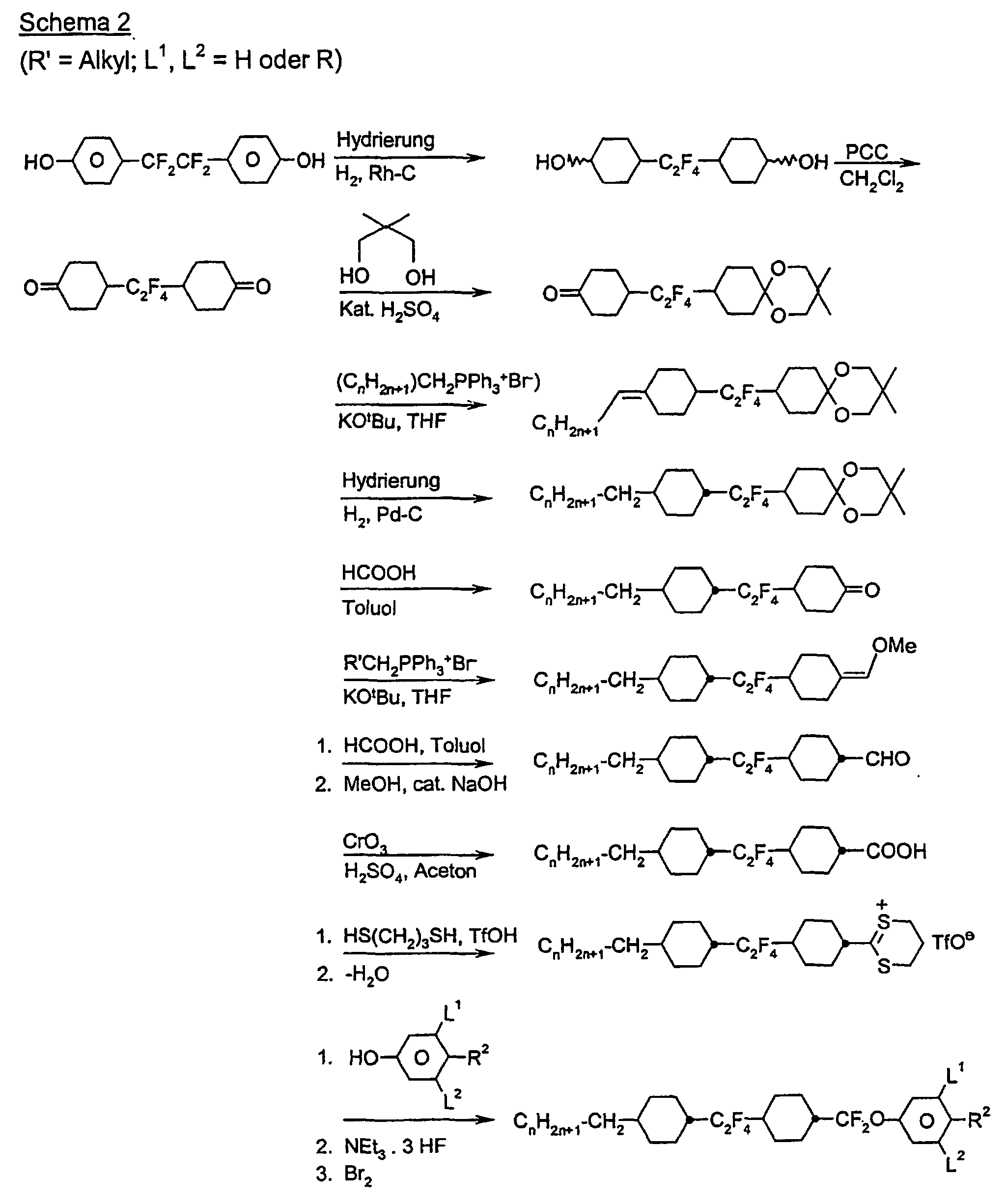

Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die Forderung nach hohem Klärpunkt, nematischer Phase bei tiefer Temperatur sowie einem hohen Δε konnte bislang nur unzureichend erfüllt werden. Flüssigkristallmischungen, wie z. B. MLC-6476 und MLC-6625 (Merck KGaA, Darmstadt, Deutschland) weisen zwar vergleichbare Klärpunkte und Tieftemperaturstabilitäten auf, sie haben jedoch relativ hohe Δn-Werte als auch höhere Schwellenspannungen von ca.≥ 1,7 V.

Andere Mischungssysteme besitzen vergleichbare Viskositäten und Werte von Δε, weisen jedoch nur Klärpunkte in der Gegend von 60 °C auf.

Die erfindungsgemäßen Flüssigkristallmischungen ermöglichen es bei Beibehaltung der nematischen Phase bis -20 °C und bevorzugt bis -30 °C, besonders bevorzugt bis -40 °C, Klärpunkte oberhalb 80°, vorzugsweise oberhalb 90°, besonders bevorzugt oberhalb 100 °C, gleichzeitig dielektrische Anisotropiewerte Δε ≥ 4, vorzugsweise ≥ 6 und einen hohen Wert für den spezifischen Widerstand zu erreichen, wodurch hervorragende STN- und MKF-Anzeigen erzielt werden können. Insbesondere sind die Mischungen durch kleine Operationsspannungen gekennzeichnet. Die TN-Schwellen liegen unterhalb 1,5 V, vorzugsweise unterhalb 1,3 V.

Es versteht sich, dass durch geeignete Wahl der Komponenten der erfindungsgemäßen Mischungen auch höhere Klärpunkte (z.B. oberhalb 110°) bei höheren Schwellenspannung oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringeren Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum eine kleinerere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Fließviskosität ν₂₀ bei 20°C ist vorzugsweise < 60 mm² · s⁻¹, besonders bevorzugt < 50 mm² · s⁻¹. Der nematische Phasenbereich ist vorzugsweise mindestens 90°, insbesondere mindestens 100°. Vorzugsweise erstreckt sich dieser Bereich mindestens von -30° bis +80°.

Messungen des "Capacity Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I Cyanophenylcyclohexane der Formel 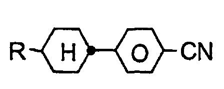 oder Ester der Formel

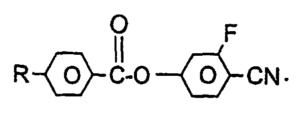

Auch die UV-Stabilität der erfindungsgemäßen Mischungen ist erheblich besser, d. h. sie zeigen eine deutlich kleinere Abnahme des HR unter UV-Belastung.

Vorzugsweise basieren die erfindungsgemäßen Medien auf mehreren (vorzugsweise zwei, drei oder mehr) Verbindungen der Formel I, d.h. der Anteil dieser Verbindungen ist 5-95 %, vorzugsweise 10-60 % und besonders bevorzugt im Bereich von 15-40 %.

Die einzelnen Verbindungen der Formeln I bis IX und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:
- Das Medium enthält vorzugsweise ein, zwei oder drei homologe Verbindungen der Formel I, wobei jedes Homologe zu maximal 10% in der Mischung enthalten ist.
- Das Medium enthält Verbindungen der Formel I, worin R¹ vorzugsweise Ethyl und/oder Propyl, ferner Butyl, Pentyl, Hexyl und Heptyl bedeutet. Verbindungen der Formel I mit kurzen Seitenketten R¹ beeinflussen positiv die elastischen Konstanten, insbesondere K_{1,} und führen zu Mischungen mit besonders niedrigen Schwellenspannungen.
- Medium enthält zusätzlich eine oder mehrere Verbindungen ausgewählt aus der Gruppe bestehend aus den allgemeinen Formeln II bis IX: 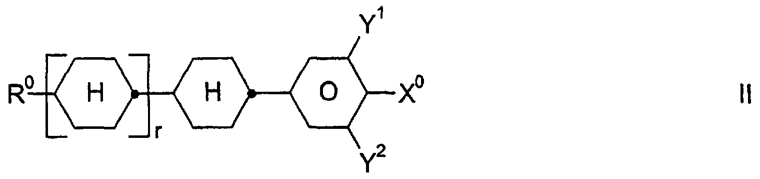 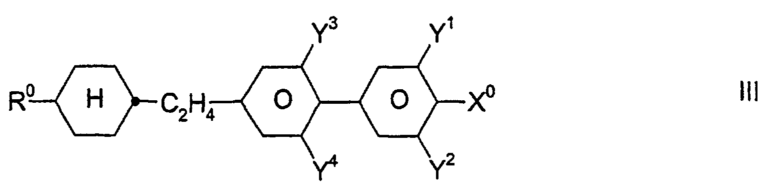 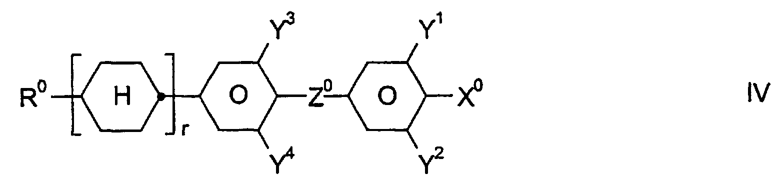 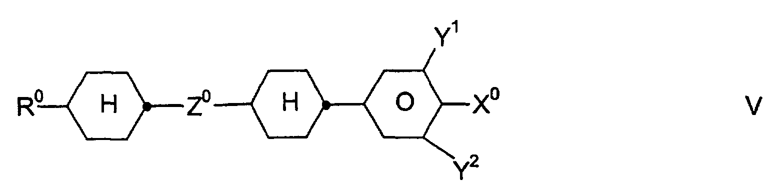 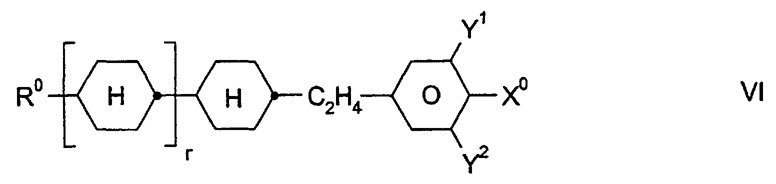 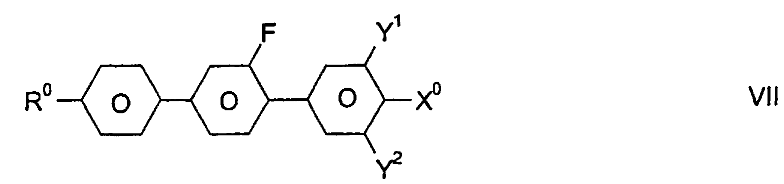 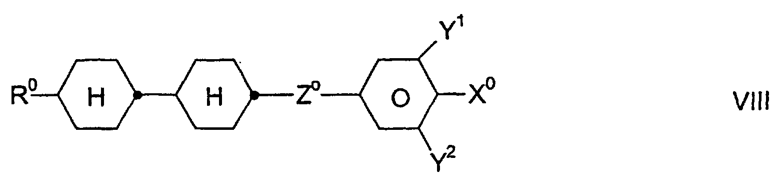 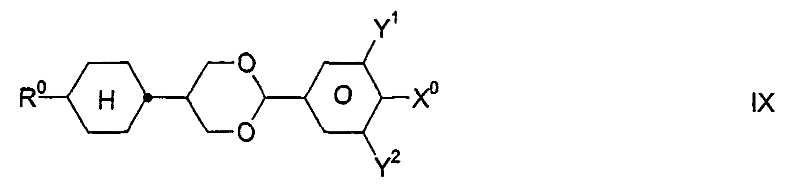 worin die einzelnen Reste die folgenden Bedeutungen haben:
   - R⁰: n-Alkyl, Oxaalkyl, Fluoralkyl, Alkenyloxy oder Alkenyl mit jeweils bis zu 9 C-Atomen
   - X⁰: F, Cl, halogeniertes Alkyl, halogeniertes Alkenyl, halogeniertes Alkenyloxy oder halogeniertes Alkoxy mit bis zu 7 C-Atomen,
   - Z⁰: -CH=CH-, -C₂H₄-, -C₂F₄-, -CF=CF-, -CF₂O- OCF₂- oder - COO-,
   - Y¹ ,Y², Y³ und Y⁴: jeweils unabhängig voneinander H oder F, und
   - r: 0 oder 1.

Die Verbindung der Formel IV ist vorzugsweise 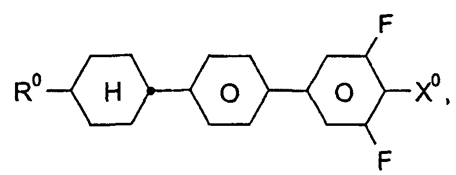 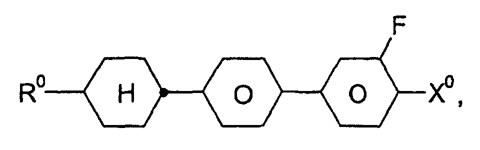 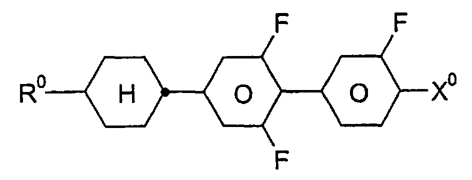 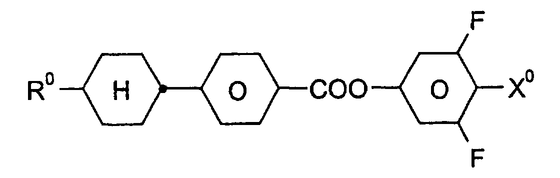 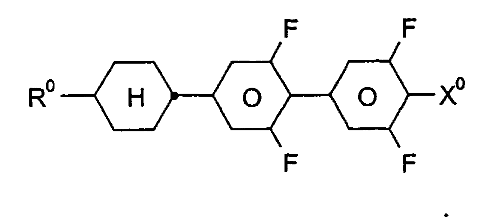 oder 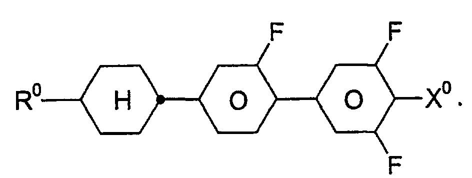
- Medium enthält zusätzlich eine oder mehrere Verbindungen der Formeln 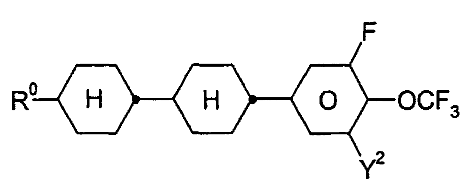 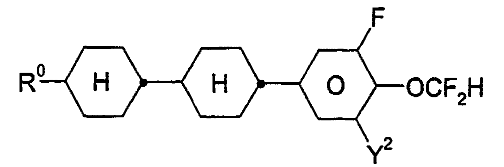 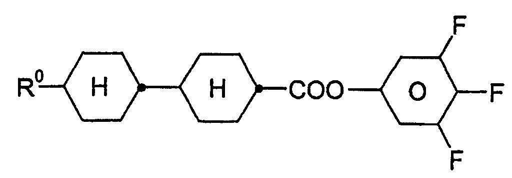 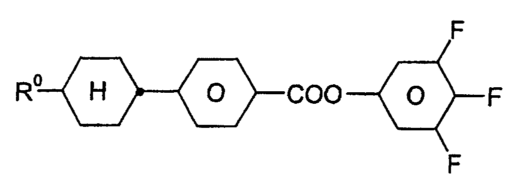 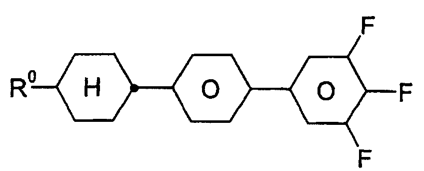 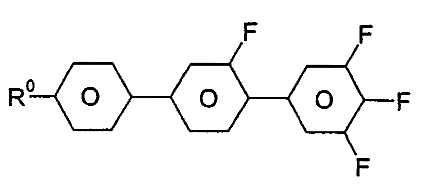 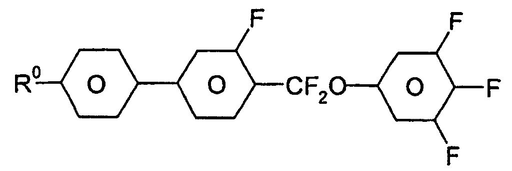 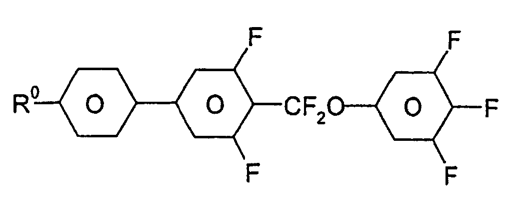 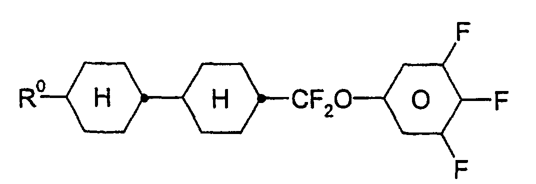 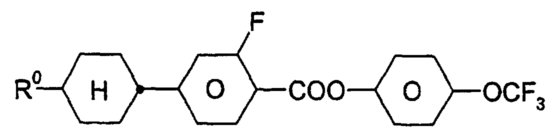 und/oder 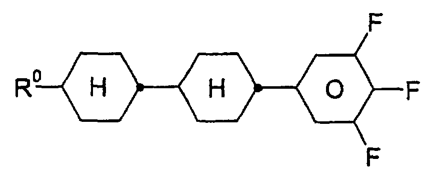 worin R⁰ und Y² die oben angegebene Bedeutung haben.
- Das Medium enthält vorzugsweise ein, zwei oder drei, ferner vier, Homologe der Verbindungen ausgewählt aus der Gruppe H1 bis H16 (n = 1-7): 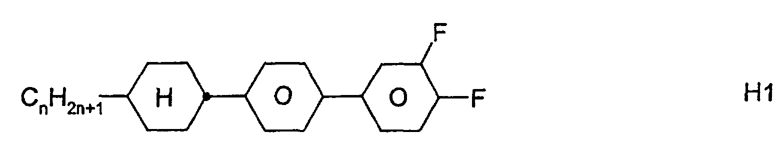 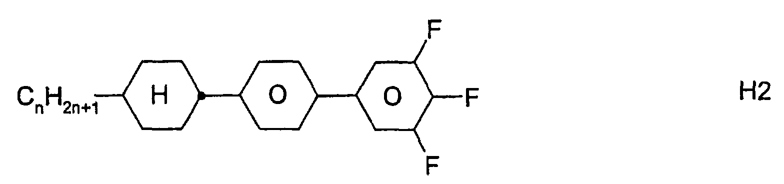 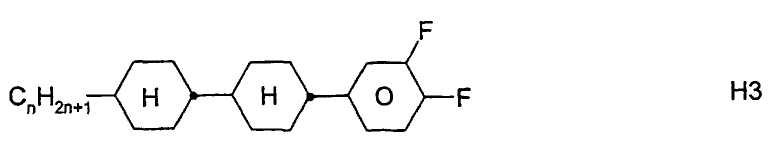 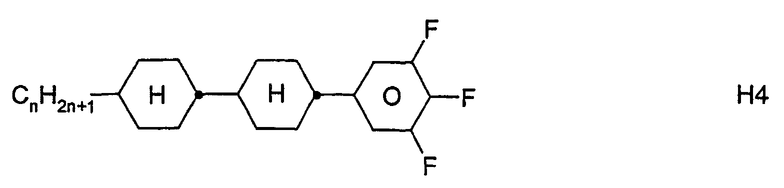 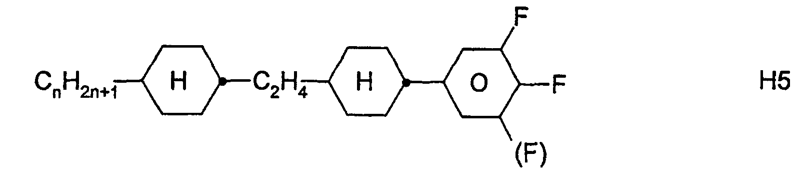 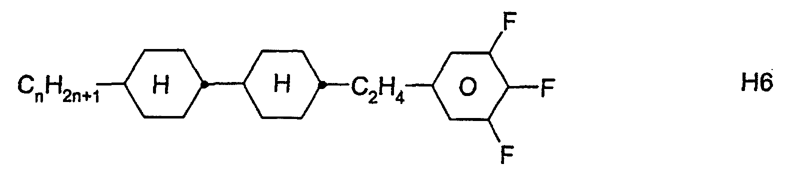 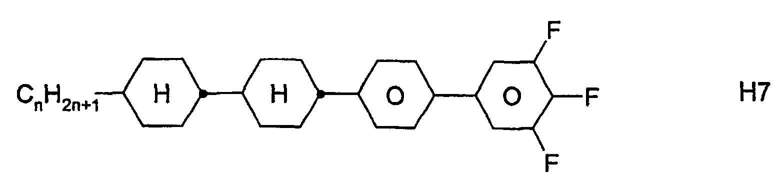 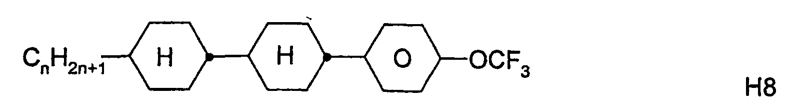 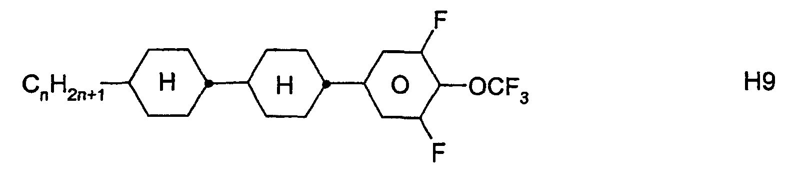 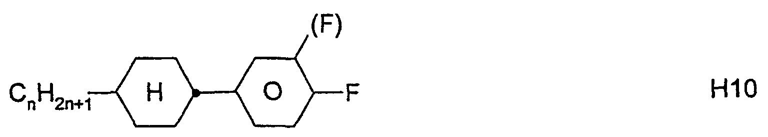 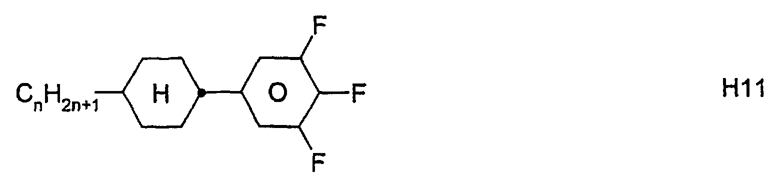 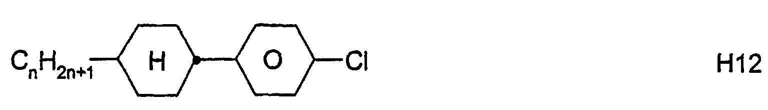 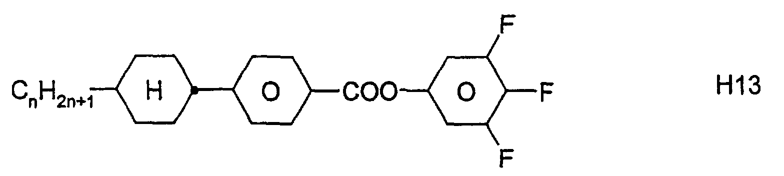 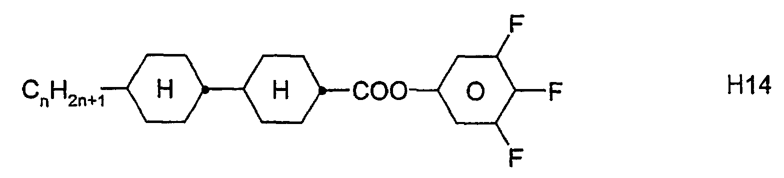 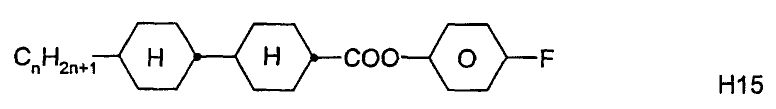 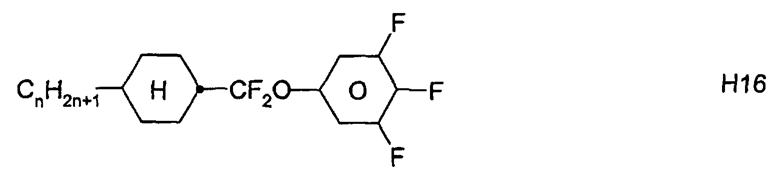
- Das Medium enthält zusätzlich ein oder mehrere Dioxane der Formeln DI und/oder DII, 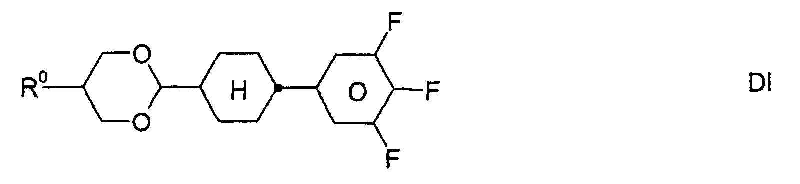 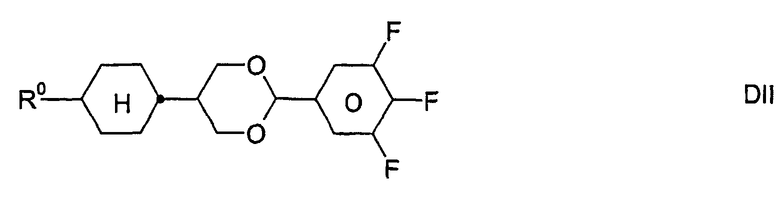 worin R⁰ die in Anspruch 7 angegebenen Bedeutungen hat. Vorzugsweise bedeutet R⁰ in den Verbindungen der Formeln DI und DII geradkettiges Alkyl oder Alkenyl mit bis zu 7 C-Atomen.
- Das Medium enthält zusätzlich eine oder mehrere Verbindungen ausgewählt aus der Gruppe bestehend aus den allgemeinen Formeln X bis XV: 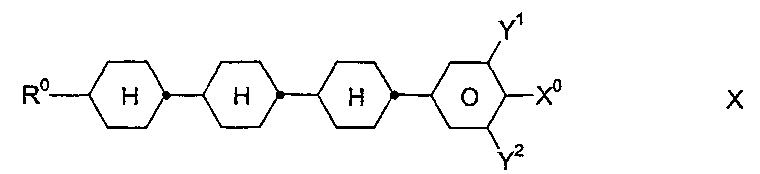 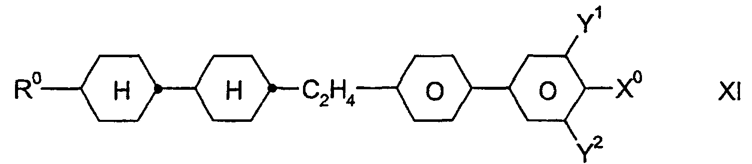 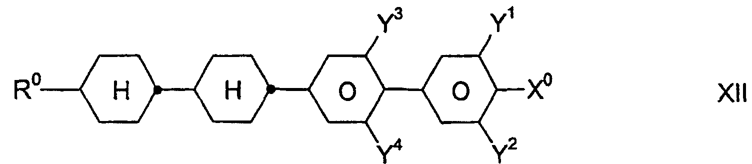 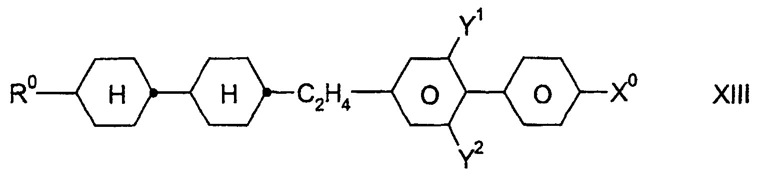 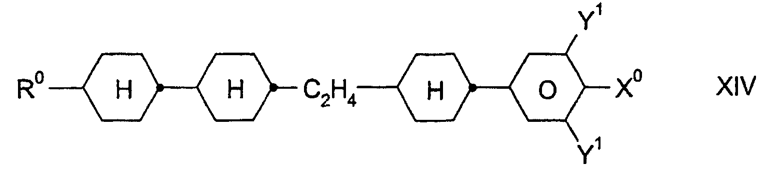 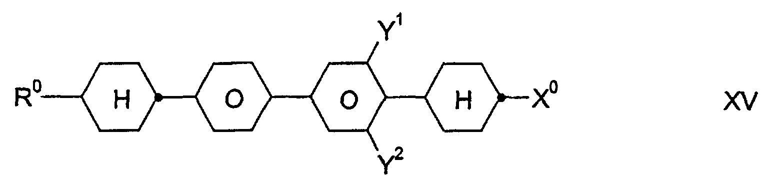 worin R⁰, X⁰, Y¹,Y², Y³ und Y⁴ jeweils unabhängig voneinander eine der in Anspruch 7 angegebene Bedeutung haben, vorzugsweise bedeutet X⁰ F, Cl, CF₃, OCF₃, OCHF₂. R⁰ bedeutet vorzugsweise Alkyl, Oxaalkyl, Fluoralkyl, Alkenyl oder Alkenyloxy.
- Der Anteil an Verbindungen der Formeln I bis lX zusammen beträgt im Gesamtgemisch mindestens 50 Gew.-%.
- Der Anteil an Verbindungen der Formel I beträgt im Gesamtgemisch 5 bis 50 Gew.-%.
- Der Anteil an Verbindungen der Formeln II bis IX im Gesamtgemisch beträgt 30 bis 70 Gew.-%. 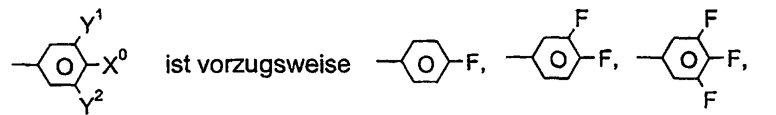 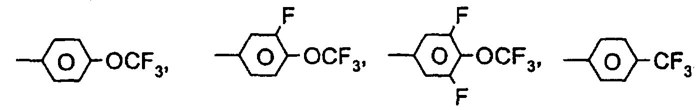 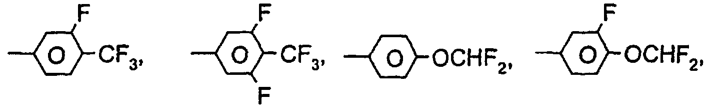 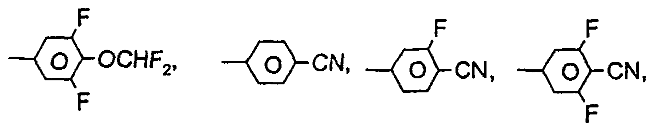 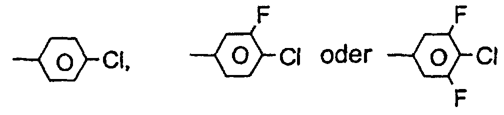
- Das Medium enthält Verbindungen der Formeln II, III, IV, V, VI, VII, VIII und/oder IX.
- R⁰ ist geradkettiges Alkyl oder Alkenyl mit 2 bis 7 C-Atomen.
- Das Medium besteht im wesentlichen aus Verbindungen der Formeln I bis XV.
- Das Medium enthält weitere Verbindungen, vorzugsweise ausgewählt aus der folgenden Gruppe bestehend aus den allgemeinen Formeln XVI bis XIX: 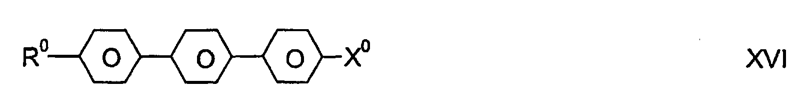 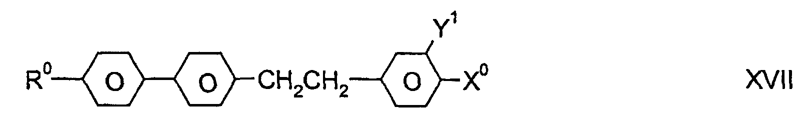 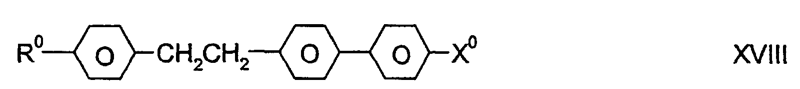 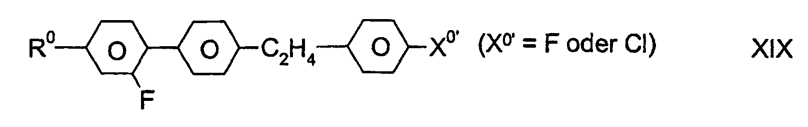 worin R⁰ und X⁰ die oben angegebene Bedeutung haben und die 1,4-Phenylenringe durch CN, Chlor oder Fluor substituiert sein können. Vorzugsweise sind die 1,4-Phenylenringe ein- oder mehrfach durch Fluoratome substituiert.
- Das Medium enthält weitere Verbindungen, vorzugsweise ausgewählt aus der folgenden Gruppe bestehend aus den Formeln RI, bis RX, 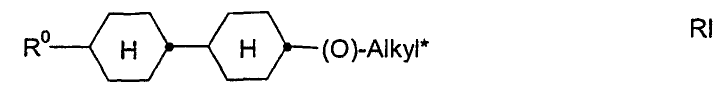 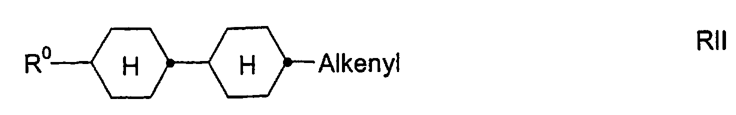 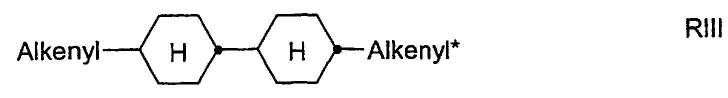 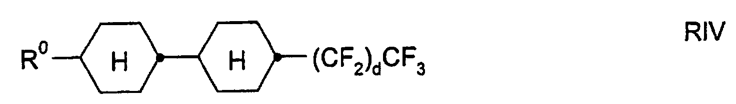 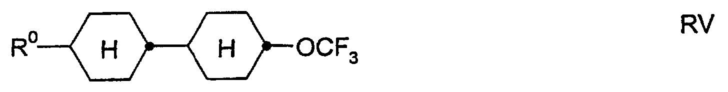 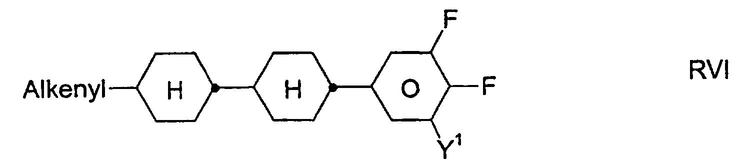 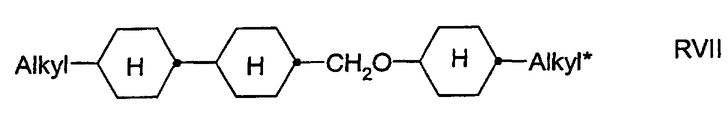 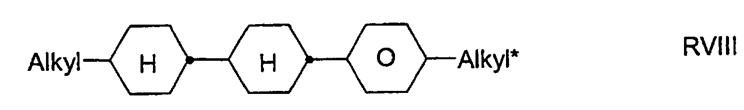 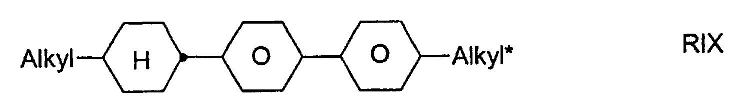 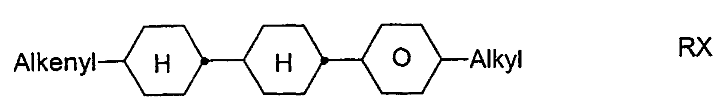 worin
   - R⁰: n-Alkyl, Oxaalkyl, Fluoralkyl, Alkenyloxy oder Alkenyl mit jeweils bis zu 9 C-Atomen,
   - d: 0, 1 oder 2,
   - Y¹: H oder F,
   - Alkyl oder Alkyl^{*}: jeweils unabhängig voneinander ein geradkettiger oder verzweigter Alkylrest mit 1-9 C-Atomen,
   - Alkenyl oder Alkenyl^{*}: jeweils unabhängig voneinander einen geradkettigen oder verzweigten Alkenylrest mit bis zu 9 C-Atomen
   bedeuten.
- Das Medium enthält vorzugsweise ein oder mehrere Verbindungen der Formeln 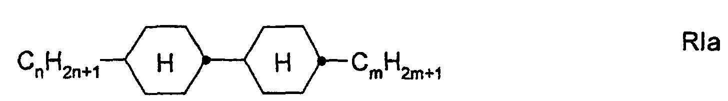 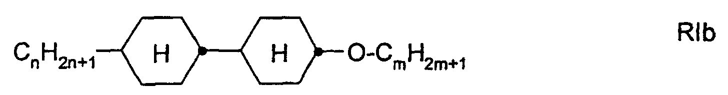 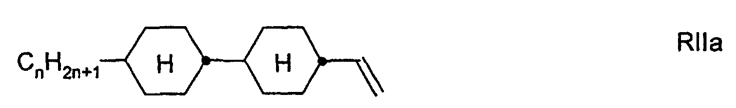 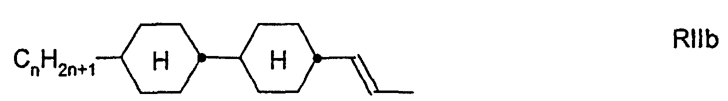 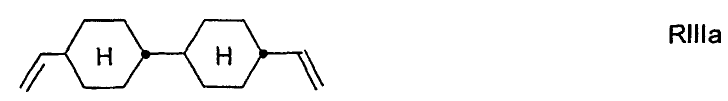 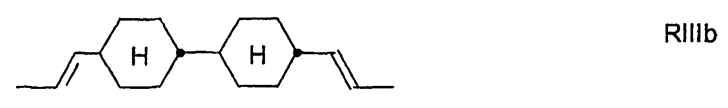 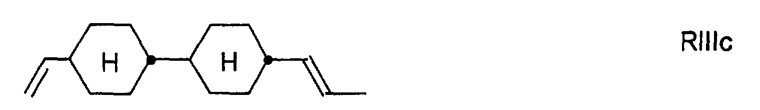 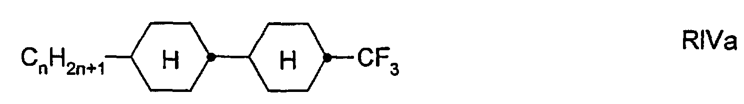 worin n und m jeweils eine ganze Zahl von 1-9 bedeuten.
- Das Gewichtsverhältnis I: (II + III + IV + V + VI + VII + VIII + IX) ist vorzugsweise 1 : 10 bis 10 : 1.
- Medium besteht im wesentlichen aus Verbindungen ausgewählt aus der Gruppe bestehend aus den allgemeinen Formeln 1 bis XV.

Es wurde gefunden, dass bereits ein relativ geringer Anteil an Verbindungen der Formel I im Gemisch mit üblichen Flüssigkristallmaterialien, insbesondere jedoch mit einer oder mehreren Verbindungen der Formel II, III, IV, V, VI, VII, VII und/oder IX zu einer beträchtlichen Erniedrigung der Schwellenspannung und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Die Verbindungen der Formeln I bis IX sind farblos, stabil und untereinander und mit anderen Flüssigkristallmaterialien gut mischbar.

Der Ausdruck "Alkyl" oder "Alkyl*"umfasst geradkettige und verzweigte Alkylgruppen mit 1-9 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" oder "Alkenyl*" umfasst geradkettige und verzweigte Alkenylgruppen mit bis zu 9 Kohlenstoffatomen, insbesondere die geradkettigen Gruppen. Besonders bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1 E-Alkenyl, C₄-C₇-3E-Alkenyl und C₅-C₇-4-Alkenyl. Beispiele bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" umfasst vorzugsweise geradkettige Reste der Formel CₙH₂ₙ₊₁-O-(CH₂)_{m,} worin n und m jeweils unabhängig voneinander 1 bis 6 bedeuten. Vorzugsweise ist n = 1 und m 1 bis 6.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine Gruppe -CH₂CH₂₋ in Z¹ führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und II + III + IV + V + VI + VII + VII + IX hängt weitgehend von den gewünschten Eigenschaften, von der Wahl der Komponenten der Formeln I, II, III, IV, V, VI, VII, VIII und/oder IX und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln I bis XV in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XV ist.

In einer besonders bevorzugten Ausführungsform enthalten die erfindungsgemäßen Medien Verbindungen der Formel II bis IX (vorzugsweise II und/oder III), worin X₀ OCF₃, OCHF₂, F, OCH=CF₂, OCF=CF₂, OCF₂CHFCF₃, oder OCF₂-CF₂H bedeutet. Eine günstige synergistische Wirkung mit den Verbindungen der Formel I führt zu besonders vorteilhaften Eigenschaften.

Die erfindungsgemäßen Mischungen mit niedriger optischer Anisotropie (Δn ≤ 0,09) sind insbesondere für reflektive Displays geeignet. Low Vₜₕ-Mischungen, sind insbesondere für 3,3 V-Treiber und 4V- oder 5V-Treiber geeignet. Für letztere Anwendungen sind Ester-freie Mischungen bevorzugt. Die erfindungsgemäßen Mischungen führen zu einer Verbesserung der Reliability (Image sticking, Point defect, etc.) und sind daher auch sehr gut für IPS-Anwendungen geeignet.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfemen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe oder chirale Dotierstoffe zugesetzt werden.

C bedeutet eine kristalline, S eine smektische, S_{c} eine smektische C, N eine nematische und I die isotrope Phase.

V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2-fachen Wert von V₁₀. Δn bezeichnet die optische Anisotropie und nₒ den Brechungsindex. Δε bezeichnet die dielektrische Anisotropie (Δε = ε_{∥}- ε_{┴}, wobei ε_{∥} die Dielektrizitätskonstante parallel zu den Moleküllängsachsen und ε_{┴} die Dielektrizitätskonstante senkrecht dazu bedeutet). Die elektrooptischen Daten wurden in einer TN-Zelle im 1. Minimum (d.h. bei einem d · Δn-Wert von 0,5) bei 20 °C gemessen, sofern nicht ausdrücklich etwas anderes angegeben wird. Die optischen Daten wurden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. n und m bedeuten jeweils unabhängig voneinander 1, 2, 3, 4, 5, 6, 7, 8, 9,10,11, 12, 13, 14 oder 15. Die Codierung gemäß Tabelle B versteht sich von selbst. in Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R2, L¹, L2 | R¹ | R2 | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-C₂H₂ₛ- | CN | H | H |
| nAm | CₙH2ₙ₊₁ | COOCₘH₂ₘ₊₁ | H | H |
| nOCCF₂.F.F | CₙH₂ₙ₊₁ | OCH₂CF₂H | F | F |

Bevorzugte Mischungskomponenten finden sich in den Tabellen A und B.

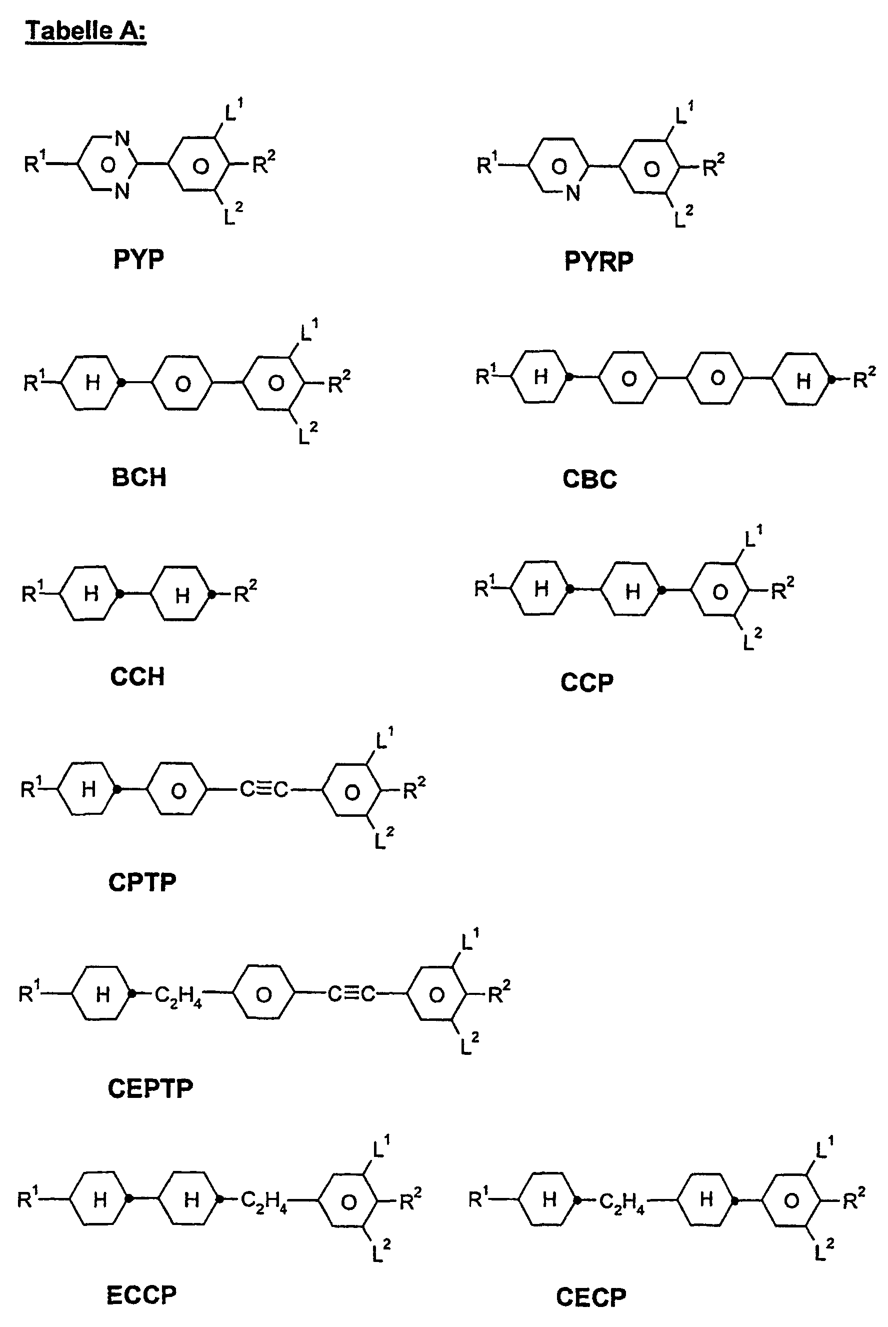

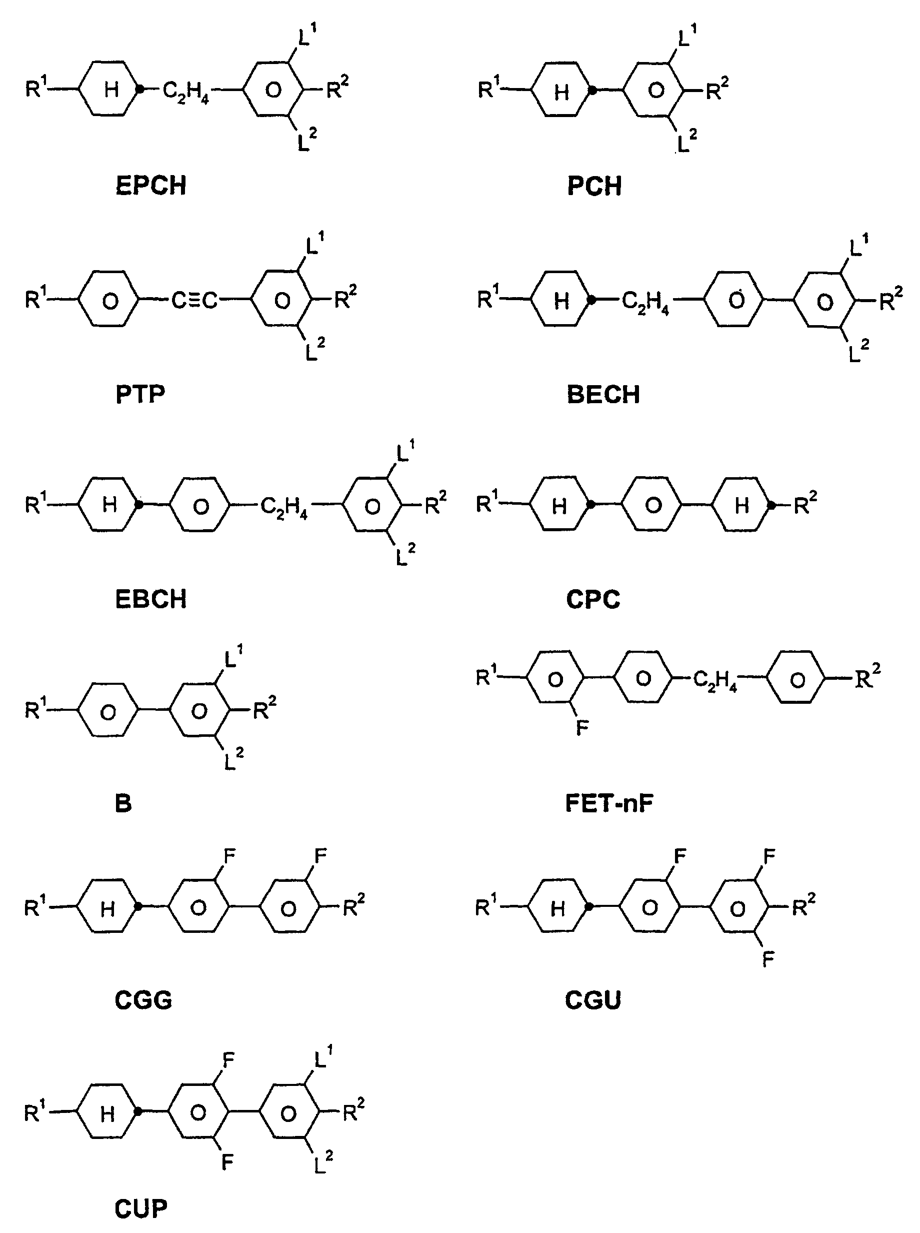

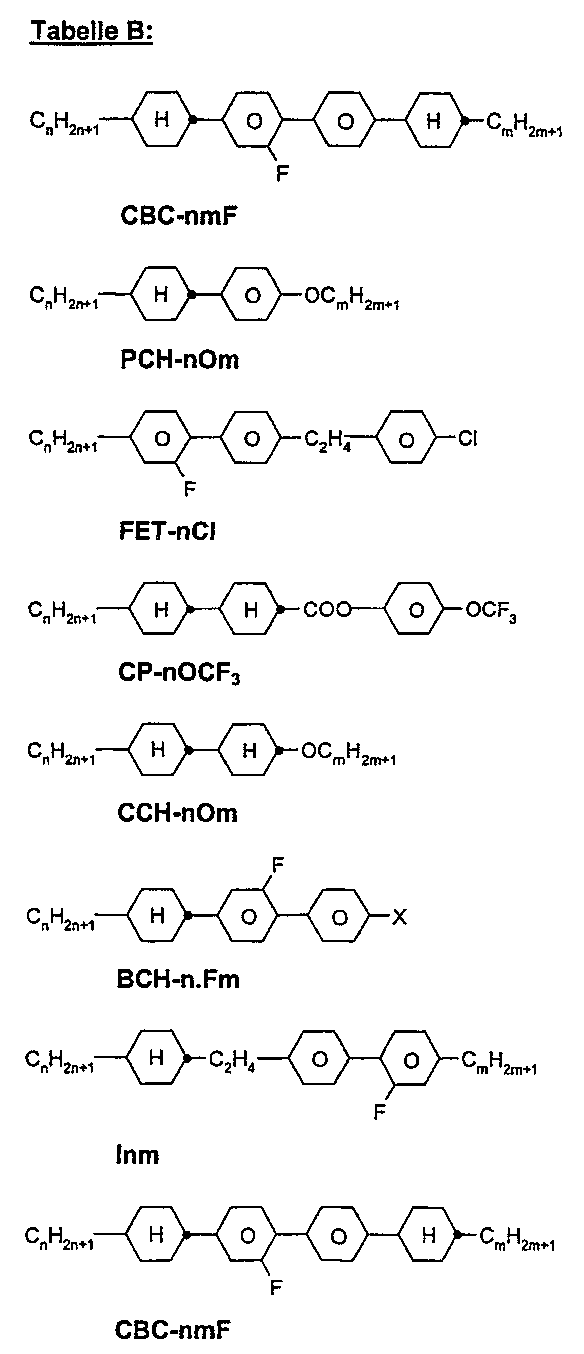

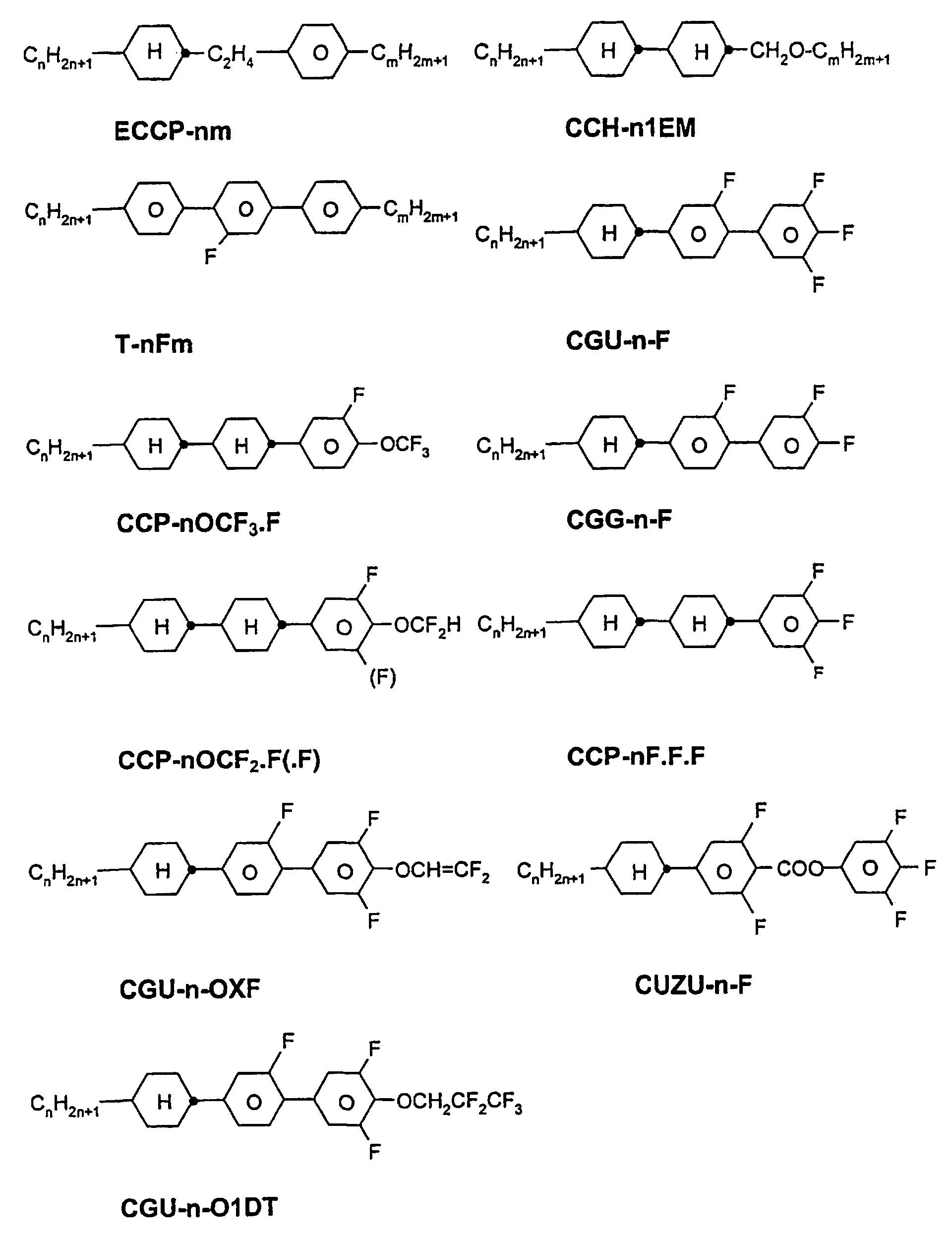

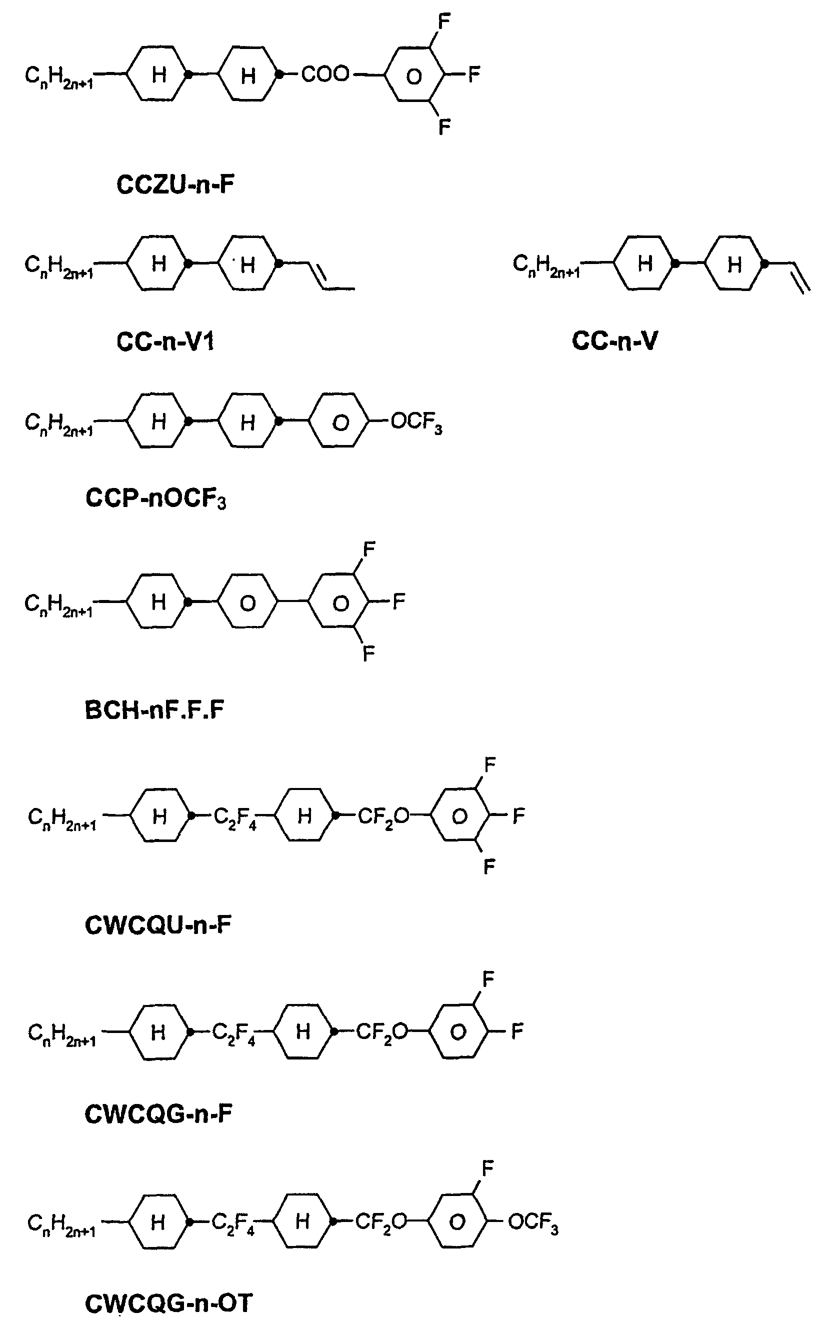

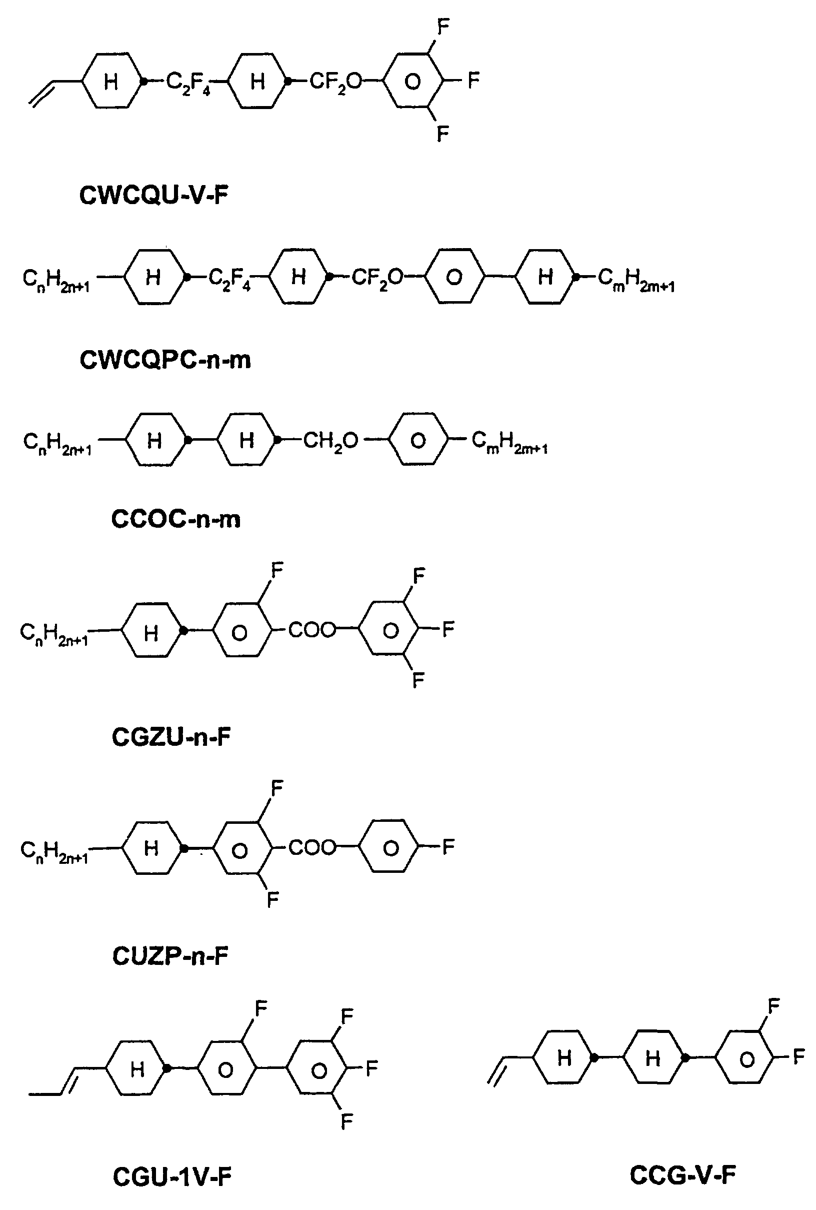

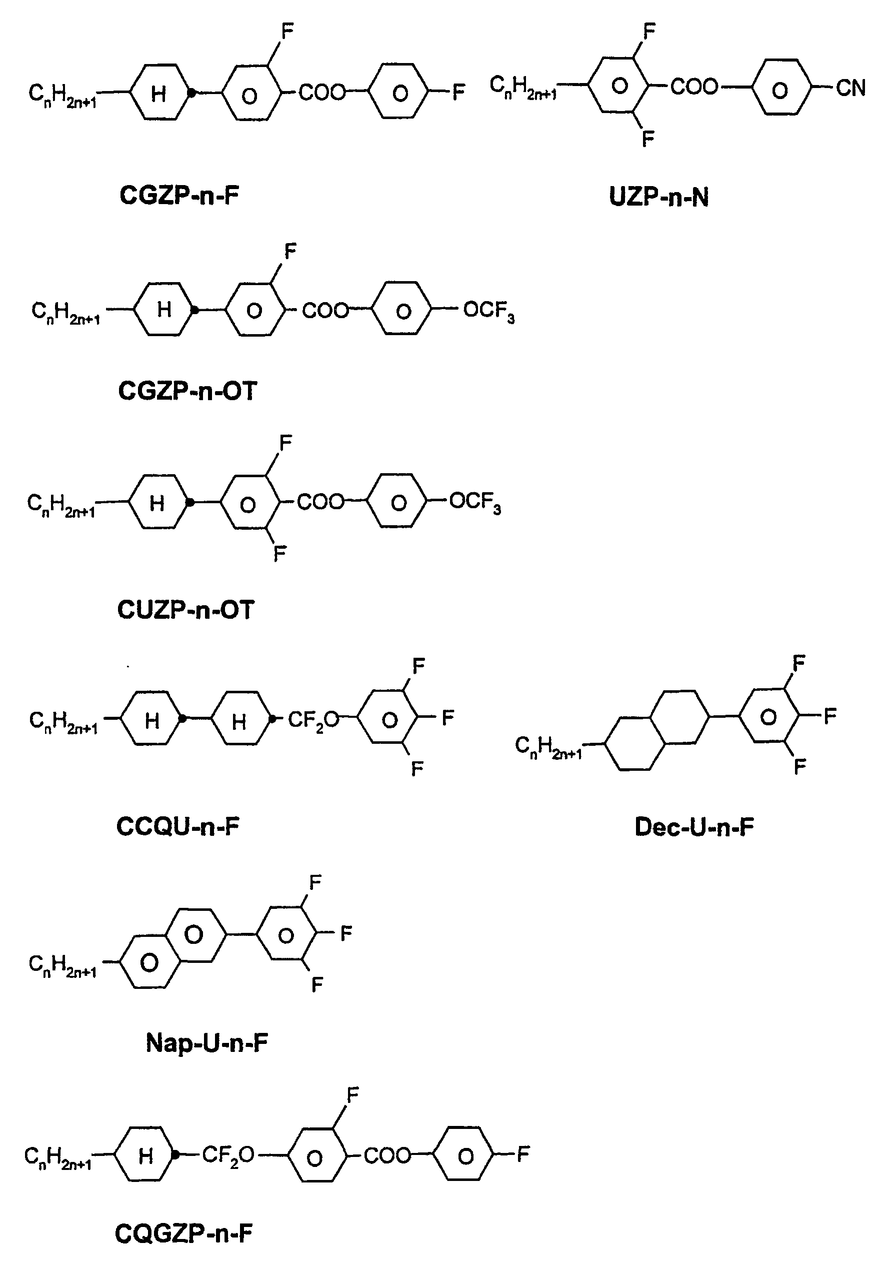

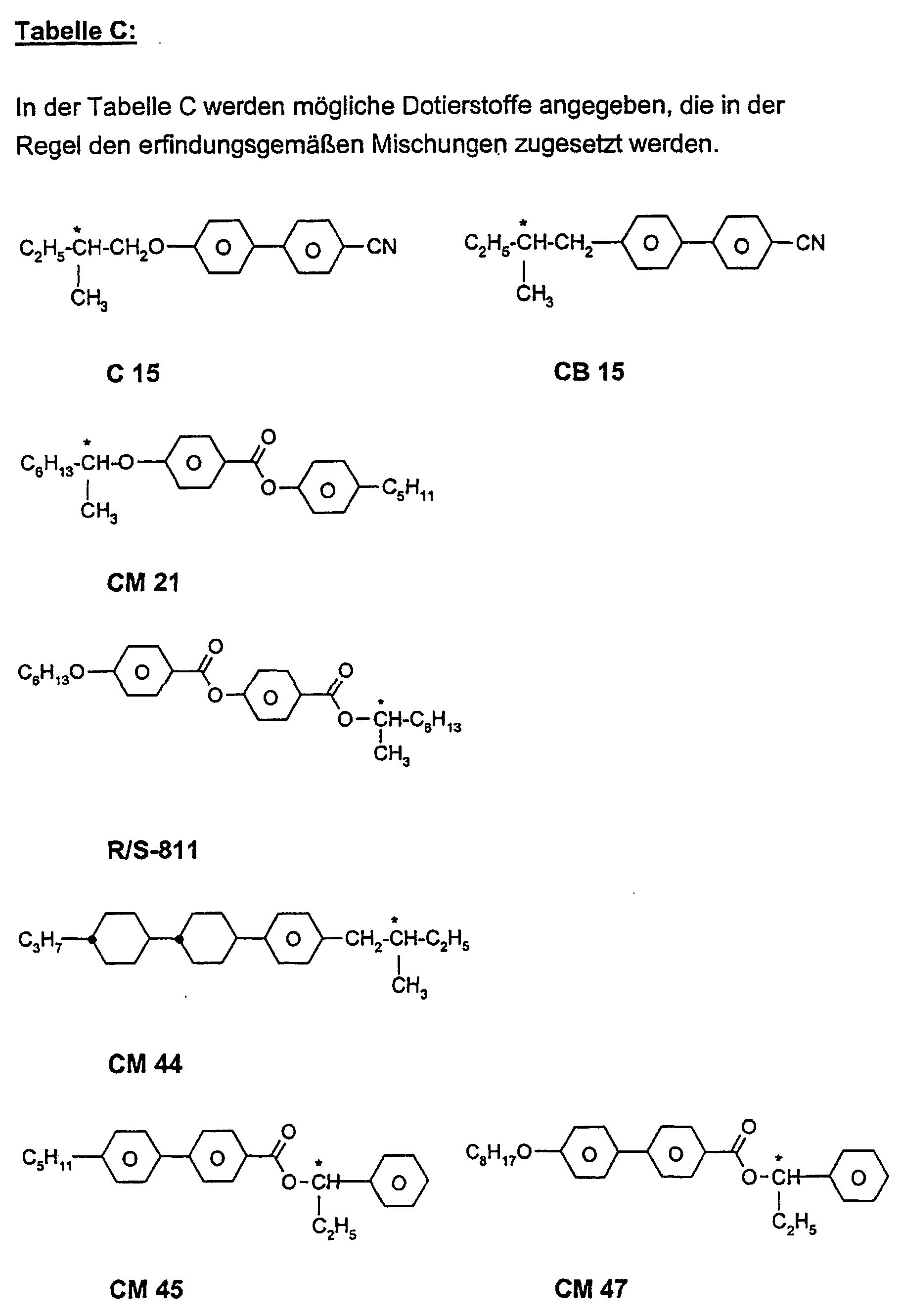

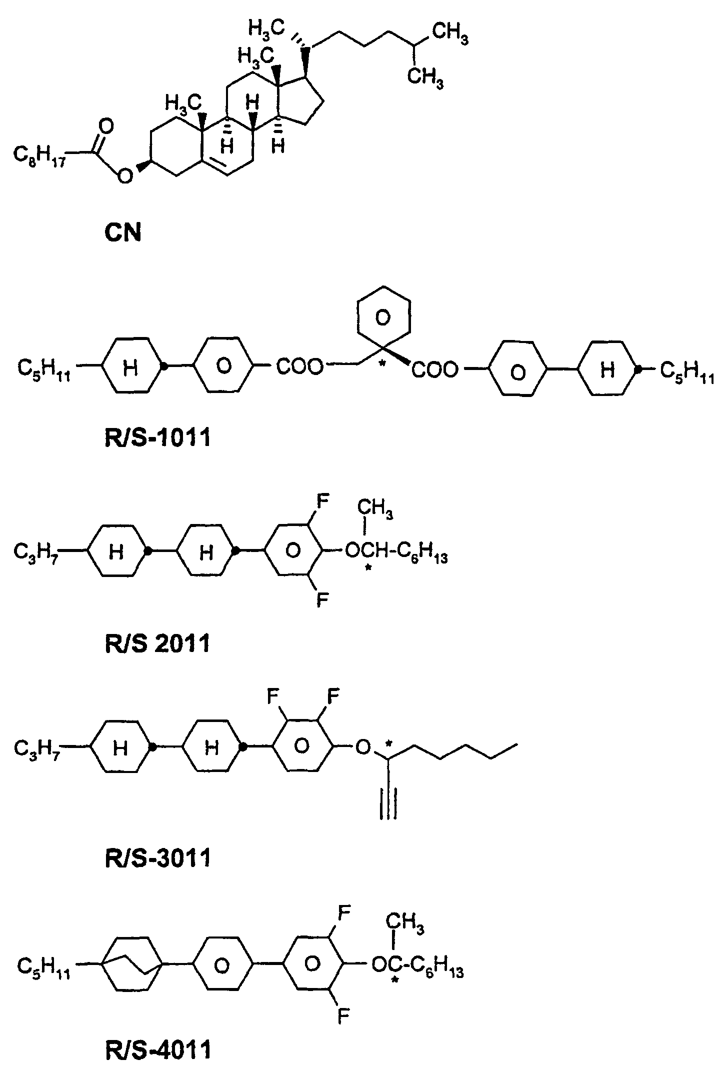

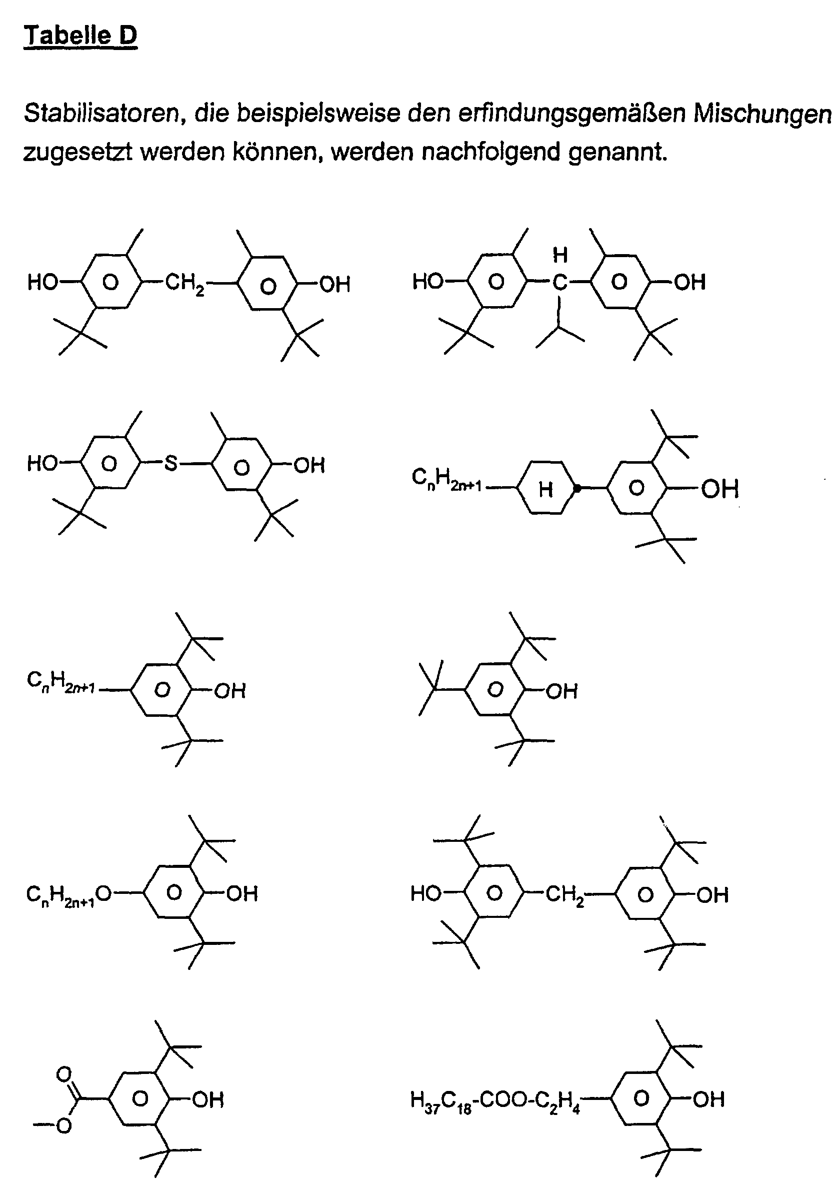

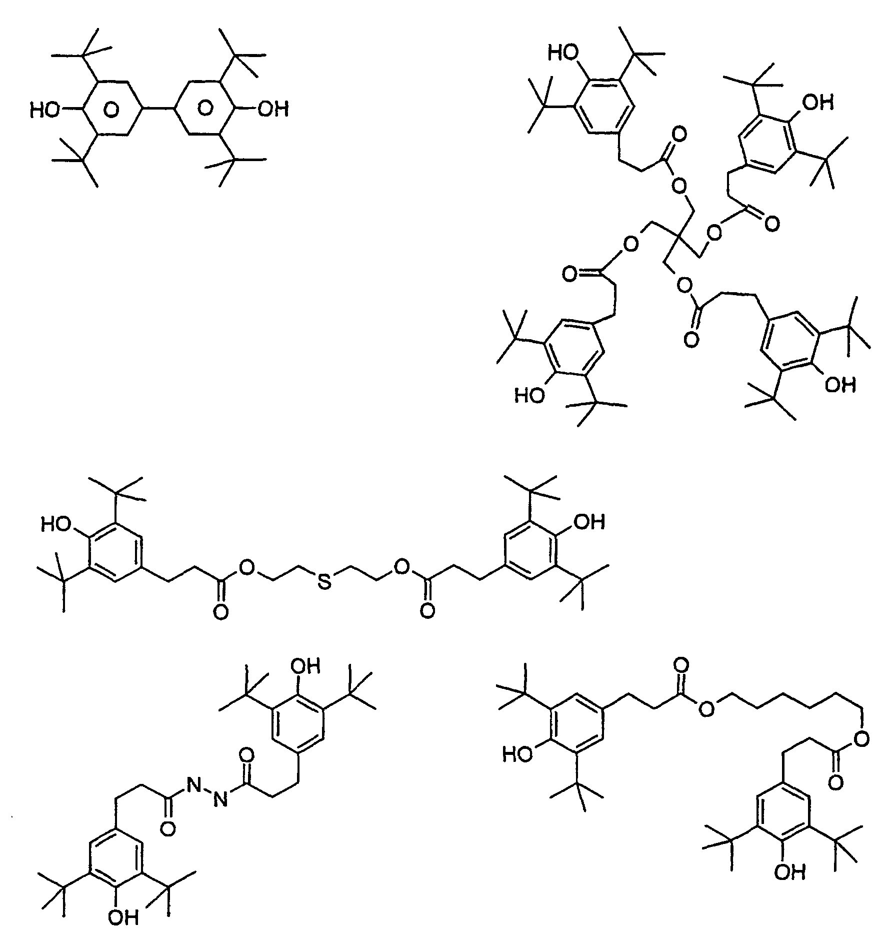

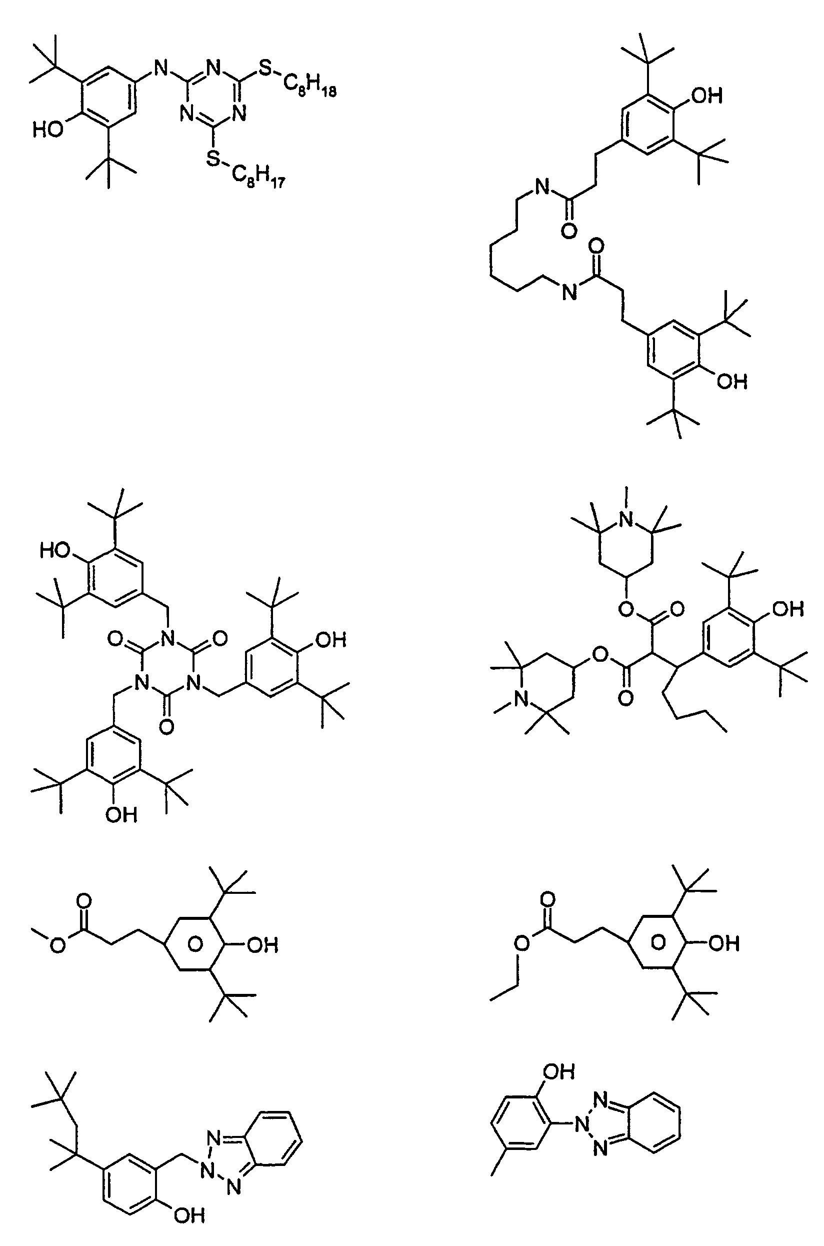

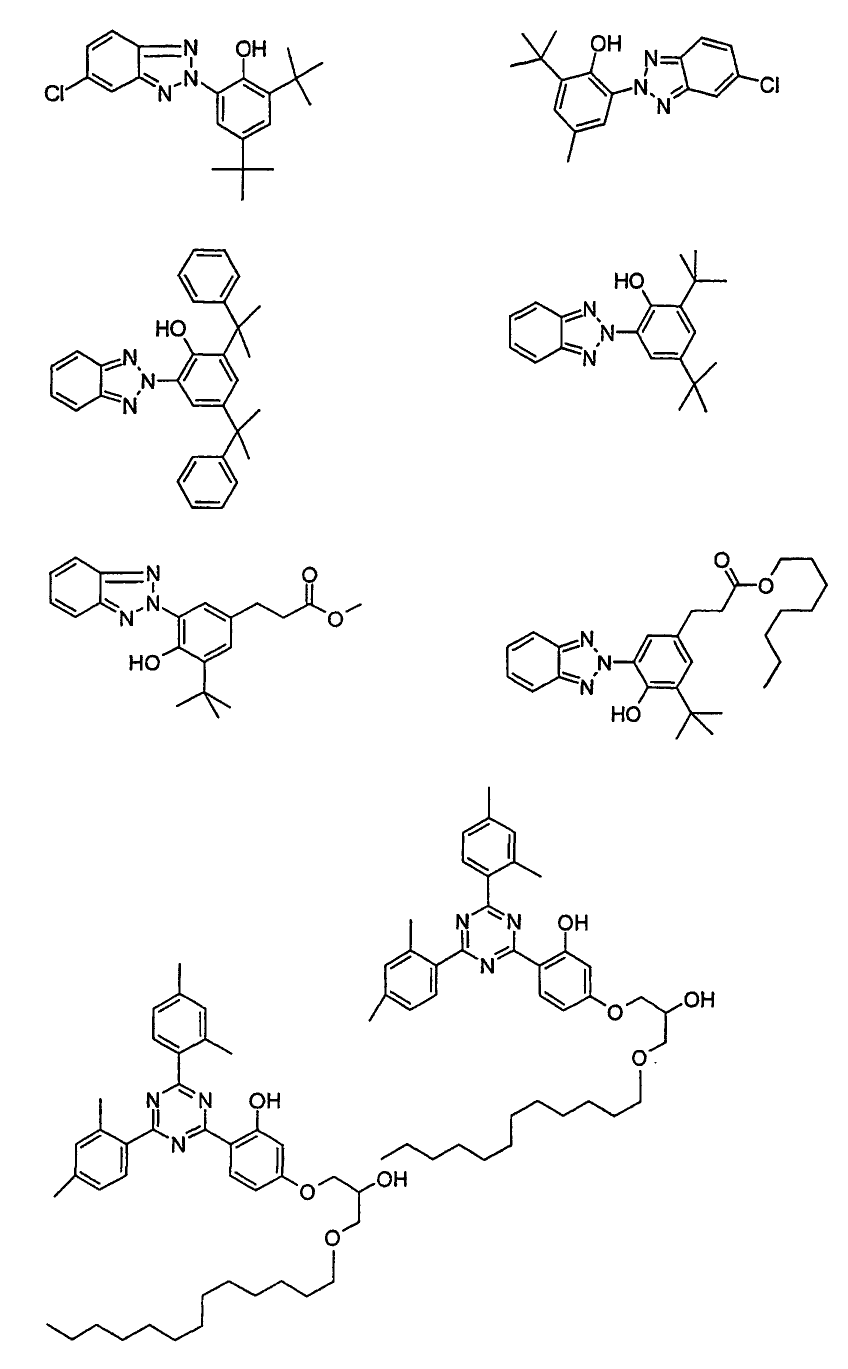

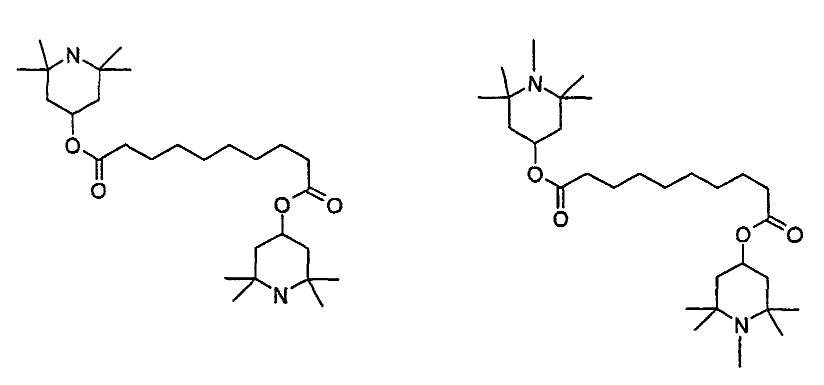

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. Klärpunkt. Femer bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C), die Fließviskosität ν₂₀ (mm²/sec) wurde bei 20 °C bestimmt. Die Rotationsviskosität γ₁ (mPa·s) wurde ebenfalls bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- n-BuLi: 1,6 molare Lösung von n-Butyllithium in n-Hexan
- DMAP: 4-(Dimethytamino)-pyridin
- THF: Tetrahydrofuran
- DCC: N,N'-Dicyclohexylcarbodiimid

### Beispiel 1

### Schritt 1.1

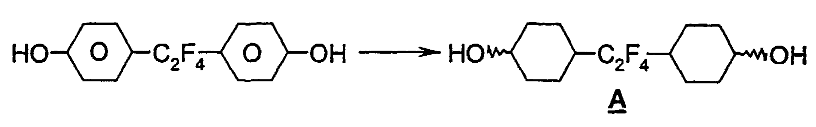

3,494 mol 1,2-Bis-(4-Hydrophenyl)tetrafluorethan werden in 15 l Isopropanol gelöst und mit 400 g 5 % Rhodium-Aktivkohle bei 60 °C und 5 bar hydriert. Nach beendeter Hydrierung wird der Katalysator abfiltriert, das Filtrat eingeengt und der Rückstand aus Ethylacetat umkristallisiert.

### Schritt 1.2

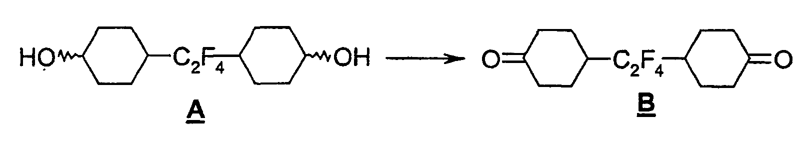

1,5 mol Pyridiniumchlorochromat, 300 g Celite® 545 in 4 I Dichlormethan und 0,97 mol A werden über Nacht bei Raumtemperatur gerührt. Anschließend wird die Suspension abgesaugt, mit Dichlormethan gewaschen und eingeengt. Der Rückstand wird mit 50 ml Petrolether/Essigester (1:1), 1 g Aktivkohle und 5 g Kieselgel gerührt und anschließend über Kieselgel abgesaugt. Das Filtrat wird in Dichlormethan gelöst und mit 25 g Schwefeltrioxid-Pyridin-Komplex über Nacht gerührt. Zuletzt wird mit Wasser extrahiert und über Kieselgel gefrittet. Mp.: 125-126 °C (Methylcyclohexan)

### Schritt 1.3

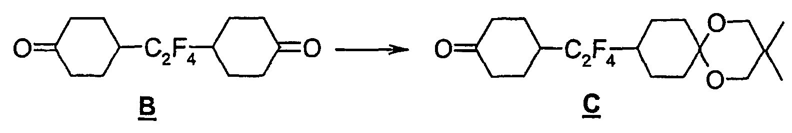

0,075 mol B, 0,230 mol 2,2-Dimethyl-1,3-propan in 250 ml Cyclohexan werden mit 0,05 g Schwefelsäure und 250 ml Wasser 24 h bei 60 °C gerührt. Man lässt auf Raumtemperatur abkühlen und der Bodensatz wird mittels einer Fritte von der flüssigen Phase abgetrennt und getrocknet. Schmelzbereich: 146-158 °C (Methylcyclohexan)

### Schritt 1.4

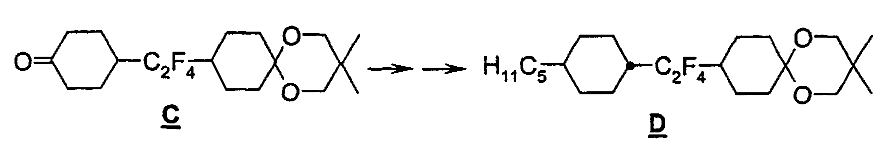

0,121 mol C und 0,145 mol Pentyltriphenylphosphoniumbromid in 400 ml abs. THF werden auf -10 °C abgekühlt und unter Rühren mit 0,160 mol Kalium-tert.-butylat in 100 ml abs. THF versetzt. Die Reaktionslösung wird über Nacht bei Raumtemperatur gerührt. Anschließend wird wie üblich aufgearbeitet. Das Rohprodukt wird mit n-Hexan über Kieselgel gefrittet. Das Filtrat wird eingeengt und der Rückstand wird in 600 ml THF gelöst mit 8 g Pd-C (5 %) versetzt und bei Raumtemperatur und 1 bar hydriert.

### Schritt 1.5

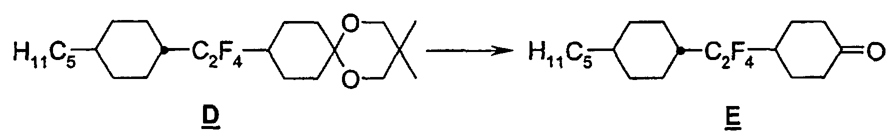

0,041 mol D, 300 ml Toluol und 100 ml Ameisensäure werden 2 Tage bei Raumtemperatur gerührt. Die Ameisensäurephase wird mit Wasser verdünnt, mit Toluol extrahiert und die vereinigten Toluolphasen werden zuletzt wir üblich aufgearbeitet. Schmelzbereich: 64-86,1 °C (Methylcyclohexan)

### Schritt 1.6

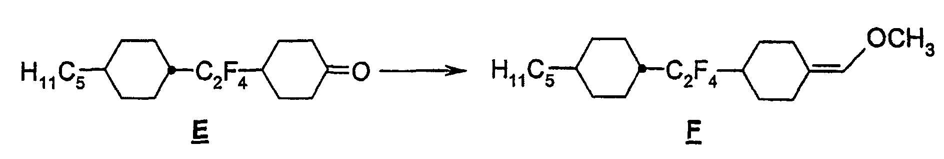

0,105 mol E, 0,119 mol Methoxy-methyl-triphenyl-phosphoniumchlorid werden in 450 ml THF vorgelegt und unter Eiskühlung wird eine Lösung von 0,134 mol Kalium-tert.-butylat in 150 ml THF zugetropft. Das Reaktionsgemisch wird über Nacht bei Raumtemperatur gerührt. Zuletzt wird wie üblich aufgearbeitet.

### Schritt 1.7

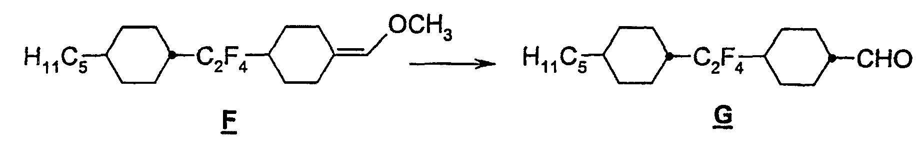

0,061 mol F, 2,915 mol Ameisensäure in 200 ml Toluol werden über Nacht bei Raumtemperatur gerührt. Anschließend wird wie üblich aufgearbeitet.

Für die isomerisierung werden 0,083 mol des Produktes, 600 ml Methanol und 0,135 mol Natronlauge 2 h bei Raumtemperatur gerührt. Anschließend wird weitere 2 h bei -20°C gerührt. Das ausgefallene Produkt wird abgesaugt und mit Methanol gewaschen.

### Schritt 1.8

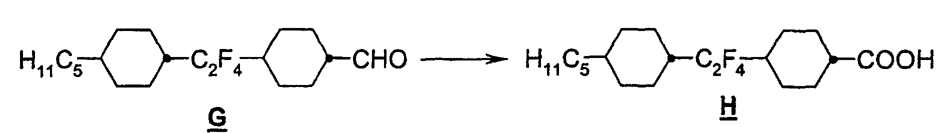

0,042 mol G in 300 ml Aceton werden bei Raumtemperatur mit 0,107 mol Chromsäure versetzt. Man rührt 24 h bei Raumtemperatur. Der Überschuss an CrO_{B} wird mit Isopropanol entfernt. Das Reaktionsgemisch wird zuletzt wie üblich aufgearbeitet. Das Rohprodukt wird bei 0 °C aus Aceton umkristallisiert. K? Sₓ 253 N 258 I

### Schritt 1.9

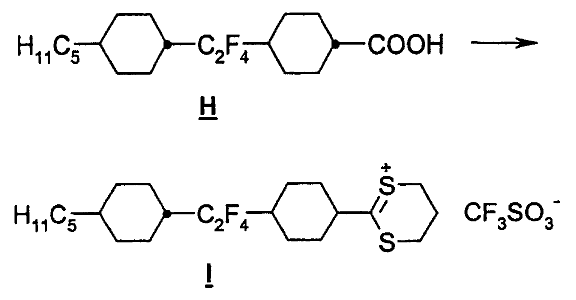

0,018 mol H werden in ein Gemisch aus je 20 ml Toluol und Isooctan suspendiert und zu dieser Suspension werden anschließend 0,022 mol 1,3-Propandithiol zugegeben. Die Suspension wird auf 60 °C erhitzt und danach werden 0,022 mol Trifluormethansulfonsäure zudosiert. Die Reaktionslösung wird 3 h am Wasserabscheider gekocht. Man lässt auf 85 °C abkühlen, versetzt mit 100 ml Dibutylether, wobei das Produkt kristallisiert. Die Kristalle werden mit Methyl-t-Butylether gewaschen und im Vakuum getrocknet. Mp.: 175-182 °C

### Schritt 1.10

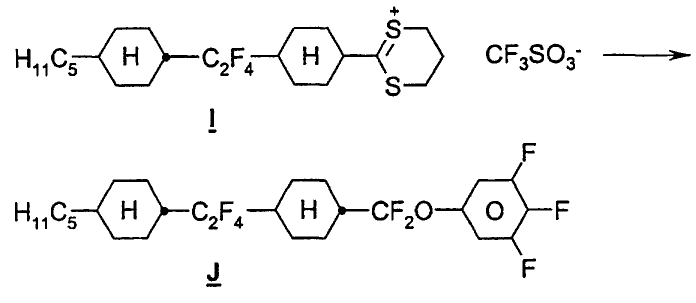

0,012 mol Triflat I werden in 80 ml Dichlormethan vorgelegt und bei -70 °C wird eine Mischung aus 0,029 mol Triethylamin, 0,015 g 3,4,5-Trifluorphenol gelöst in 50 ml Dichlormethan zugetropft. Man lässt das Reaktionsgemisch 1 h bei -70 °C rühren, versetzt mit 0,124 mol Triethylamintrishydrofluorid, rührt weitere 10 min und gibt dann 0,063 mol Brom gelöst in 30 ml Dichlormethan hinzu. Nach 90 min Rühren, lässt man die Reaktionslösung auf -20 °C erwärmen und gießt sie auf 350 ml eines Gemisches aus 1 N NaOH-Lösung und 35 ml ges. NaHSO₃-Lösung. Nach beendeter Gasentwicklung wird die organische Phase abgetrennt und wie üblich aufgearbeitet. Das Produkt wird mit n-Hexan über Kieselgel filtriert und aus Ethanol umkristallisiert. Zuletzt wird das Produkt in n-Heptan gelöst und über Nacht mit Kupferpulver gerührt. Das Gemisch wird eingeengt, wobei das Produkt auskristallisiert.
K 36 S_{B} 123 N 163,9 I; Δε = 9,1; Δn = 0,0815

Analog werden die folgenden Verbindungen der Formel 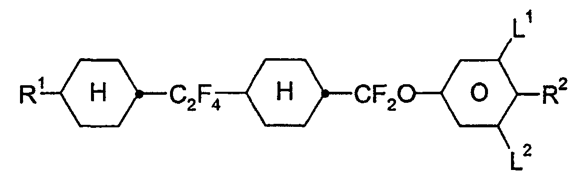 hergestellt:

| R¹ | R² | L¹ | L² | |
|---|---|---|---|---|
| CH₃ | F | H | H | |
| CH₃ | F | F | H | |
| CH₃ | F | F | F | |
| C₂H₅ | F | H | H | |
| C₂H₅ | F | F | H | |
| C₂H₅ | F | F | F | K 33 S_{B} 100 N 140,5 l |
| n-C₃H₇ | F | H | H | |
| n-C₃H₇ | F | F | H | ? -68 S_{B} 149 N 179,0 I; Δε = 6,2; Δn = 0,0865 |
| n-C₃H₇ | F | F | F | K 49 S_{B} 114 N 164,4 I; Δε = 9,9; Δn = 0,0825 |
| n-C₄H₉ | F | H | H | |
| n-C₄H₉ | F | F | H | |
| n-C₄H₉ | F | F | F | |
| n-C₅H₁₁ | F | H | H | |
| n-C₅H₁₁ | F | F | H | K-15 S_{B} 157 N 179,3 I; Δε = 6.4; Δn = 0,0835 |
| n-C₆H₁₃ | F | H | H | |
| n-C₆H₁₃ | F | F | H | |
| n-C₆H₁₃ | F | F | F | |
| n-C₇H₁₃ | F | H | H | |
| n-C₇H₁₃ | F | F | H | |
| n-C₇H₁₃ | F | F | F | |
| CH₂=CH | F | H | H | |
| CH₂=CH | F | F | H | |
| CH₂=CH | F | F | F | K 61 S_{?} (58) N 152,5 l, Δε = 10,0; Δn = 0,0779 |
| CH₃CH=CH | F | H | H | |
| CH₃CH=CH | F | F | H | |
| CH₃CH=CH | F | F | F | |
| CH₂=CHC₂H₄ | F | H | H | |
| CH₂=CHC₂H₄ | F | F | H | |
| CH₂=CHC₂H₄ | F | F | F | |
| CH₃CH=CHC₂H₄ | F | H | H | |
| CH₃CH=CHC₂H₄ | F | F | H | |
| CH₃CH=CHC₂H₄ | F | F | F | |
| (CH₃)₂CH | F | H | H | |
| (CH₃)₂CH | F | F | H | |
| (CH₃)₂CH | F | F | F | |
| (CH₃)₂CHCH₂ | F | H | H | |
| (CH₃)₂CHCH₂ | F | F | H | |
| (CH₃)₂CHCH₂ | F | F | F | |
| CH₃ | OCF₃ | H | H | |
| CH₃ | OCF₃ | F | H | |
| GH₃ | OCF₃ | F | F | |
| C₂H₅ | OCF₃ | H | H | |
| C₂H₅ | OCF₃ | F | H | |
| C₂H₅ | OCF₃ | F | F | |
| n-C₃H₇ | OCF₃ | H | H | |
| n-C₃H₇ | OCF₃ | F | H | K? -48 S_{B} 160 N 184,4 I; Δε = 8,3; Δn = 0,0866 |
| n-C₃H₇ | OCF₃ | F | F | |
| n-C₄H₉ | OCF₃ | H | H | |
| n-C₄H₉ | OCF₃ | F | H | |
| n-C₄H₉ | OCF₃ | F | F | |
| n-C₅H₁₁ | OCF₃ | H | H | |
| n-C₅H₁₁ | OCF₃ | F | H | |
| n-C₅H₁₁ | OCF₃ | F | F | |
| n-C₆H₁₃ | OCF₃ | H | H | |
| n-C₆H₁₃ | OCF₃ | F | H | |
| n-C₆H₁₃ | OCF₃ | F | F | |
| n-C₇H₁₅ | OCF₃ | H | H | |
| n-C₇H₁₅ | OCF₃ | F | H | |
| n-C₇H₁₅ | OCF₃ | F | F | |
| CH₂=CH | OCF₃ | H | H | |
| CH₂=CH | OCF₃ | F | H | |
| CH₂=CH | OCF₃ | F | F | |
| CH₃CH=CH | OCF₃ | H | H | |
| CH₃CH=CH | OCF₃ | F | H | |
| CH₃CH=CH | OCF₃ | F | F | |
| CH₂=CHC₂H₄ | OCF₃ | H | H | |
| CH₂=CHC₂H₄ | OCF₃ | F | H | |
| CH₂₌CHC₂H₄ | OCF₃ | F | F | |
| CH₃CH=CHC₂H₄ | OCF₃ | H | H | |
| CH₃CH=CHC₂H₄ | OCF₃ | F | H | |
| CH₃CH=CHC₂H₄ | OCF₃ | F | F | |
| (CH₃)₂CH | OCF₃ | H | H | |
| (CH₃)₂CH | OCF₃ | F | H | |
| (CH₃)₂CH | OCF₃ | F | F | |
| (CH₃)₂CHCH₂ | OCF₃ | H | H | |
| (CH₃)₂CHCH₂ | OCF₃ | F | H | |
| (CH₃)₂CHCH₂ | OCF₃ | F | F | |
| CH₃ | OCHFCF₃ | H | H | |
| CH₃ | OCHFCF₃ | F | H | |
| CH₃ | OCHFCF₃ | F | F | |
| C₂H₅ | OCHFCF₃ | H | H | |
| C₂H₅ | OCHFCF₃ | F | H | |
| C₂H₅ | OCHFCF₃ | F | F | |
| n-C₃H₇ | OCHFCF₃ | H | H | |
| n-C₃H₇ | OCHFCF₃ | F | H | |
| n-C₃H₇ | OCHFCF₃ | F | F | |
| n-C₄H₉ | OCHFCF₃ | H | H | |
| n-C₄H₉ | OCHFCF₃ | F | H | |
| n-C₄Hg | OCHFCF₃ | F | F | |
| n-C₅H₁₁ | OCHFCF₃ | H | H | |
| n-C₅H₁₁ | OCHFCF₃ | F | H | |
| n-C₅H₁₁ | OCHFCF₃ | F | F | |
| n-C₆H₁₃ | OCHFCF₃ | H | H | |
| n-C₆H₁₃ | OCHFCF₃ | F | H | |
| n-C₆H₁₃ | OCHFCF₃ | F | F | |
| CH₂=CH | OCHFCF₃ | H | H | |
| CH₂=CH | OCHFCF₃ | F | H | |
| CH₂=CH | OCHFCF₃ | F | F | |
| CH₃CH=CH | OCHFCF₃ | H | H | |
| CH₃CH=CH | OCHFCF₃ | F | H | |
| CH₃CH=CH | OCHFCF₃ | F | F | |
| CH₂=CHC₂H₄ | OCHFCF₃ | H | H | |
| CH₂=CHC₂H₄ | OCHFCF₃ | F | H | |
| CH₂=CHC₂H₄ | OCHFCF₃ | F | F | |
| CH₃CH=CHC₂Hₐ | OCHFCF₃ | H | H | |
| CH₃CH=CHC₂H₄ | OCHFCF₃ | F | H | |
| CH₃CH=CHC₂H₄ | OCHFCF₃ | F | F | |
| (CH₃)₂CH | OCHFCF₃ | H | H | |
| (CH₃)₂CH | OCHFCF₃ | F | H | |
| (CH₃)₂CH | OCHFCF₃ | F | F | |
| (CH₃)₂CHCH₂ | OCHFCF₃ | H | H | |
| (CH₃)₂CHCH₂ | OCHFCF₃ | F | H | |
| (CH₃)₂CHCH₂ | OCHFCF₃ | F | F | |
| CH₃ | OCF₂CHFCF₃ | H | H | |
| CH₃ | OCF₂CHFCF₃ | F | H | |
| CH₃ | OCF₂CHFCF₃ | F | F | |
| C₂H₅ | OCF₂CHFCF₃ | H | H | |
| C₂H₅ | OCF₂CHFCF₃ | F | H | |
| C₂H₅ | OCF₂CHFCF₃ | F | F | |
| n-C₃H₇ | OCF₂CHFCF₃ | H | H | |
| n-C₃H₇ | OCF₂CHFCF₃ | F | H | |
| n-C₃H₇ | OCF₂CHFCF₃ | F | F | |
| n-C₄H₉ | OCF₂CHFCF₃ | H | H | |
| n-C₄H₉ | OCF₂CHFCF₃ | F | H | |
| n-C₄H₉ | OCF₂CHFCF₃ | F | F | |
| n-C₅H₁₁ | OCF₂CHFCF₃ | H | H | |
| n-C₅H₁₁ | OCF₂CHFCF₃ | F | H | |
| n-C₅H₁₁ | OCF₂CHFCF₃ | F | F | |
| n-C₆H₁₃ | OCF₂CHFCF₃ | H | H | |
| n-C₆H₁₃ | OCF₂CHFCF₃ | F | H | |
| n-C₆H₁₃ | OCF₂CHFCF₃ | F | F | |
| CH₂=CH | OCF₂CHFCF₃ | H | H | |
| CH₂=CH | OCF₂CHFCF₃ | F | H | |
| CH₂=CH | OCF₂CHFCF₃ | F | F | |
| CH₃CH=CH | OCF₂CHFCF₃ | H | H | |
| CH₃CH=CH | OCF₂CHFCF₃ | F | H | |
| CH₃CH=CH | OCF₂CHFCF₃ | F | F | |
| CH₂=CHC₂H₄ | OCF₂CHFCF₃ | H | H | |
| CH₂=CHC₂H₄ | OCF₂CHFCF₃ | F | H | |
| CH₂=CHC₂H₄ | OCF₂CHFCF₃ | F | F | |
| CH₃CH=CHC₂H₄ | OCF₂CHFCF₃ | H | H | |
| CH₃CH=CHC₂H₄ | OCF₂CHFCF₃ | F | H | |
| CH₃CH=CHC₂H₄ | OCF₂CHFCF₃ | F | F | |
| (CH₃)₂CH | OCF₂CHFCF₃ | H | H | |
| (CH₃)₂CH | OCF₂CHFCF₃ | F | H | |
| (CH₃)₂CH | OCF₂CHFCF₃ | F | F | |
| (CH₃)₂CHCH₂ | OCF₂CHFCF₃ | H | H | |
| (CH₃)₂CHCH₂ | OCF₂CHFCF₃ | F | H | |
| (CH₃)₂CHCH₂ | OCF₂CHFCF₃ | F | F | |
| CH₃ | OCHF₂ | H | H | |
| CH₃ | OCHF₂ | F | H | |
| CH₃ | OCHF₂ | F | F | |
| C₂H₅ | OCHF₂ | H | H | |
| C₂H₅ | OCHF₂ | F | H | |
| C₂H₅ | OCHF₂ | F | F | |
| n-C₃H₇ | OCHF₂ | H | H | |
| n-C₃H₇ | OCHF₂ | F | H | |
| n-C₃H₇ | OCHF₂ | F | F | |
| n-C₄H₉ | OCHF₂ | H | H | |
| n-C₄H₉ | OCHF₂ | F | H | |
| n-C₄H₉ | OCHF₂ | F | F | |
| n-C₅H₁₁ | OCHF₂ | H | H | |
| n-C₅H₁₁ | OCHF₂ | F | H | |
| n-C₅H₁₁ | OCHF₂ | F | F | |
| n-C₆H₁₃ | OCHF₂ | H | H | |
| n-C₆H₁₃ | OCHF₂ | F | H | |
| n-C₆H₁₃ | OCHF₂ | F | F | |
| CH₂=CH | OCHF₂ | H | H | |
| CH₂=CH | OCHF₂ | F | H | |
| CH₂=CH | OCHF₂ | F | F | |
| CH₃CH=CH | OCHF₂ | H | H | |
| CH₃CH=CH | OCHF₂ | F | H | |
| CH₃CH=CH | OCHF₂ | F | F | |
| CH₂=CHC₂H₄ | OCHF₂ | H | H | |
| CH₂=CHC₂H₄ | OCHF₂ | F | H | |
| CH₂=CHC₂H₄ | OCHF₂ | F | F | |
| CH₃CH=CHC₂H₄ | OCHF₂ | H | H | |
| CH₃CH=CHC₂H₄ | OCHF₂ | F | H | |
| CH₃CH=CHC₂H₄ | OCHF₂ | F | F | |
| (CH₃)₂CH | OCHF₂ | H | H | |
| (CH₃)₂CH | OCHF₂ | F | H | |
| (CH₃)₂CH | OCHF₂ | F | F | |
| (CH3)₂CHCH₂ | OCHF₂ | H | H | |
| (CH₃)₂CHCH₂ | OCHF₂ | F | H | |
| (CH₃)₂CHCH₂ | OCHF₂ | F | F | |
| CH₃ | CF₃ | H | H | |
| CH₃ | CF₃ | F | H | |
| CH₃ | CF₃ | F | F | |
| C₂H₅ | CF₃ | H | H | |
| C₂H₅ | CF₃ | F | H | |
| C₂H₅ | CF₃ | F | F | |
| n-C₃H₇ | CF₃ | H | H | |
| n-C₃H₇ | CF₃ | F | H | |
| n-C₃H₇ | CF₃ | F | F | |
| n-C₄H₉ | CF₃ | H | H | |
| n-C₄H₉ | CF₃ | F | H | |
| n-C₄H₉ | CF₃ | F | F | |
| n-C₅H₁₁ | CF₃ | H | H | |
| n-C₅H₁₁ | CF₃ | F | H | |
| n-C₅H₁₁ | CF₃ | F | F | |
| n-C₆H₁₃ | CF₃ | H | H | |
| n-C₆H₁₃ | CF₃ | F | H | |
| n-C₆H₁₃ | CF₃ | F | F | |
| CH₂=CH | CF₃ | H | H | |
| CH₂=CH | CF₃ | F | H | |
| CH₂=CH | CF₃ | F | F | |
| CH₃CH=CH | CF₃ | H | H | |
| CH₃CH=CH | CF₃ | F | H | |
| CH₃CH=CH | CF₃ | F | F | |
| CH₂=CHC₂H₄ | CF₃ | H | H | |
| CH₂=CHC₂H₄ | CF₃ | F | H | |
| CH₂=CHC₂H₄ | CF₃ | F | F | |
| CH₃CH=CHC₂H₄ | CF₃ | H | H | |
| CH₃CH=CHC₂H₄ | CF₃ | F | H | |
| CH₃CH=CHC₂H₄ | CF₃ | F | F | |
| (CH₃)₂CH | CF₃ | H | H | |
| (CH₃)₂CH | CF₃ | F | H | |
| (CH₃)₂CH | CF₃ | F | F | |
| (CH₃)₂CHCH₂ | CF₃ | H | H | |
| (CH₃)₂CHCH₂ | CF₃ | F | H | |
| (CH₃)₂CHCH₂ | CF₃ | F | F | |
| CH₃ | CN | H | H | |
| CH₃ | CN | F | H | |
| CH₃ | CN | F | F | |
| C₂H₅ | CN | H | H | |
| C₂H₅ | CN | F | H | |
| C₂H₅ | CN | F | F | |
| n-C₃H₇ | CN | H | H | |
| n-C₃H₇ | CN | F | H | |
| n-C₃H₇ | CN | F | F | |
| n-C₄H₉ | CN | H | H | |
| n-C₄H₉ | CN | F | H | |
| n-C₄H₉ | CN | F | F | |
| n-C₅H₁₁ | CN | H | H | |
| n-C₅H₁₁ | CN | F | H | |
| n-C₅H₁₁ | CN | F | F | |
| n-C₆H₁₃ | CN | H | H | |
| n-C₆H₁₃ | CN | F | H | |
| n-C₆H₁₃ | CN | F | F | |
| CH₂=CH | CN | H | H | |
| CH₂=CH | CN | F | H | |
| CH₂=CH | CN | F | F | |
| CH₃CH=CH | CN | H | H | |
| CH₃CH=CH | CN | F | H | |
| CH₃CH=CH | CN | F | F | |
| CH₂=CHC₂H₄ | CN | H | H | |
| CH₂=CHC₂H₄ | CN | F | H | |
| CH₂=CHC₂H₄ | CN | F | F | |
| CH₃CH=CHC₂H₄ | CN | H | H | |
| CH₃CH=CHC₂H₄ | CN | F | H | |
| CH₃CH=CHC₂H₄ | CN | F | F | |
| (CH₃)₂CH | CN | H | H | |
| (CH₃)₂CH | CN | F | H | |
| (CH₃)₂CH | CN | F | F | |
| (CH₃)₂CHCH₂ | CN | H | H | |
| (CH₃)₂CHCH₂ | CN | F | H | |
| (CH₃)₂CHCH₂ | CN | F | F | |
| CH₃ | SF₅ | H | H | |
| CH₃ | SF₅ | F | H | |
| CH₃ | SF₅ | F | F | |
| C₂H₅ | SF₅ | H | H | |
| C₂H₅ | SF₅ | F | H | |
| C₂H₅ | SF₅ | F | F | |
| n-C₃H₇ | SF₅ | H | H | |
| n-C₃H₇ | SF₅ | F | H | |
| n-C₃H₇ | SF₅ | F | F | |
| n-C₄H₉ | SF₅ | H | H | |
| n-C₄H₉ | SF₅ | F | H | |
| n-C₄H₉ | SF₅ | F | F | |
| n-C₅H₁₁ | SF₅ | H | H | |
| n-C₅H₁₁ | SF₅ | F | H | |
| n-C₅H₁₁ | SF₅ | F | F | |
| n-C₆H₁₃ | SF₅ | H | H | |
| n-C₆H₁₃ | SF₅ | F | H | |
| n-C₆H₁₃ | SF₅ | F | F | |
| CH₂=CH | SF₅ | H | H | |
| CH₂=CH | SF₅ | F | H | |
| CH₂=CH | SF₅ | F | F | |
| CH₃CH=CH | SF₅ | H | H | |
| CH₃CH=CH | SF₅ | F | H | |
| CH₃CH=CH | SF₅ | F | F | |
| CH₂=CHC₂H₄ | SF₅ | H | H | |
| CH₂=CHC₂H₄ | SF₅ | F | H | |
| CH₂=CHC₂H₄ | SF₅ | F | F | |
| CH₃CH=CHC₂H₄ | SF₅ | H | H | |
| CH₃CH=CHC₂H₄ | SF₅ | F | H | |
| CH₃CH=CHC₂H₄ | SF₅ | F | F | |
| (CH₃)₂CH | SF₅ | H | H | |
| (CH₃)₂CH | SF₅ | F | H | |
| (CH₃)₂CH | SF₅ | F | F | |
| (CH₃)₂CHCH₂ | SF₅ | H | H | |
| (CH₃)₂CHCH₂ | SF₅ | F | H | |
| (CH₃)₂CHCH₂ | SF₅ | F | F | |
| CH₃ | OCH=CF₂ | H | H | |
| CH₃ | OCH=CF₂ | F | H | |
| CH₃ | OCH=CF₂ | F | F | |
| C₂H₅ | OCH=CF₂ | H | H | |
| C₂H₅ | OCH=CF₂ | F | H | |
| C₂H₅ | OCH=CF₂ | F | F | |
| n-C₃H₇ | OCH=CF₂ | H | H | |
| n-C₃H₇ | OCH=CF₂ | F | H | |
| n-C₃H₇ | OCH=CF₂ | F | F | |
| n-C₄H₉ | OCH=CF₂ | H | H | |
| n-C₄H₉ | OCH=CF₂ | F | H | |
| n-C₄H₉ | OCH=CF₂ | F | F | |
| n-C₅H₁₁ | OCH=CF₂ | H | H | |
| n-C₅H₁₁ | OCH=CF₂ | F | H | |
| n-C₅H₁₁ | OCH=CF₂ | F | F | |
| n-C₆H₁₃ | OCH=CF₂ | H | H | |
| n-C₆H₁₃ | OCH=CF₂ | F | H | |
| n-C₆H₁₃ | OCH=CF₂ | F | F | |
| CH₂=CH | OCH=CF₂ | H | H | |
| CH₂=CH | OCH=CF₂ | F | H | |
| CH₂=CH | OCH=CF₂ | F | F | |
| CH₃CH=CH | OCH=CF₂ | H | H | |
| CH₃CH=CH | OCH=CF₂ | F | H | |
| CH₃CH=CH | OCH=CF₂ | F | F | |
| CH₂=CHC₂H₄ | OCH=CF₂ | H | H | |
| CH₂=CHC₂H₄ | OCH=CF₂ | F | H | |
| CH₂=CHC₂H₄ | OCH=CF₂ | F | F | |
| CH₃CH=CHC₂H₄ | OCH=CF₂ | H | H | |
| CH₃CH=CHC₂H₄ | OCH=CF₂ | F | H | |
| CH₃CH=CHC₂H₄ | OCH=CF₂ | F | F | |
| (CH₃)₂CH | OCH=CF₂ | H | H | |
| (CH₃)₂CH | OCH=CF₂ | F | H | |
| (CH₃)₂CH | OCH=CF₂ | F | F | |
| (CH₃)₂CHCH₂ | OCH=CF₂ | H | H | |
| (CH₃)₂CHCH₂ | OCH=CF₂ | F | H | |
| (CH₃)₂CHCH₂ | OCH=CF₂ | F | F | |
| CH₃ | CH=CF₂ | H | H | |
| CH₃ | CH=CF₂ | F | H | |
| CH₃ | CH=CF₂ | F | F | |
| C₂H₅ | CH=CF₂ | H | H | |
| C₂H₅ | CH=CF₂ | F | H | |
| C₂H₅ | CH=CF₂ | F | F | |
| n-C₃H₇ | CH=CF₂ | H | H | |
| n-C₃H₇ | CH=CF₂ | F | H | |
| n-C₃H₇ | CH=CF₂ | F | F | |
| n-C₄H₉ | CH=CF₂ | H | H | |
| n-C₄H₉ | CH=CF₂ | F | H | |
| n-C₄H₉ | CH=CF₂ | F | F | |
| n-C₅H₁₁ | CH=CF₂ | H | H | |
| n-C₅H₁₁ | CH=CF₂ | F | H | |
| n-C₅H₁₁ | CH=CF₂ | F | F | |
| n-C₆H₁₃ | CH=CF₂ | H | H | |
| n-C₆H₁₃ | CH=CF₂ | F | H | |
| n-C₆H₁₃ | CH=CF₂ | F | F | |
| CH₂=CH | CH=CF₂ | H | H | |
| CH₂=CH | CH=CF₂ | F | H | |
| CH₂=CH | CH=CF₂ | F | F | |
| CH₃CH=CH | CH=CF₂ | H | H | |
| CH₃CH=CH | CH=CF₂ | F | H | |
| CH₃CH=CH | CH=CF₂ | F | F | |
| CH₂=CHC₂H₄ | CH=CF₂ | H | H | |
| CH₂=CHC₂H₄ | CH=CF₂ | F | H | |
| CH₂=CHC₂H₄ | CH=CF₂ | F | F | |
| CH₃CH=CHC₂H₄ | CH=CF₂ | H | H | |
| CH₃CH=CHC₂H₄ | CH=CF₂ | F | H | |
| CH₃CH=CHC₂H₄ | CH=CF₂ | F | F | |
| (CH₃)₂CH | CH=CF₂ | H | H | |
| (CH₃)₂CH | CH=CF₂ | F | H | |
| (CH₃)₂CH | CH=CF₂ | F | F | |
| (CH₃)₂CHCH₂ | CH=CF₂ | H | H | |
| (CH₃)₂CHCH₂ | CH=CF₂ | F | H | |
| (CH₃)₂CHCH₂ | CH=CF₂ | F | F | |
| CH₃ | CF=CF₂ | H | H | |
| CH₃ | CF=CF₂ | F | H | |
| CH₃ | CF=CF₂ | F | F | |
| C₂H₅ | CF=CF₂ | H | H | |
| C₂H₅ | CF=CF₂ | F | H | |
| C₂H₅ | CF=CF₂ | F | F | |
| n-C₃H₇ | CF=CF₂ | H | H | |
| n-C₃H₇ | CF=CF₂ | F | H | |
| n-C₃H₇ | CF=CF₂ | F | F | |
| n-C₄H₉ | CF=CF₂ | H | H | |
| n-C₄H₉ | CF=CF₂ | F | H | |
| n-C₄H₉ | CF=CF₂ | F | F | |
| n-C₅H₁₁ | CF=CF₂ | H | H | |
| n-C₅H₁₁ | CF=CF₂ | F | H | |
| n-C₅H₁₁ | CF=CF₂ | F | F | |
| n-C₆H₁₃ | CF=CF₂ | H | H | |
| n-C₆H₁₃ | CF=CF₂ | F | H | |
| n-C₆H₁₃ | CF=CF₂ | F | F | |
| CH₂=CH | CF=CF₂ | H | H | |
| CH₂=CH | CF=CF₂ | F | H | |
| CH₂=CH | CF=CF₂ | F | F | |
| CH₃CH=CH | CF=CF₂ | H | H | |
| CH₃CH=CH | CF=CF₂ | F | H | |
| CH₃CH=CH | CF=CF₂ | F | F | |
| CH₂=CHC₂H₄ | CF=CF₂ | H | H | |
| CH₂=CHC₂H₄ | CF=CF₂ | F | H | |
| CH₂=CHC₂H₄ | CF=CF₂ | F | F | |
| CH₃CH=CHC₂H₄ | CF=CF₂ | H | H | |
| CH₃CH=CHC₂H₄ | CF=CF₂ | F | H | |
| CH₃CH=CHC₂H₄ | CF=CF₂ | F | F | |
| (CH₃)₂CH | CF=CF₂ | H | H | |
| (CH₃)₂CH | CF=CF₂ | F | H | |
| (CH₃)₂CH | CF=CF₂ | F | F | |
| (CH₃)₂CHCH₂ | CF=CF₂ | H | H | |
| (CH₃)₂CHCH₂ | CF=CF₂ | F | H | |
| (CH₃)₂CHCH₂ | CF=CF₂ | F | F | |
| CH₃ | OCF=CF₂ | H | H | |
| CH₃ | OCF=CF₂ | F | H | |
| CH₃ | OCF=CF₂ | F | F | |
| C₂H₅ | OCF=CF₂ | H | H | |
| C₂H₅ | OCF=CF₂ | F | H | |
| C₂H₅ | OCF=CF₂ | F | F | |
| n-C₃H7 | OCF=CF₂ | H | H | |
| n-C₃H₇ | OCF=CF₂ | F | H | |
| n-C₃H₇ | OCF=CF₂ | F | F | |
| n-C₄H₉ | OCF=CF₂ | H | H | |
| n-C₄H₉ | OCF=CF₂ | F | H | |
| n-C₄H₉ | OCF=CF₂ | F | F | |
| n-C₅H₁₁ | OCF=CF₂ | H | H | |
| n-C₅H₁₁ | OCF=CF₂ | F | H | |
| n-C₅H₁₁ | OCF=CF₂ | F | F | |
| n-C₆H₁₃ | OCF=CF₂ | H | H | |
| n-C₆H₁₃ | OCF=CF₂ | F | H | |
| n-C₆H₁₃ | OCF=CF₂ | F | F | |
| CH₂=CH | OCF=CF₂ | H | H | |
| CH₂=CH | OCF=CF₂ | F | H | |
| CH₂=CH | OCF=CF₂ | F | F | |
| CH₃CH=CH | OCF=CF₂ | H | H | |
| CH₃CH=CH | OCF=CF₂ | F | H | |
| CH₃CH=CH | OCF=CF₂ | F | F | |
| CH₂=CHC₂H₄ | OCF=CF₂ | H | H | |
| CH₂=CHC₂H₄ | OCF=CF₂ | F | H | |
| CH₂=CHC₂H₄ | OCF=CF₂ | F | F | |
| CH₃CH=CHC₂H₄ | OCF=CF₂ | H | H | |
| CH₃CH=CHC₂H₄ | OCF=CF₂ | F | H | |
| CH₃CH=CHC₂H₄ | OCF=CF₂ | F | F | |
| (CH₃)₂CH | OCF=CF₂ | H | H | |
| (CH₃)₂CH | OCF=CF₂ | F | H | |
| (CH₃)₂CH | OCF=CF₂ | F | F | |
| (CH₃)₂CHCH₂ | OCF=CF₂ | H | H | |
| (CH₃)₂CHCH₂ | OCF=CF₂ | F | H | |
| (CH₃)₂CHCH₂ | OCF=CF₂ | F | F | |

### Beispiel 2

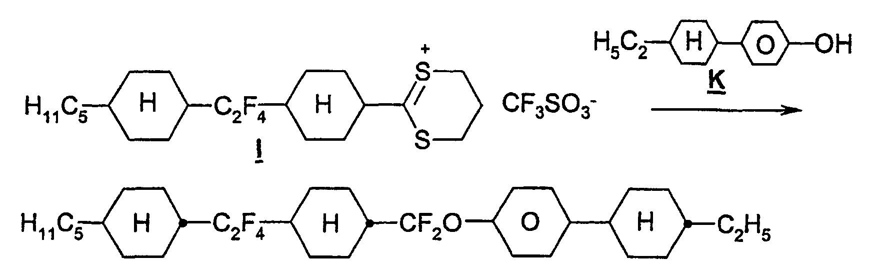

0,014 mol Triflat I (aus Schritt 1.9) werden in 80 ml Dichlormethan bei - 70 °C vorgelegt und mit einer Mischung aus 0,032 mol Triethylamin und 0,028 mol K in 50 ml Dichlormethan zugetropft. Man lässt 1 h bei -70 °C rühren, versetzt mit 0,140 mol Triethylamintrishydrofluorid, lässt weitere 10 min rühren und gibt 0,070 mol 1,3-Dibrom-5,5-dimethylhydantoin suspendiert in 50 ml Dichlormethan hinzu. Nach 90 min Rühren lässt man die Reaktionslösung auf 20 °C erwärmen und gießt die Lösung auf ein Gemisch bestehend aus 1 N NaOH-Lösung und 35 ml ges. NaHSO₃-Lösung. Nach beendeter Gasentwicklung wird die organische Phase abgetrennt und die wässrige Phase wird mit Dichlormethan extrahiert. Die vereinigten organischen Phasen werden zuletzt wie üblich aufgearbeitet. K 97 S_{B} 219 N 277,9 l; Δε = 1,9; Δn = 0,1055

Analog werden die folgenden Verbindungen der Formel 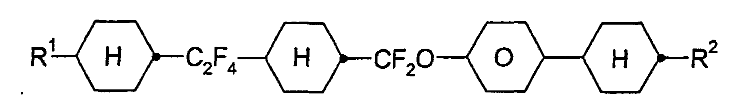 hergestellt.

| R¹ | R² |
|---|---|
| CH₃ | CH₃ |
| CH₃ | C₂H₅ |
| CH₃ | n-C₃H₇ |
| CH₃ | n-C₄H₅ |
| CH₃ | n-C₅H₁₁ |
| CH₃ | n-C₆H₁₃ |
| C₂H₅ | CH₃ |
| C₂H₅ | C₂H₅ |
| C₂H₅ | n-C₃H₇ |
| C₂H₅ | n-C₄H₅ |
| C₂H₅ | n-C₅H₁₁ |
| C₂H₅ | n-C₆H₁₃ |
| n-C₃H₇ | CH₃ |
| n-C₃H₇ | C₂H₅ |
| n-C₃H₇ | n-C₃H₇ |
| n-C₃H-₇ | n-C₄H₉ |
| n-C₃H₇ | n-C₅H₁₁ |
| n-C₃H₇ | n-C₆H₁₃ |
| n-C₄H₉ | CH₃ |
| n-C₄H₉ | C₂H₅ |
| n-C₄H₉ | n-C₃H₇ |
| n-C₄H₉ | n-C₄H₉ |
| n-C₄H₉ | n-C₅H₁₁ |
| n-C₄H₉ | n-C₆H₁₃ |
| n-C₅H₁₁ | CH₃ |
| n-C₅H₁₁ | n-C₃H₇ |
| n-C₅H₁₁ | n-C₄H₉ |
| n-C₅H₁₁ | n-C₅H₁₁ |
| n-C₅H₁₁ | n-C₆H₁₃ |
| n-C₆H₁₃ | CH₃ |
| n-C₆H₁₃ | C₂H₅ |
| n-C₆H₁₃ | n-C₃H₇ |
| n-C₆H₁₃ | n-C₄H₉ |
| n-C₆H₁₃ | n-C₅H₁₁ |
| n-C₆H₁₃ | n-C₆H₁₃ |
| CH₂=CH | CH₃ |
| CH₂=CH | C₂H₅ |
| CH₂=CH | n-C₃H₇ |
| CH₂=CH | n-C₄H₉ |
| CH₂=CH | n-C₅H₁₁ |
| CH₂=CH | n-C₆H₁₃ |
| CH₃ | CH₂=CH |
| C₂H₅ | CH₂=CH |
| n-C₃H₇ . | CH₂=CH |
| n-C₄H₉ | CH₂=CH |
| n-C₅H₁₁ | CH₂=CH |
| n-C₆H₁₃ | CH₂=CH |
| CH₃CH=CH | CH₃ |
| CH₃CH=CH | C₂H₅ |
| CH₃CH=CH | n-C₃H₇ |
| CH₃CH=CH | n-C₄Hg |
| CH₃CH=CH | n-C₅H₁₁ |
| CH₃CH=CH | n-C₆H₁₃ |
| CH₂₌CH-C₂H₄ | CH₃ |
| CH₂=CH-C₂H₄ | C₂H₅ |
| CH₂=CH-C₂H₄ | n-C₃H₇ |
| CH₂=CH-C₂H₄ | n-C₄H₉ |
| CH₂=CH-C₂H₄ | n-C₅H₁₁ |
| CH₂=CH-C₂H₄ | n-C₆H₁₃ |
| (CH₃)₂CH | CH₃ |
| (CH₃)₂CH | C₂H₅ |
| (CH₃)₂CH | n-C₃H₇ |
| (CH₃)₂CH | n-C₄H₉ |
| (CH₃)₂CH | n-C₅H₁₁ |
| (CH₃)₂CH | n-C₆H₁₃ |
| (CH₃)₂CHCH₂ | CH₃ |
| (CH₃)₂CHCH₂ | C₂H₅ |
| (CH₃)₂CHCH₂ | n-C₃H₇ |
| (CH₃)₂CHCH₂ | n-C₄H₉ |
| (CH₃)₂CHCH₂ | n-C₅H₁₁ |
| (CH₃)₂CHCH₂ | n-C₆H₁₃ |

### Mischungsbeispiele

### Beispiel M1

| | | | |
|---|---|---|---|
| BCH-3F.F | 10,83 % | Klärpunkt [°C] | 96,2 |
| BCH-5F.F | 9,02 % | d · Δn [µm] | 0,50 |
| ECCP-30CF₃ | 4,51 % | Verdrillung [°] | 90 |
| ECCP-50CF₃ | 4,51 % | γ₁ [mPa · s, 20 °C] | 144 |
| CBC-33F | 1,80% | | |
| CBC-53F | 1,80 % | | |
| CBC-55F | 1,80 % | | |
| PCH-6F | 7,22 % | | |
| PCH-7F | 5,41 % | | |
| CCP-20CF₃ | 7,22 % | | |
| CCP-30CF₃ | 10,83 % | | |
| CCP-40CF₃ | 6,32 % | | |
| CCP-50CF₃ | 9,92 % | | |
| PCH-5F | 9,02 % | | |
| CWCQU-3-F | 9,78 % | | |

### Beispiel M2

| | | | |
|---|---|---|---|
| PCH-5F | 3,20 % | Klärpunkt [°C] | 132,3 |
| CCP-20CF₂.F.F | 17,04 % | Δε [kHz, 20 °C] | 9,2 |
| CCP-30CF₂.F.F | 16,00 % | d · Δn [µm] | 0,50 |
| CCP-50CF₂.F.F | 17,04 % | Verdrillung [°] | 90 |
| CUP-2F.F | 5,36 % | | |
| CUP-3F.F | 5,36 % | | |
| CBC-33F | 5,36 % | | |
| CBC-53F | 5,36 % | | |
| CBC-55F | 5,28 % | | |
| CWCQU-3-F | 20,02 % | | |

### Beispiel M3 (für 4V Treiber)

| | | | |
|---|---|---|---|
| CC-5-V | 2,0 % | Klärpunkt [°C] | 102,6 |
| CCG-V-F | 10,0 % | Δn [589 nm, 20°C] | 0,0825 |
| CCP-20CF₃.F | 12,0 % | Δε [1 kHz, 20°C] | 6,8 |
| CCP-30CF₃.F | 12,0% | γ₁ [mPa·s, 20°C] | 147 |
| CCP-20CF₃ | 9,0 % | d · Δn [µm] | 0,50 |
| CCP-30CF₃ | 9,0 % | Verdrillung [°] | 90 |
| CCP-40CF₃ | 8,0 % | | |
| CCP-3F.F.F | 10,0 % | | |
| CCP-5F.F.F | 8,0 % | | |
| CWCQU-3-F | 10,0 % | | |
| CC-3-V1 | 10,0 % | | |

### Mischunq M4 (für 4V Treiber)

| | | | |
|---|---|---|---|
| CC-5-V | 7,0 % | Klärpunkt [°C] | 102,5 |
| CCG-V-F | 10,0 % | Δn [589 nm, 20°C] | 0,0832 |
| CCP-20CF₃.F | 12,0 % | Δε [1 kHz, 20°C] | 6,8 |
| CCP-30CF₃.F | 12,0 % | γ1 [mPa·s, 20°C] | 146 |
| CCP-20CF₃ | 9,0 % | d · Δn [µm] | 0,50 |
| CCP-30CF₃ | 9,0 % | Verdrillung [°] | 90 |
| CCP-3F.F.F | 10,0 % | | |
| CCP-5F.F.F | 7,0 % | | |
| CWCQU-3-F | 8,0 % | | |
| CCGU-3-F | 6,0 % | | |
| CC-3-V1 | 10,0 % | | |

### Beispiel M5 (für 4V Treiber)

| | | | |
|---|---|---|---|
| CC-5-V | 11,0 % | Klärpunkt [°C] | 102,3 |
| CCG-V-F | 10,0 % | Δn [589 nm, 20°C] | 0,0845 |
| CCP-20CF₃.F | 12,0% | Δε [1 kHz, 20°C] | 6,8 |
| CCP-30CF₃.F | 12,0 % | γ1 [mPa·s, 20°C] | 145 |
| CCP-20CF₃ | 9,0 % | d · Δn [µm] | 0,50 |
| CCP-30CF₃ | 9,0 % | Verdrillung [°] | 90 |
| CCP-3F.F.F | 11,0 % | | |
| CBC-33F | 4,0 % | | |
| CWCQU-n-F | 10,0 % | | |
| CCGU-3-F | 6,0 % | | |
| PCH-7F | 6,0 % | | |

### Beispiel M6 (für 3,3V Treiber)

| | | | |
|---|---|---|---|
| CCP-30CF₃ | 8,0 % | Klärpunkt [°C] | 72,5 |
| CCP-40CF₃ | 5,0 % | Δn [589 nm, 20°C] | 0,0908 |
| CCP-20CF₃.F | 12,0% | Δε [1 kHz, 20 °C] | 9,8 |
| CCP-30CF₃.F | 12,0 % | γ₁ [mPa·s, 20°C] | 141 |
| CCP-2F.F.F | 11,0 % | d · An [µm] | 0,50 |
| CCP-3F.F.F | 9,0 % | Verdrillung [°] | 90 |
| CGU-2-F | 10,0 % | | |
| CGU-3-F | 10,0 % | | |
| CGU-5-F | 9,0 % | | |
| CWCQU-3-F | 8,5 % | | |
| CC-3-V1 | 5,5 % | | |

### Beispiel M7 (für 3,3V Treiber)

| | | | |
|---|---|---|---|
| CCP-20CF₃ | 8,0 % | Klärpunkt [°C] | 72,5 |
| CCP-30CF₃ | 8,0 % | Δn [589 nm, 20 °C] | 0,0884 |
| CCP-40CF₃ | 8,0 % | Δε [1 kHz, 20 °C] | 9,9 |
| CCP-20CF₃.F | 12,0 % | γ1 [mPa.s, 20 °C] | 143 |
| CCP-2F.F.F | 11,0 % | d ·Δn [µm] | 0,50 |
| CCP-3F.F.F | 8,5 % | Verdrillung [°] | 90 |
| CGU-2-F | 10,0 % | | |
| CGU-3-F | 10.0% | | |
| CGU-5-F | 5,0 % | | |
| CCGU-3-F | 3,5% | | |
| CCH-3CF₃ | 6,0 % | | |
| CWCQU-3-F | 10,0 % | | |

### Beispiel M8 (low Δn)

| | | | |
|---|---|---|---|
| CC-5V | 3,0 % | Klärpunkt [°C] | 79,5 |
| CCH-3CF₃ | 7,0 % | Δn [589 nm, 20°C] | 0,0709 |
| CCH-5CF₃ | 8,0 % | Δε [1 kHz, 20°C] | 9,6 |
| CCP-2F.F.F | 11,0 % | γ1 [mPa·s, 20°C] | 151 |
| CCP-3F.F.F | 12,0 % | VHR [%] | 98,3 |
| CCP-5F.F.F | 5,0 % | V₁₀ [V] | 1,42 |
| CCP-20CF₃.F | 12,0% | | |
| CCP-30CF₃.F | 6,0 % | | |
| CGU-2-F | 8,0 % | | |
| CCOC-4-3 | 3,0 % | | |
| CWCQU-3-F | 25,0 % | | |

### Beispiel M9 (low Δn)

| | | | |
|---|---|---|---|
| CCP-2F.F.F | 11,0 % | Klärpunkt [°C] | 78,5 |
| CCP-3F.F.F | 12,0 % | Δn [589 nm, 20 °C] | 0,0654 |
| CCP-5F.F.F | 5,0 % | VHR [%] | 97,5 |
| CCZU-2-F | 5,0 % | d . Δn [µm] | 0,50 |
| CCZU-3-F | 16,0 % | Verdrillung [°C] | 90 |
| CCZU-5-F | 4,0 % | V₁₀ [V] | 1,29 |
| CCH-3CF₃ | 8,0 % | | |
| CCH-5CF₃ | 8,0 % | | |
| PCH-7F | 5,0 % | | |
| CWCQU-n-F | 26,0 % | | |

### Beispiel M10 (für 3,3 V Treiber)

| | | | |
|---|---|---|---|
| CCP-2F.F.F | 7,0 % | Klärpunkt [°C] | 83,5 |
| CCP-3F.F.F | 10,0 % | Δn [589 nm, 20 °C] | 0,0932 |
| CCP-30CF₃.F | 12,0 % | Δε [1 kHz, 20 °C] | 10,9 |
| CCP-20CF₃ | 7,0 % | γ1 [mPa·s, 20 °C] | 184 |
| CCP-30CF₃ | 8,0 % | | |
| CGU-2-F | 10,0 % | | |
| CGU-3-F | 10,0 % | | |
| CGU-5-F | 10,5 % | | |
| CCG-V-F | 7,5 % | | |
| CWCQU-3-F | 8,0 % | | |
| CWCQU-5-F | 10,0 % | | |

### Beispiel M11 (für 3,8 V bzw. 4V Treiber)

| | | | |
|---|---|---|---|
| PGU-2-F | 7,0 % | Klärpunkt [°C] | 72,0 |
| PGU-3-F | 2,0 % | Δn [589 nm, 20 °C] | 0,0936 |
| CGZP-2-OT | 5,0 % | γ1 [mPa·s, 20 °C] | 78 |
| CGZP-3-OT | 2,0 % | V_{10,0,20} [V] | 1,55 |
| BCH-3F.F.F | 15,0 % | | |
| CCP-2F.F.F | 10,0 % | | |
| CCZU-2-F | 3,0 % | | |
| CC-3-V1 | 12,0 % | | |
| CC-5-V | 15,0 % | | |
| CCH-35 | 6,0 % | | |
| PCH-302 | 10,0 % | | |
| CCP-V-1 | 4,0 % | | |
| CWCQU-2-F | 4,0% | | |
| CWCQU-3-F | 5,0 % | | |

### Beispiel M12

| | | | |
|---|---|---|---|
| BCH-3F.F | 10,8 % | Klärpunkt [°C] | 96,2 |
| BCH-5F.F | 9,0 % | Δn [589 nm, 20 °C] | 0,0950 |
| ECCP-30CF₃ | 4,5 % | Δε [1 kHz, 20 °C] | 5,7 |
| ECCP-50CF₃ | 4,5 % | | |
| CBC-33F | 1,8 % | | |
| CBC-53F | 1,8 % | | |
| CBC-55F | 1,8% | | |
| PCH-6F | 7,2 % | | |
| PCH-7F | 5,4 % | | |
| CCP-20CF₃ | 7,2 % | | |
| CCP-30CF₃ | 10,8 % | | |
| CCP-40CF₃ | 6,3 % | | |
| CCP-50CF₃ | 9,9 % | | |
| PCH-5F | 9,0 % | | |
| CWCQU-5-F | 10,0 % | | |

### Beispiel M13

| | | | |
|---|---|---|---|
| BCH-3F.F | 10,8 % | Klärpunkt [°C] | 93,7 |
| BCH-5F.F | 9,0 % | γ1 [mPa.s, 20 °C] | 184 |
| ECCP-30CF₃ | 4,5 % | | |
| ECCP-50CF₃ | 4,5 % | | |
| CBC-33F | 1,8% | | |
| CBC-53F | 1,8 % | | |
| CBC-55F | 1,8% | | |
| PCH-6F | 7,2 % | | |
| PCH-7F | 5,4 % | | |
| CCP-20CF₃ | 7,2 % | | |
| CCP-30CF₃ | 10,8% | | |
| CCP-40CF₃ | 6,3 % | | |
| CCP-50CF₃ | 9,9% | | |
| PCH-5F | 9,0% | | |
| CWCQU-2-F | 10,0 % | | |

### Beispiel M14

| | | | |
|---|---|---|---|
| BCH-3F.F | 10,8 % | Klärpunkt [°C] | 94,4 |
| BCH-5F.F | 9,0 % | Δn [589 nm, 20°C] | 0,0951 |
| ECCP-30CF₃ | 4,5 % | Δε [1 kHz, 20°C] | 5,7 |
| ECCP-50CF₃ | 4,5% | | |
| CBC-33F | 1,8% | | |
| CBC-53F | 1,8% | | |
| CBC-55F | 1,8% | | |
| PCH-6F | 7,2 % | | |
| PCH-7F | 5,4 % | | |
| CCP-20CF₃ | 7,2 % | | |
| CCP-30CF₃ | 10,8% | | |
| CCP-40CF₃ | 6,3 % | | |
| CCP-50CF₃ | 9,9 % | | |
| PCH-5F | 9,0 % | | |
| CWCQU-V-F | 10,0 % | | |

### Beispiel M15

| | | | |
|---|---|---|---|
| BCH-3F.F | 10,8 % | Klärpunkt [°C] | 109,9 |
| BCH-5F.F | 9,0 % | Δn [589 nm, 20°C] | 0,0974 |
| ECCP-30CF₃ | 4,5 % | Δε [1 kHz, 20 °C] | 4,9 |
| ECCP-50CF₃ | 4.5 % | | |
| CBC-33F | 1,8 % | | |
| CBC-53F | 1,8 % | | |
| CBC-55F | 1,8 % | | |
| PCH-6F | 7,2 % | | |
| PCH-7F | 5,4 % | | |
| CCP-20CF₃ | 7,2 % | | |
| CCP-30CF₃ | 10,8% | | |
| CCP-40CF₃ | 6,3 % | | |
| CCP-50CF₃ | 9,9 % | | |
| PCH-5F | 9,0 % | | |
| CWCQPC-5-2 | 10,0 % | | |

### Beispiel M16

| | | | |
|---|---|---|---|
| BCH-3F.F | 10,8 % | Klärpunkt [°C] | 98,6 |
| BCH-5F.F | 9,0 5 | Δn [589 nm, 20°C] | 0,0952 |
| ECCP-30CF₃ | 4,5 % | Δε [1 kHz, 20 °C] | 5,4 |
| ECCP-50CF₃ | 4,5 % | | |
| CBC-33F | 1,8 % | | |
| CBC-53F | 1.8% | | |
| CBC-55F | 1,8 % | | |
| PCH-6F | 7,2 % | | |
| PCH-7F | 5,4 % | | |
| CCP-20CF₃ | 7,2 % | | |
| CCP-30CF₃ | 10,8% | | |
| CCP-40CF₃ | 6,3 % | | |
| CCP-50CF₃ | 9,9 % | | |
| PCH-5F | 9,0 % | | |
| CWCQG-5-F | 10,0 % | | |

### Beispiel M17

| | | | |
|---|---|---|---|
| BCH-3F.F | 10,8 % | Klärpunkt [°C] | 97,8 |
| BCH-5F.F | 9,0 % | Δn [589 nm, 20°C] | 0,0955 |
| ECCP-30CF₃ | 4,5 % | Δε [1 kHz, 20 °C] | 5,6 |
| ECCP-50CF₃ | 4,5% | | |
| CBC-33F | 1,8% | | |
| CBC-53F | 1,8% | | |
| CBC-55F | 1,8% | | |
| PCH-6F | 7,2 % | | |
| PCH-7F | 5,4 % | | |
| CCP-20CF₃ | 7,2 % | | |
| CCP-30CF₃ | 10,8 % | | |
| CCP-40CF₃ | 6,3 % | | |
| CCP-50CF₃ | 9,9 % | | |
| PCH-5F | 9,0 % | | |
| CWCQG-3-OT | 10,0 % | | |

### Beispiel M18

| | | | |
|---|---|---|---|
| BCH-3F.F | 10,8% | Klärpunkt [°C] | 98,2 |
| BCH-5F.F | 9,0 % | Δn [589 nm, 20°C] | 0,0955 |
| ECCP-30CF₃ | 4,5 % | Δε [1 kHz, 20 °C] | 5,4 |
| ECCP-50CF₃ | 4,5% | | |
| CBC-33F | 1,8% | | |
| CBC-53F | 1,8% | | |
| CBC-55F | 1,8% | | |
| PCH-6F | 7,2 % | | |
| PCH-7F | 5,4 % | | |
| CCP-20CF₃ | 7,2 % | | |
| CCP-30CF₃ | 10,8% | | |
| CCP-40CF₃ | 6,3 % | | |
| CCP-50CF₃ | 9,9 % | | |
| PCH-5F | 9,0 % | | |
| CWCQG-3-F | 10,0 % | | |

## Patentansprüche

1. Flüssigkristalline Verbindungen der Formel I, 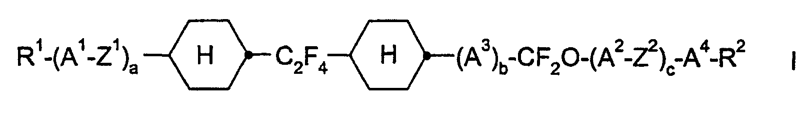 worin
R¹ und R² jeweils unabhängig voneinander einen unsubstituierten, einen einfach durch CN oder CF₃ oder mindestens einfach durch Halogen substituierten Alkylrest mit 1 bis zu 15 C-Atomen, wobei in diesen Resten auch eine oder mehrere CH₂-Gruppen durch -O-, -S-, -CH=CH-, - C≡C-, -OC-O- oder -O-CO- so ersetzt sein können, dass O-Atome nicht direkt miteinander verknüpft sind, und R² auch CN, SF₅, F, Cl, NCS oder SCN,
A¹,A²,A³ und A⁴
a) einen 1,4-Cyclohexenylen- oder 1,4-Cyclohexylenrest, worin eine oder zwei nicht benachbarte CH₂-Gruppen durch -O- oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl-, 1,4-Bicyclo[2,2,2]-octylen-, einen Naphthalin-2,6-diyl, Decahydronaphthalin-2,6-diyl, und 1,2,3,4-Tetrahydronaphthalin-2,6-diyl
wobei die Reste a), b) und c) ein oder mehrfach durch Halogenatome substituiert sein können,
Z¹ und Z² jeweils unabhängig voneinander -CO-O-, -O-CO-, -CF₂O-, -OCF₂-, -CH₂O-, -OCH₂-, -CH₂CH₂-, -(CH₂)₄-, - C₂F₄-, -CF₂CH₂-, -CH₂CF₂-, -CF=CF-, -CH=CH-, -C≡C- oder eine Einfachbindung,
a 0, 1 oder 2,
b 0, 1 oder 2, und
c 0, 1 oder 2, wobei a + b + c ≤ 2 ist,
bedeutet.

2. Flüssigkristalline Verbindungen nach Anspruch 1, **dadurch gekennzeichnet, dass** R¹ ein geradkettiger Alkylrest mit 1 bis 10 C-Atomen oder ein Alkenylrest mit 2 bis 10 C-Atomen bedeutet.

3. Flüssigkristalline Verbindungen nach Anspruch 1 oder 2, **dadurch gekennzeichnet, dass**
R² F, Cl, CN, CF₃, SF₅, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCCIFCF₂CF₃
bedeutet.

4. Flüssigkristalline Verbindungen nach einem der Ansprüche 1 bis 3, **dadurch gekennzeichnet, dass** c = 0 bedeutet.

5. Flüssigkristalline Verbindungen nach Anspruch 1 der Formeln I1 bis I24: 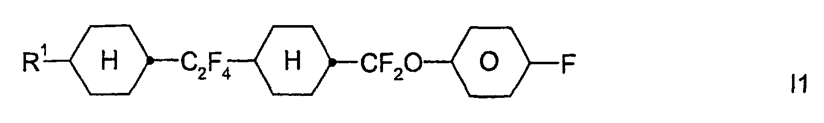 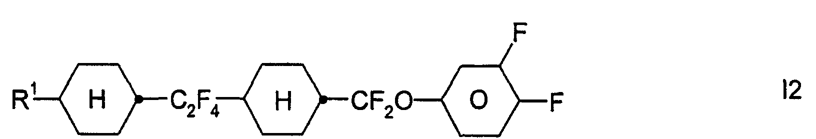 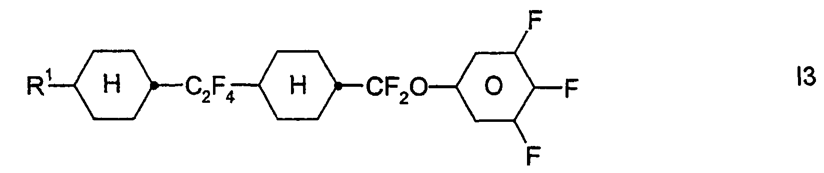 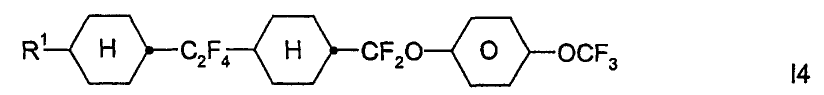 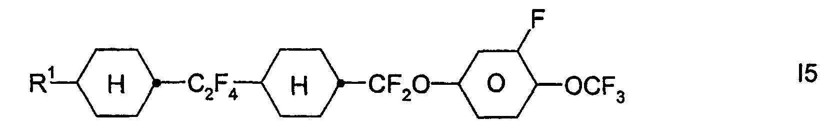 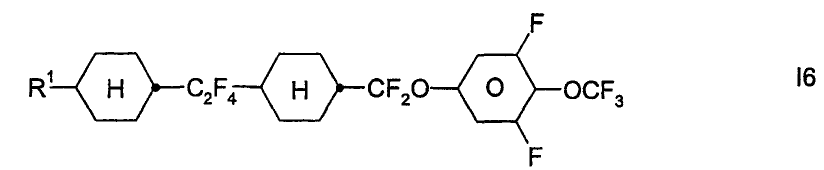 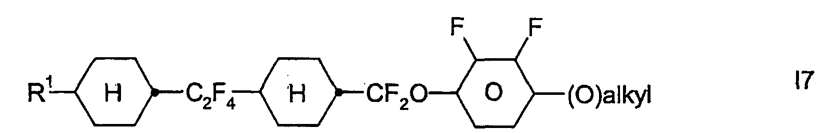 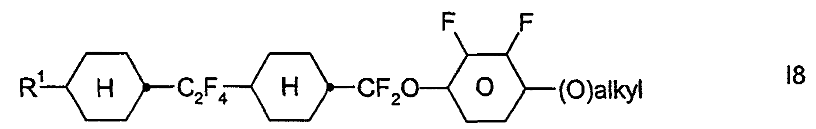 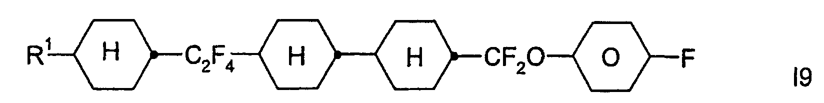 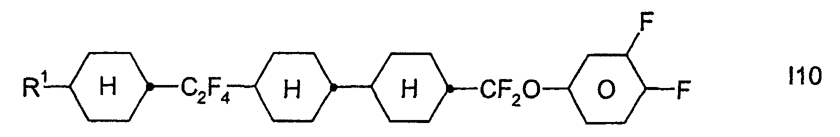 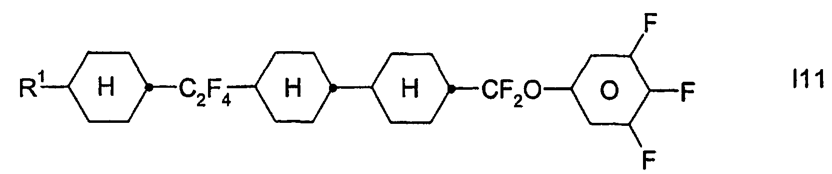 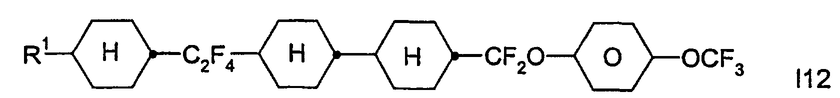 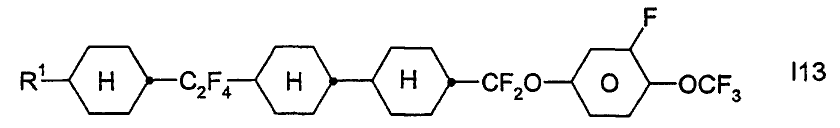 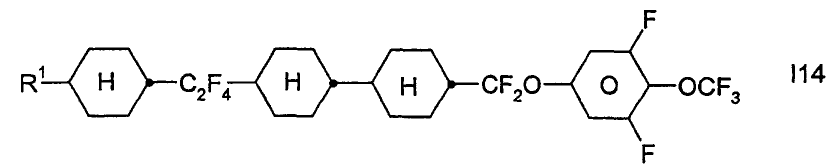 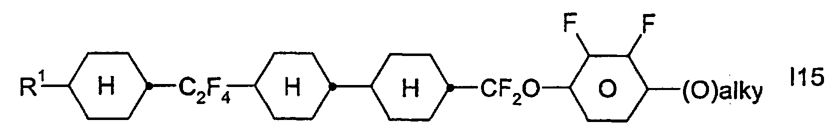 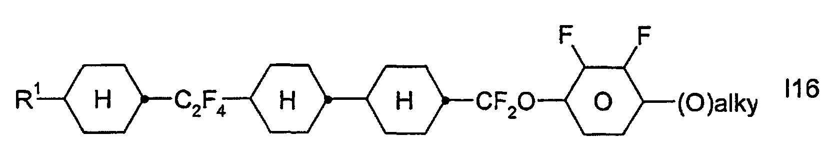 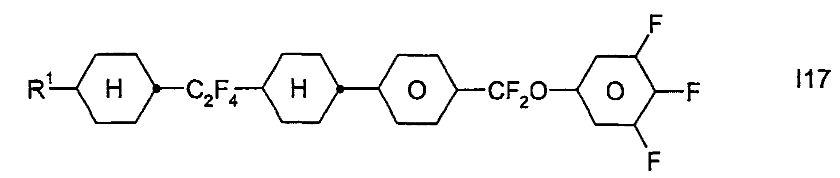 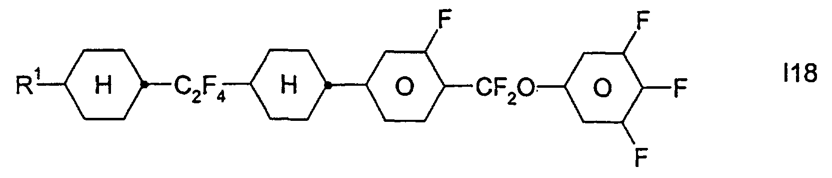 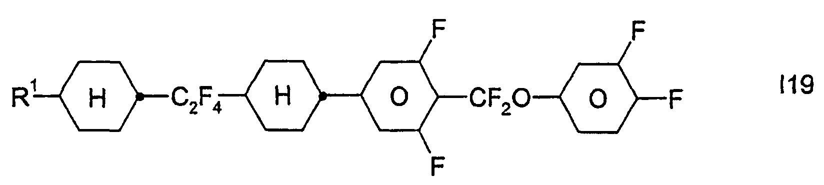 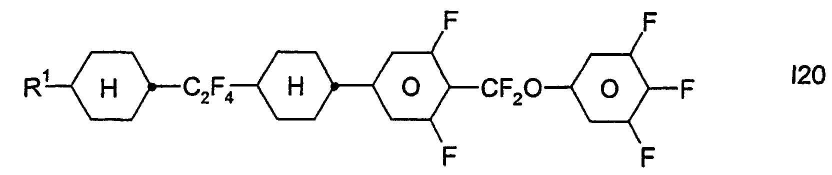 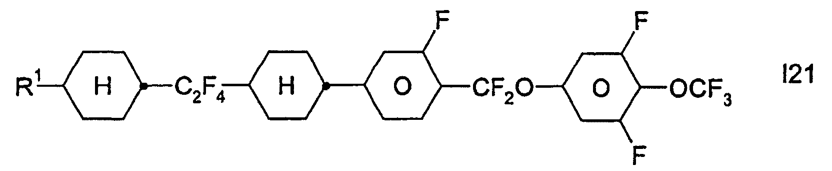 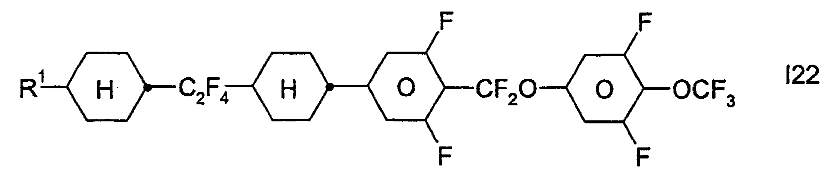 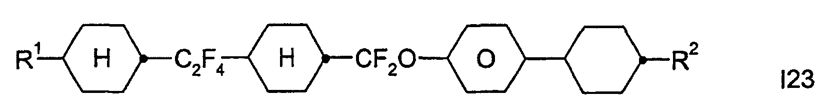 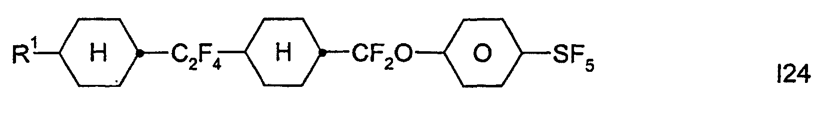 worin
R¹ die in Anspruch 1 angegebene Bedeutung hat und "alkyl" ein geradkettiger oder verzweigter Alkylrest mit 1-9 C-Atomen ist.

6. Flüssigkristallines Medium enthaltend mindestens zwei mesogene Verbindungen, **dadurch gekennzeichnet, dass** es mindestens eine Verbindung der Formel I nach Anspruch 1 enthält.

7. Flüssigkristallines Medium nach Anspruch 6, **dadurch gekennzeichnet, dass** es zusätzlich eine oder mehrere Verbindungen ausgewählt aus der Gruppe bestehend aus den allgemeinen Formeln II, III, IV, V, VI, VII, VIII und IX enthält: 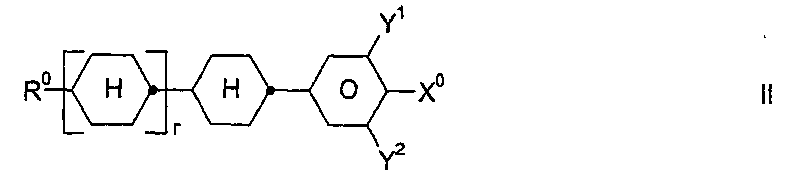 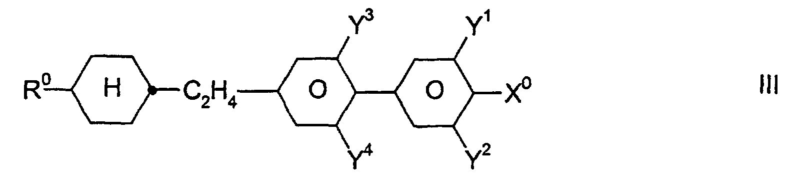 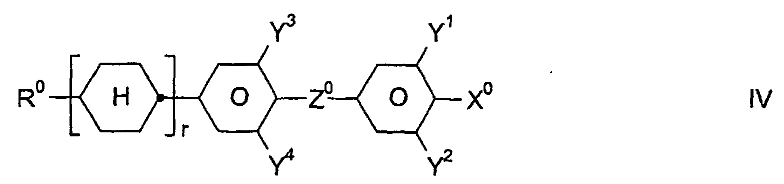 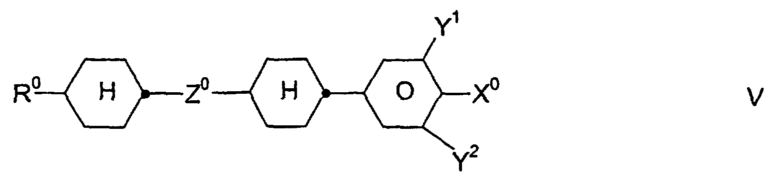 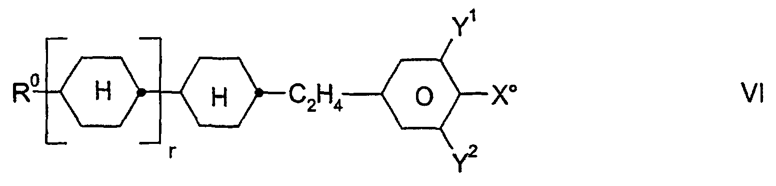 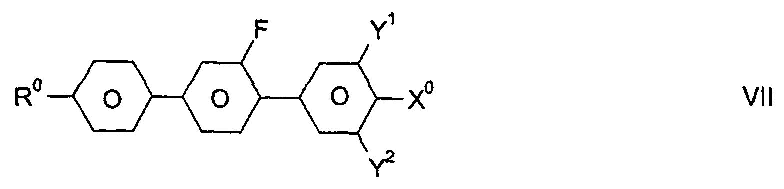 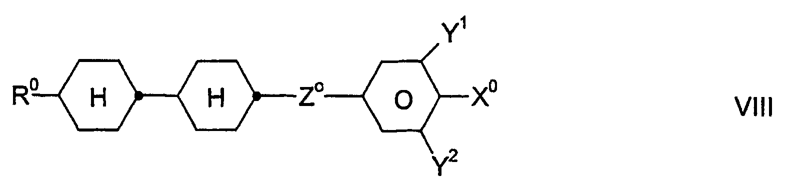 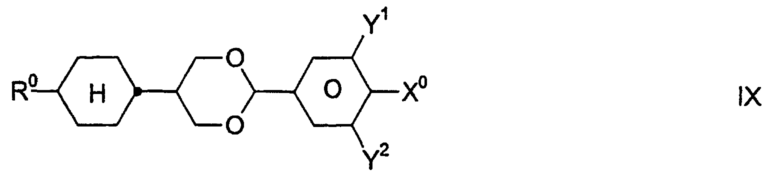 worin die einzelnen Reste die folgenden Bedeutungen haben:
R⁰ n-Alkyl, Oxaalkyl, Fluoralkyl, Alkenyloxy oder Alkenyl mit jeweils bis zu 9 C-Atomen
X⁰ F, Cl, halogeniertes Alkyl, halogeniertes Alkenyl, halogeniertes Alkenyloxy oder halogeniertes Alkoxy mit bis zu 7 C-Atomen,
Z⁰ -CH=CH-, -C₂H₄-, -C₂F₄-, -CF=CF-, -CF₂O-, -OCF₂- oder - COO-,
Y¹,Y², Y³ und Y⁴ jeweils unabhängig voneinander H oder F, und
r 0 oder 1.

8. Medium nach Anspruch 6 oder 7, **dadurch gekennzeichnet, dass** der Anteil an Verbindungen der Formel I bis IX im Gesamtgemisch mindestens 50 Gew.-% beträgt.

9. Medium nach einem der Ansprüche 6 bis 8, **dadurch gekennzeichnet, dass** es zusätzlich eine oder mehrere Verbindungen der Formeln RI bis RX, 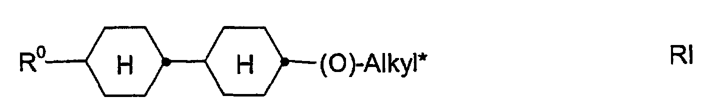 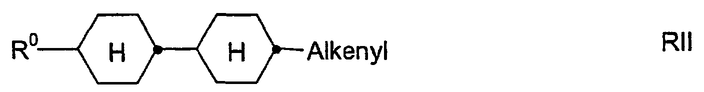 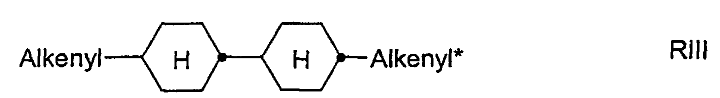 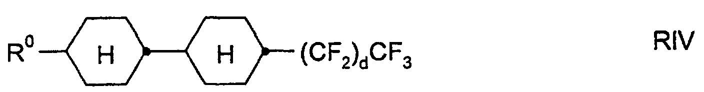 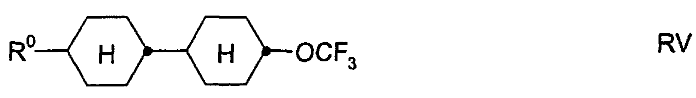 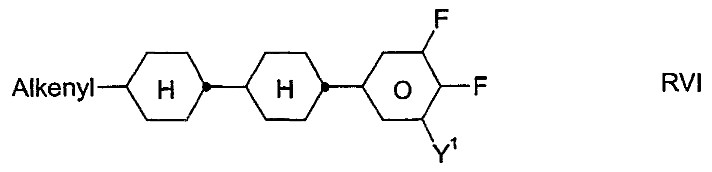 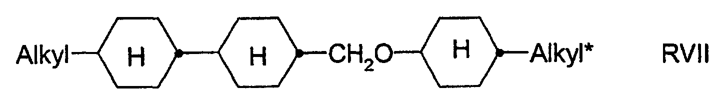 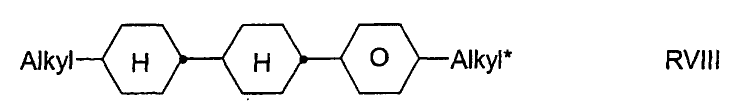 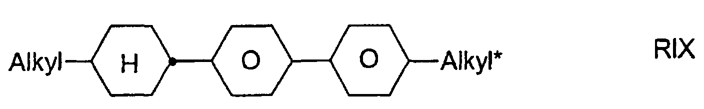 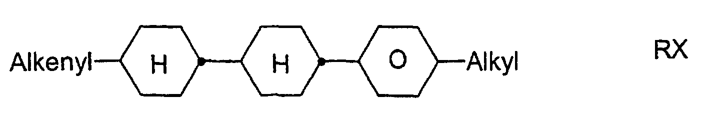 worin
R⁰ n-Alkyl, Oxaalkyl, Fluoralkyl, Alkenyloxy oder Alkenyl mit jeweils bis zu 9 C-Atomen,
d 0, 1 oder 2,
Y¹ H oder F,
Alkyl oder Alkyl^{*} jeweils unabhängig voneinander ein geradkettiger oder verzweigter Alkylrest mit 1-9 C-Atomen,
Alkenyl oder Alkenyl^{*} jeweils unabhängig voneinander einen geradkettigen oder verzweigten Alkenylrest mit bis zu 9 C-Atomen
bedeuten.
enthält.

10. Medium nach Anspruch 7, **dadurch gekennzeichnet, dass** X⁰ F oder OCF₃ und Y² H oder F bedeuten.

11. Verwendung des flüssigkristallinen Mediums nach Anspruch 6 für elektrooptische Zwecke.

12. Elektrooptische Flüssigkristallanzeige enthaltend ein flüssigkristallines Medium nach Anspruch 6.

## Claims

1. Liquid-crystalline compounds of the formula I 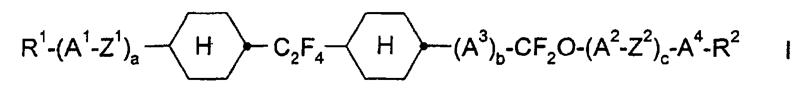 in which
R¹ and R² each, independently of one another, denote an alkyl radical having 1 to 15 C atoms which is unsubstituted, monosubstituted by CN or CF₃ or at least monosubstituted by halogen, where, in addition, one or more CH₂ groups in these radicals may be replaced by -O-, -S-, - CH=CH-, -C≡C-, -OC-O- or -O-CO- in such a way that O atoms are not linked directly to one another, and R² also denotes CN, SF₅, F, Cl, NCS or SCN,
A¹, A², A³ and A⁴ denote
a) a 1,4-cyclohexenylene or 1,4-cyclohexylene radical, in which one or two non-adjacent CH₂ groups may be replaced by -O- or -S-,
b) a 1,4-phenylene radical, in which one or two CH groups may be replaced by N,
c) a radical from the group consisting of piperidine-1,4-diyl, 1,4-bicyclo[2.2.2]octylene, naphthalene-2,6-diyl, decahydronaphthalene-2,6-diyl and 1,2,3,4-tetrahydronaphthalene-2,6-diyl,
where the radicals a), b) and c) may be mono- or polysubstituted by halogen atoms,
Z¹ and Z² each, independently of one another, denote -CO-O-, -O-CO-, -CF₂O-, -OCF₂-, -CH₂O-, -OCH₂-, -CH₂CH₂-, -(CH₂)₄-, -C₂F₄-, -CF₂CH₂-, -CH₂CF₂-, -CF=CF-, - CH=C_{H}-, -C≡C- or a single bond,
a denotes 0, 1 or 2,
b denotes 0, 1 or 2, and
c denotes 0, 1 or 2, where a + b + c is≤ 2.

2. Liquid-crystalline compounds according to Claim 1, **characterised in that** R¹ denotes a straight-chain alkyl radical having 1 to 10 C atoms or an alkenyl radical having 2 to 10 C atoms.

3. Liquid-crystalline compounds according to Claim 1 or 2, **characterised in that**
R² denotes F, Cl, CN, CF₃, SF₅, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃,OCF₂CHFCF₃,OCC)FCF₂CF₃.

4. Liquid-crystalline compounds according to one of Claims 1 to 3, **characterised in that** c = 0.

5. Liquid-crystalline compounds according to Claim 1 of the formulae I1 to 124: 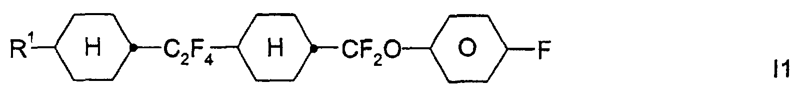 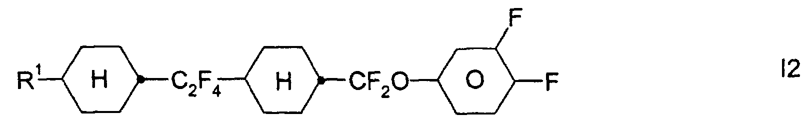 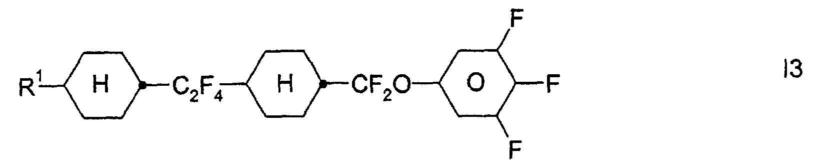 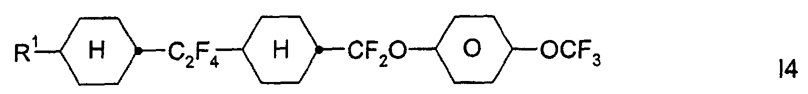 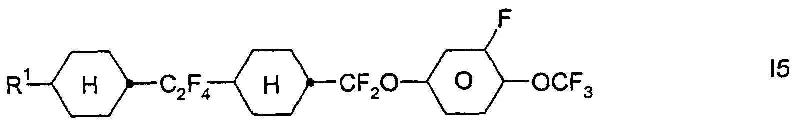 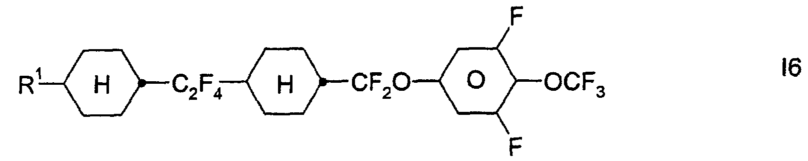 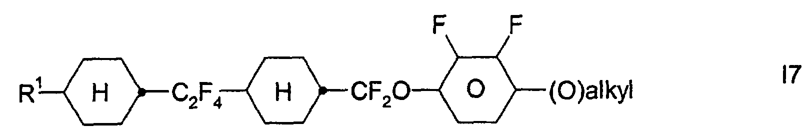 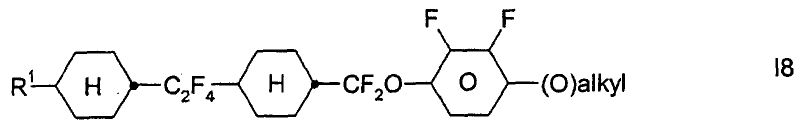 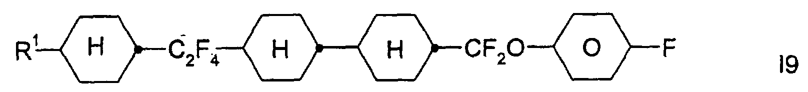 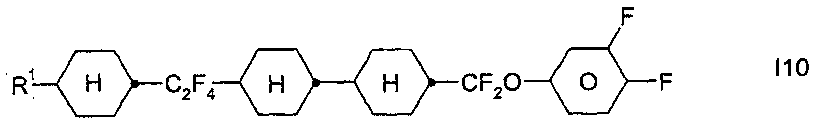 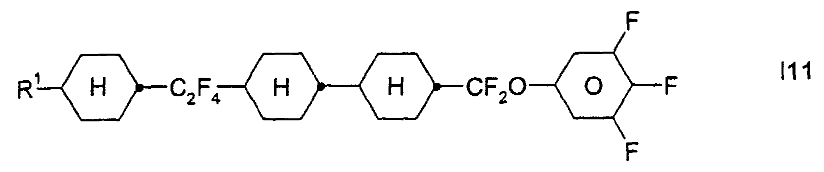 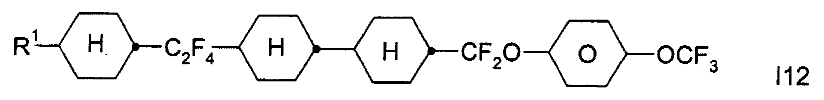 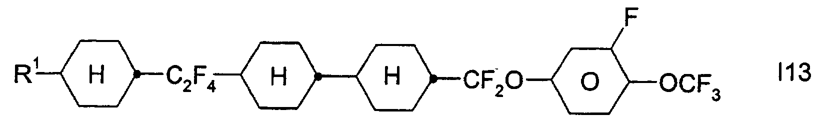 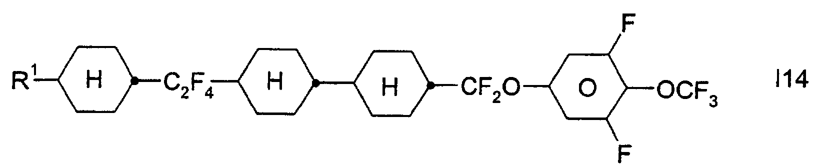 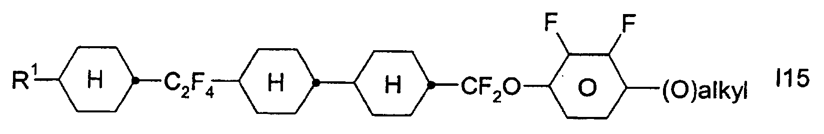 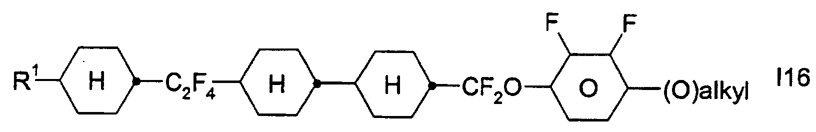 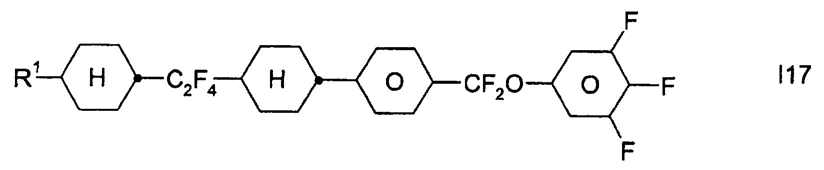 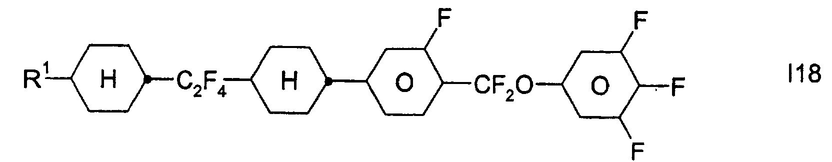 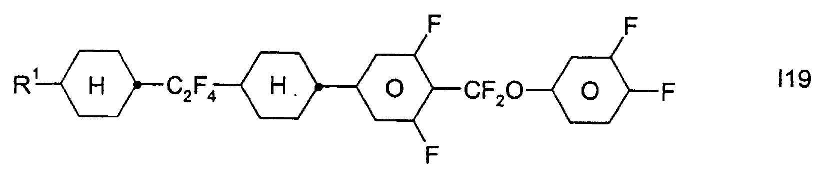 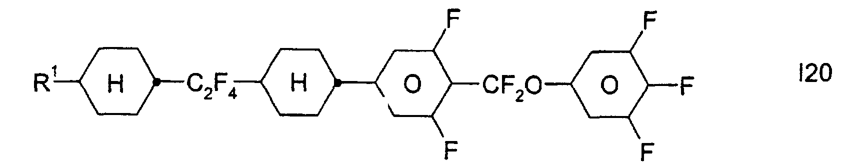 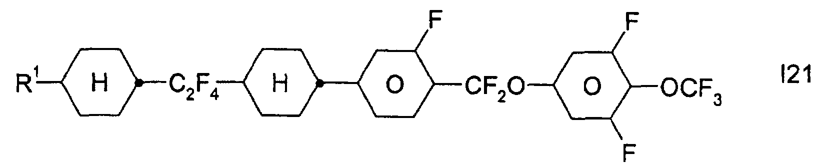 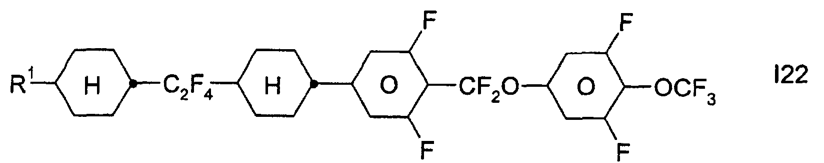 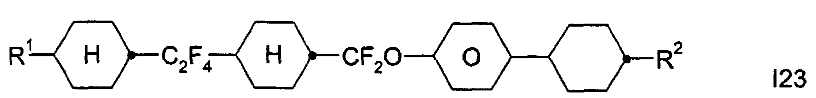 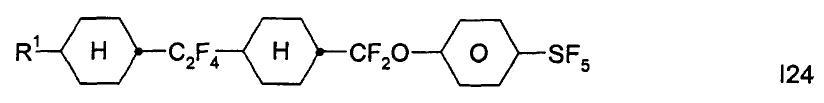 in which
R¹ has the meaning given in Claim 1, and "alkyl" is a straight-chain or branched alkyl radical having 1-9 C atoms.

6. Liquid-crystalline medium comprising at least two mesogenic compounds, **characterised in that** it comprises at least one compound of the formula I according to Claim 1.

7. Liquid-crystalline medium according to Claim 6, **characterised in that** it additionally comprises one or more compounds selected from the group consisting of the general formulae II, III, IV, V, VI, VII, VIII and IX: 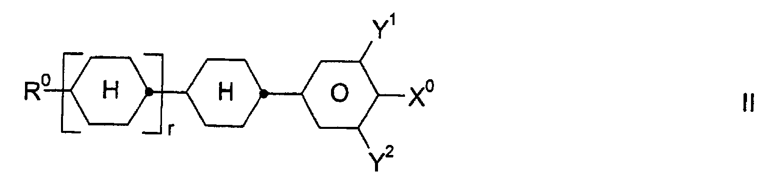 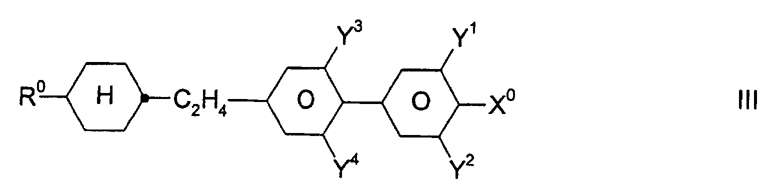 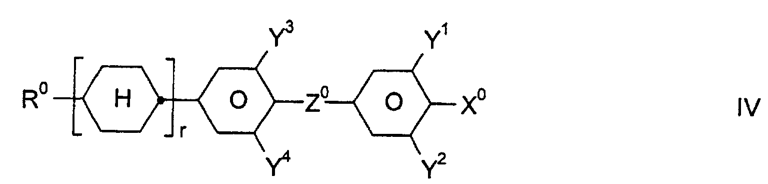 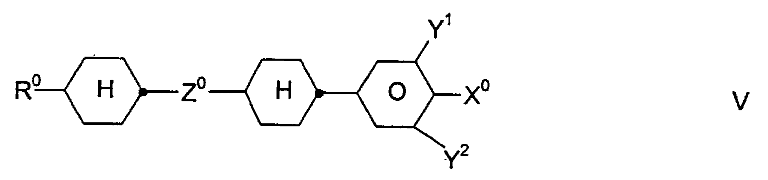 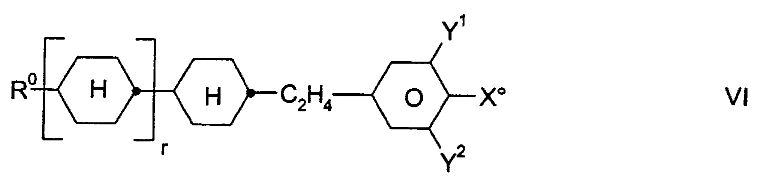 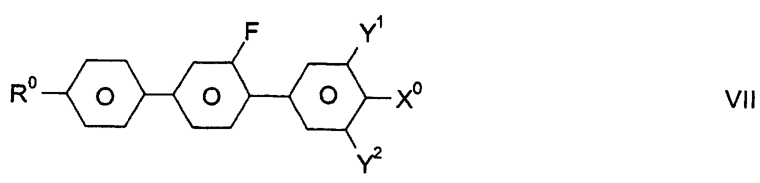 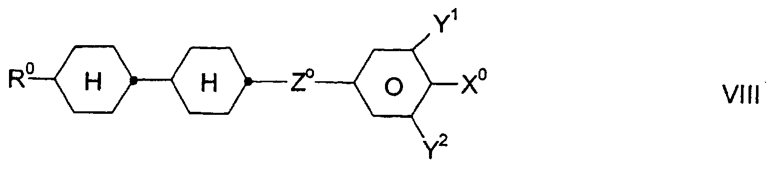 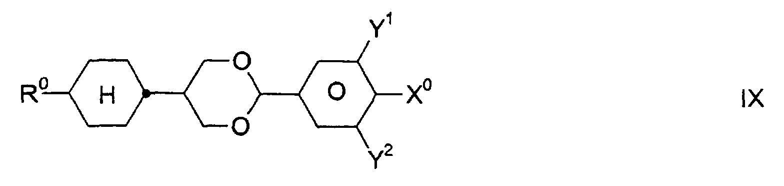 in which the individual radicals have the following meanings:
R⁰ n-alkyl, oxaalkyl, fluoroalkyl, alkenyloxy or alkenyl, each having up to 9 C atoms,
X⁰ F, Cl, halogenated alkyl, halogenated alkenyl, halogenated alkenyloxy or halogenated alkoxy having up to 7 C atoms,
Z⁰ -CH=CH-, -C₂H₄-, -C₂F₄-, -CF=CF-, -CF₂O-, -OCF₂- or - COO-,
Y¹,Y², Y³ and Y⁴ each, independently of one another, H or F, and
r 0 or 1.

8. Medium according to Claim 6 or 7, **characterised in that** the proportion of compounds of the formulae I to IX in the mixture as a whole is at least 50% by weight.

9. Medium according to one of Claims 6 to 8, **characterised in that** it additionally comprises one or more compounds of the formulae RI to RX: 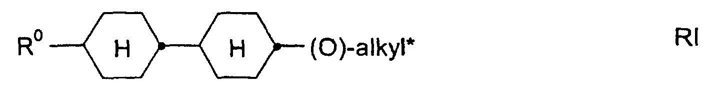 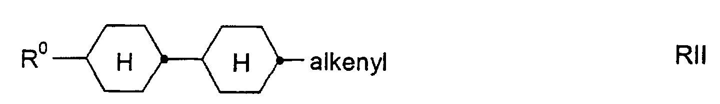 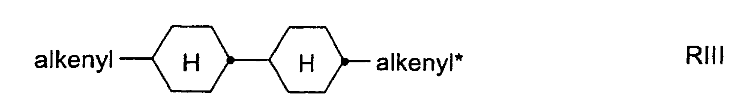 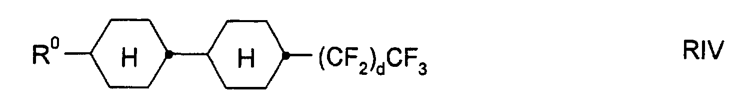 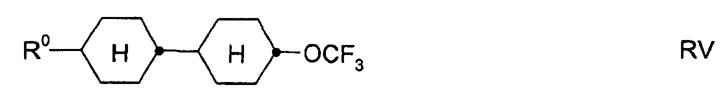 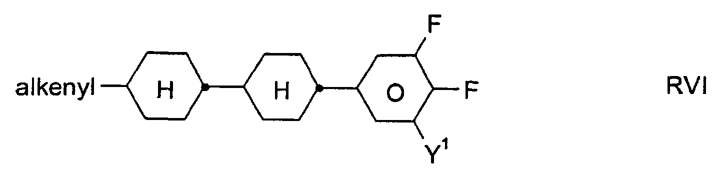 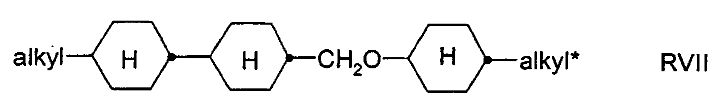 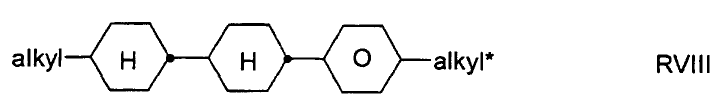 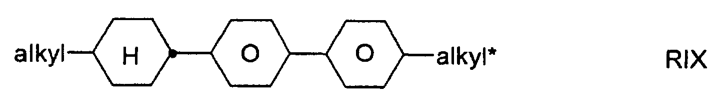 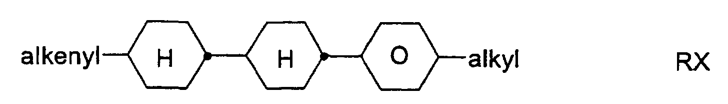 in which
R⁰ denotes n-alkyl, oxaalkyl, fluoroalkyl, alkenyloxy or alkenyl, each having up to 9 C atoms,
d denotes 0, 1 or 2,
Y¹ denotes H or F,
alkyl or alkyl^{*} each, independently of one another, denote a straight-chain or branched alkyl radical having 1-9 C atoms,
alkenyl or alkenyl* each, independently of one another, denote a straight-chain or branched alkenyl radical having up to 9 C atoms.

10. Medium according to Claim 7, **characterised in that** X⁰ denotes F or OCF₃ and Y² denotes H or F.

11. Use of the liquid-crystalline medium according to Claim 6 for electro-optical purposes.

12. Electro-optical liquid-crystal display containing a liquid-crystalline medium according to Claim 6.

## Revendications

1. Composés de cristaux liquides de la formule I 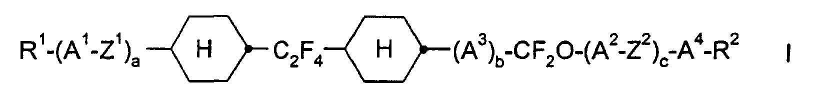 dans laquelle
R¹ et R² chacun, indépendamment l'un de l'autre, représente un radical alkyle qui comporte de 1 à 15 atomes de C, lequel est non substitué, monosubstitué par CN ou CF₃ ou au moins monosubstitué par halogène, dans laquelle, en plus, un ou plusieurs groupes CH₂ dans ces radicaux peuvent être remplacés par -O-, -S-, - CH=CH-, -C≡C-, -OC-O- ou -O-CO- de telle sorte que des atomes de O ne soient pas liés directement les uns aux autres, et R² représente également CN, SF₅, F, CI, NCS ou SCN,
A¹, A², A³ et A⁴ représentent
a) un radical 1,4-cyclohexénylène ou 1,4-cyclohexylène, où un ou deux groupes CH₂ non adjacents peuvent être remplacés par -O- ou -S-,
b) un radical 1,4-phénylène, où un ou deux groupes CH peuvent être remplacés par N,
c) un radical pris parmi le groupe comprenant pipéridine-1,4-diyle, 1,4-bicyclo[2.2.2]octylène, naphthalène-2,6-diyle, décahydronaphthalène-2,6-diyle et 1,2,3,4-tétrahydronaphthalène-2,6-diyle,
dans laquelle les radicaux a), b) et c) peuvent être mono- ou poly- substitués par des atomes halogène,
Z¹ et Z² chacun, indépendamment l'un de l'autre, représentent - CO-O-, -O-CO-, -CF₂O-, -OCF₂-, -CH₂O-, -OCH₂-, -CH₂CH₂-, -(CH₂)₄-, -C₂F₄-, -CF₂CH₂-, -CH₂CF₂-, - CF=CF-, -CH=CH-, -C≡C- ou une liaison simple,
a représente 0, 1 ou 2,
b représente 0, 1 ou 2, et
c représente 0, 1 ou 2, où a + b + c est ≤ 2.

2. Composés de cristaux liquides selon la revendication 1, **caractérisé en ce que** R¹ représente un radical alkyle à chaîne droite qui comporte de 1 à 10 atomes de C ou un radical alcényle qui comporte de 2 à 10 atomes de C.

3. Composés de cristaux liquides selon la revendication 1 ou 2, **caractérisé en ce que**
R² représente F, CI, CN, CF₃, SF₅, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCCIFCF₂CF₃.

4. Composés de cristaux liquides selon l'une des revendications 1 à 3, **caractérisé en ce que** c = 0.

5. Composés de cristaux liquides selon la revendication 1 des formules I1 à I24: 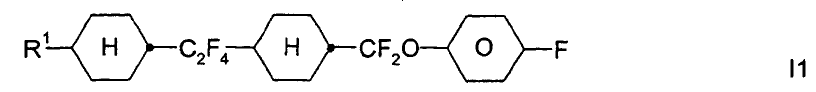 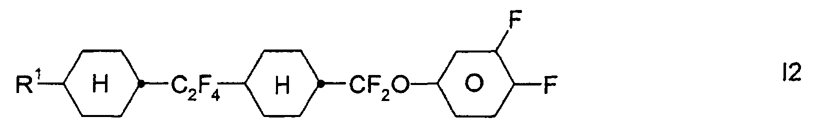 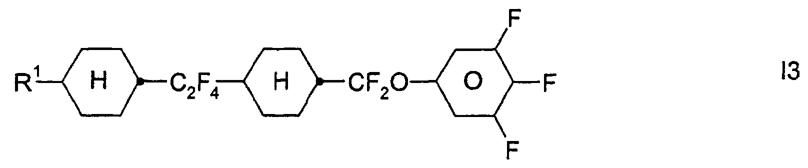 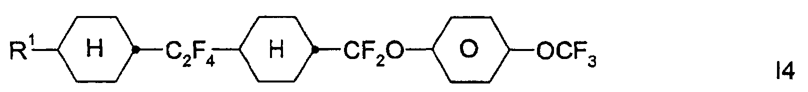 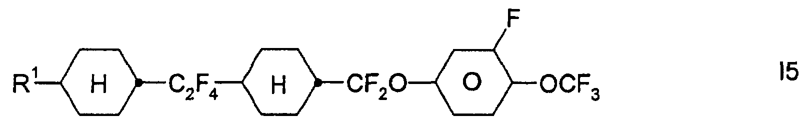 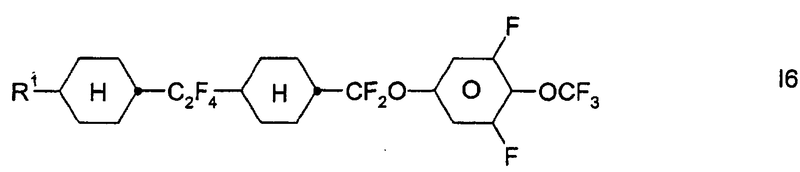 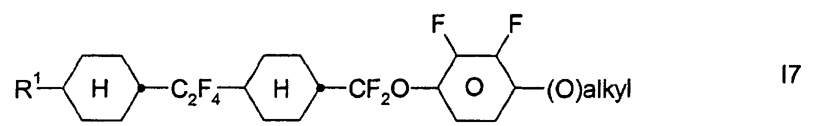 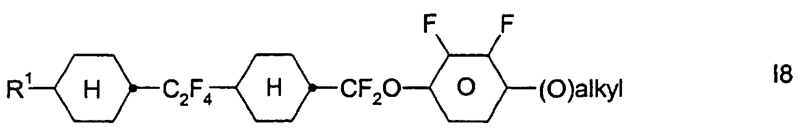 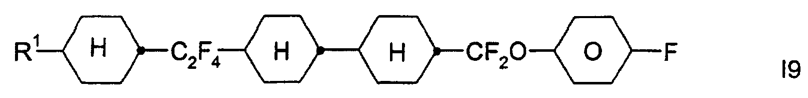 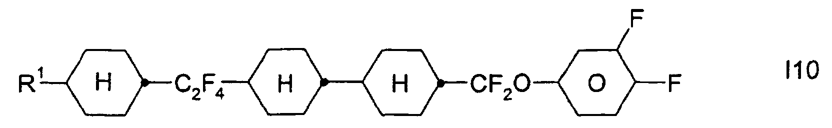 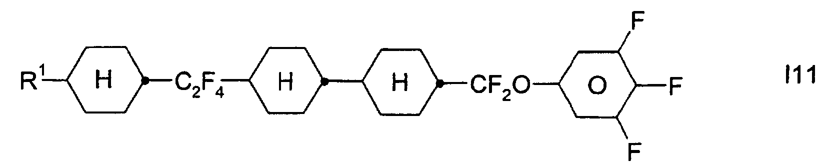 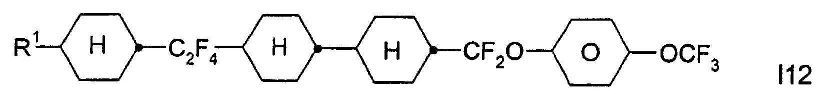 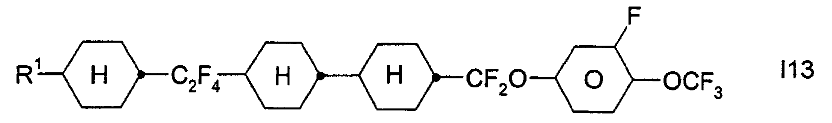 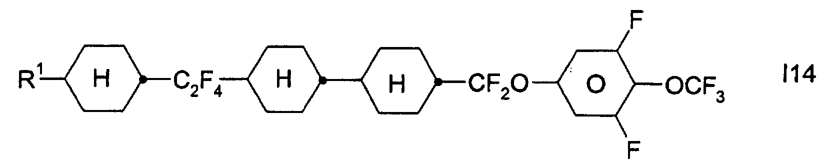 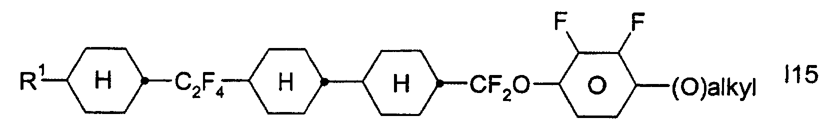 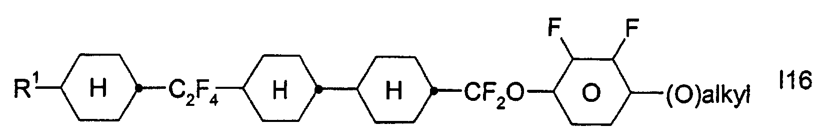 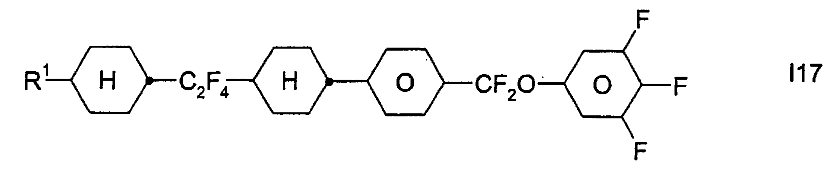 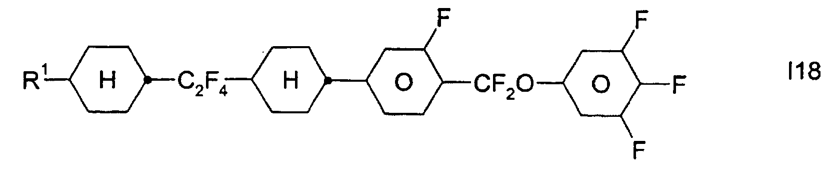 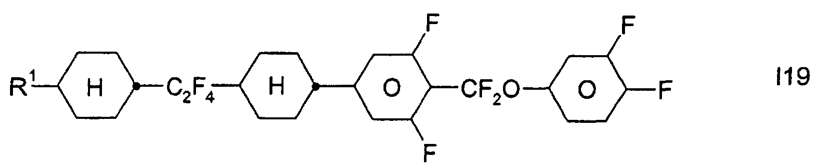 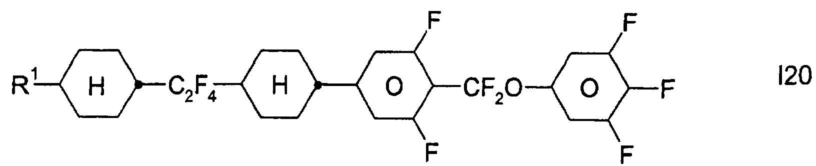 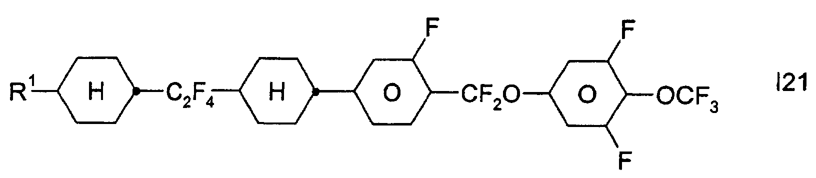 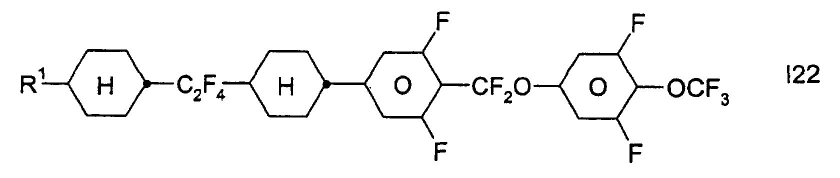 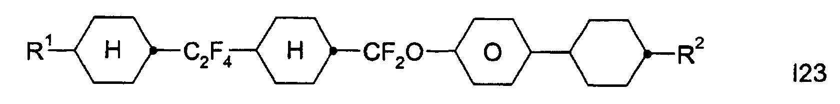 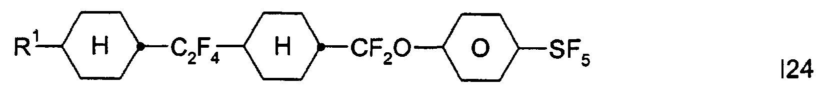 dans lesquelles
R¹ présente la signification qui est donnée dans la revendication 1, et "alkyl" est un radical alkyle à chaîne droite ou dérivé qui comporte de 1 à 9 atomes de C.

6. Milieu de cristaux liquides qui comprend au moins deux composés mésogéniques, **caractérisé en ce qu'**il comprend au moins un composé de la formuleI selon la revendication 1.

7. Milieu de cristaux liquides selon la revendication 6, **caractérisé en ce qu'**il comprend de façon additionnelle un ou plusieurs composés qui sont choisis parmi le groupe qui est constitué par les formules générales II, III, IV, V, VI, VII, VIII et IX: 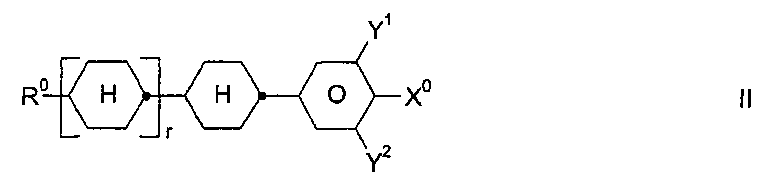 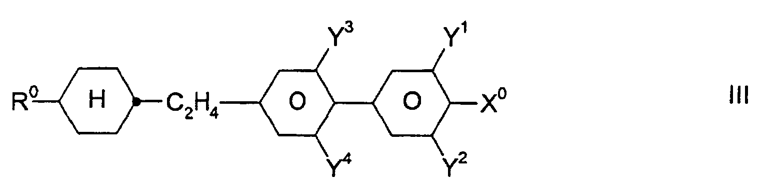 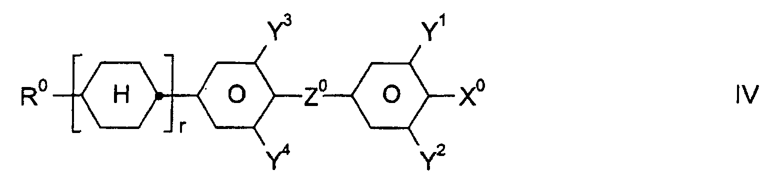 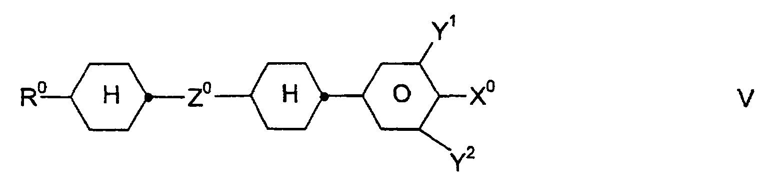 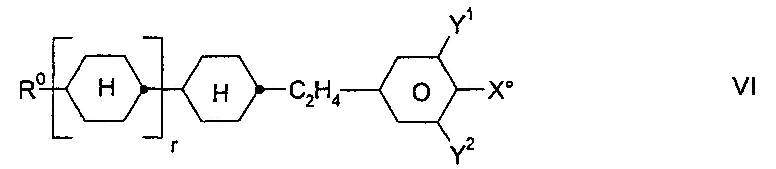 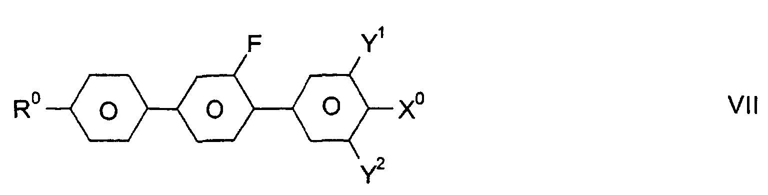 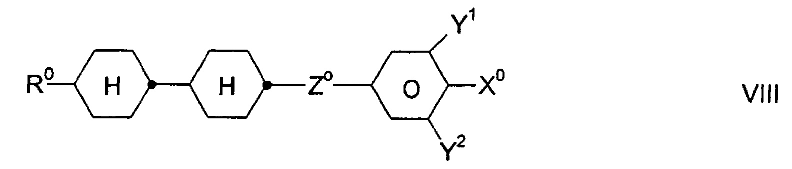 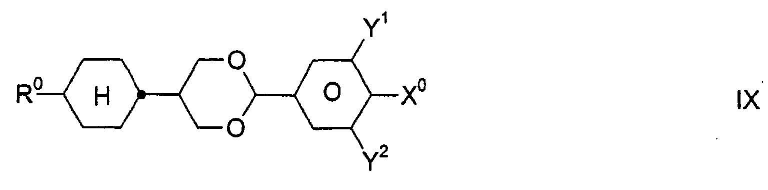 dans lesquelles les radicaux individuels présentent les significations qui suivent:
R⁰ n-alkyle, oxaalkyle, fluoroalkyle, alcényloxy ou alcényle, chacun comportant jusqu'à 9 atomes de C,
X⁰ F, CI, alkyle halogéné, alcényle halogéné, alcényloxy halogéné ou alkoxy halogéné comportant jusqu'à 7 atomes de C,
Z⁰ -CH=CH-, -C₂H₄-, -C₂F₄-, -CF=CF-, -CF₂O-, -OCF₂- ou -COO-,
Y¹,Y², Y³ et Y⁴ chacun, indépendamment les uns des autres, H ou F, et
r 0 ou 1.

8. Milieu selon la revendication 6 ou 7, **caractérisé en ce que** la proportion de composés des formules I à IX dans le mélange pris dans sa globalité est d'au moins 50% en poids.

9. Milieu selon l'une des revendications 6 à 8, **caractérisé en ce qu'**il comprend de façon additionnelle un ou plusieurs composés des formules RI à RX: 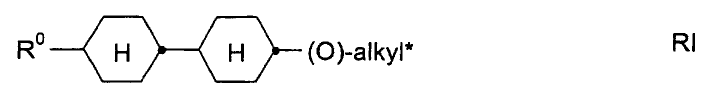 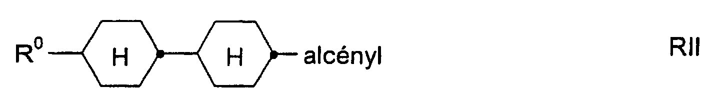 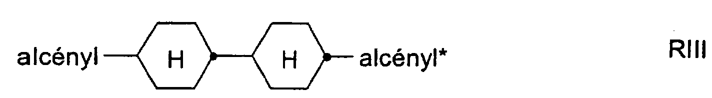 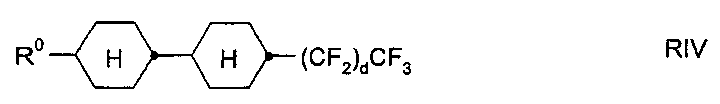 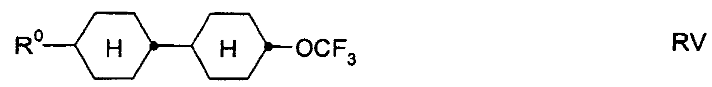 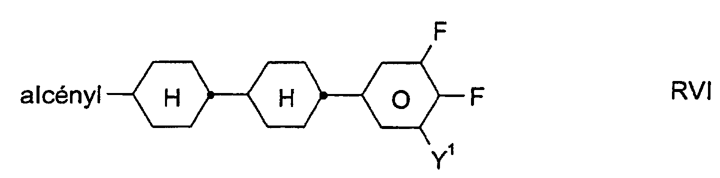 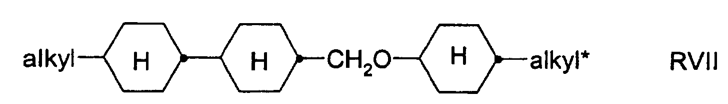 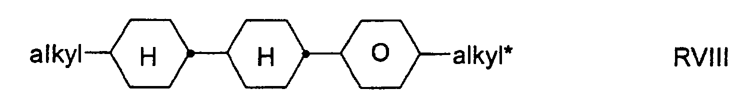 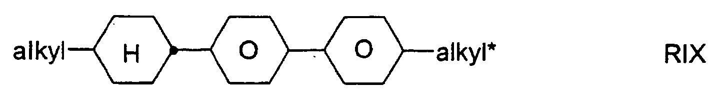 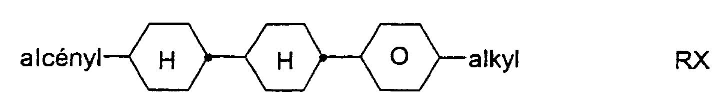 dans lesquelles
R⁰ représente n-alkyle, oxaalkyle, fluoroalkyle, alcényloxy ou alcényle, chacun comportant jusqu'à 9 atomes de C,
d représente 0, 1 ou 2,
Y¹ représente H ou F,
alkyl ou alkyl^{*} chacun, indépendamment l'un de l'autre, représente un radical alkyle à chaîne droite ou dérivé qui comporte de 1 à 9 atomes de C,
alcényl ou alcényl^{*} chacun, indépendemment l'un de l'autre, représente un radical alcényle à chaîne droite ou dérivé qui comporte de 1 à 9 atomes de C.

10. Milieu selon la revendication 7, **caractérisé en ce que** X⁰ représente F ou OCF₃ et Y² représente H ou F.

11. Utilisation du milieu de cristaux liquides selon la revendication 6 à des fins électro-optiques.

12. Affichage à cristaux liquides électro-optique qui contient un milieu de cristaux liquides selon la revendication 6.
